# EUROPEAN PATENT SPECIFICATION

(11) **EP 1 446 387 B1**
(45) Date of publication and mention of the grant of the patent: **04.11.2009**
(21) Application number: 02780507.6
(22) Date of filing: 15.11.2002
(51) Int. Cl.: C07D 241/20, C07D 241/18, C07D 241/26, C07D 401/12, C07D 403/12, C07D 405/12, C07D 491/04, C07D 409/12, C07D 401/04, C07D 401/14, A61K 31/4965, A61P 25/00

(54) **SUBSTITUTED ARYL 1,4-PYRAZINE DERIVATIVES**
SUBSTITUIERTE ARYL 1,4-PYRAZIN DERIVATE
DERIVES D'ARYLE-1,4-PYRAZINE SUBSTITUES

(30) Priority: 21.11.2001 US 332052 P; 21.02.2002 US 358546 P; 13.06.2002 US 338285 P; 13.09.2002 US 410378 P
(43) Date of publication of application: 18.08.2004
(73) Proprietor: Pharmacia & Upjohn Company LLC, Kalamazoo, MI 49001 (US)
(72) Inventor: VERHOEST, Patrick, R., Augusta, MI 49012 (US); HOFFMAN, Robert, L., Kalamazoo, MI 49009 (US); CORBETT, Jeffrey, W., Portage, MI 49024 (US); ENNIS, Michael, D., Mattawan, MI 49071 (US); FRANK, Kristine, E., Portage, MI 49009 (US); FU, Jian-Min, Kalamazoo, MI 49009 (US)
(74) Representative: Lane, Graham Mark Hamilton
(86) International application number: PCT/US2002/033642
(87) International publication number: WO 2003/045924

(56) References cited:
- WO-A-01/60806
- WO-A-98/38174
- H. NAKAMURA: "SYNTHESIS A. CHEMIOLUMINESCENCE OF 5-[PYRIDYL-,2-PYRAZINYL-, AND (SUBSTIT. 2-PYRAZINYL)AMINO]-1,2,4-TRIOXANES." BULLETIN OF THE CHEMICAL SOCIETY OF JAPAN., vol. 61, no. 10, 1988, pages 3776-8, XP002233212 JAPAN PUBLICATIONS TRADING CO. TOKYO., JP ISSN: 0009-2673
- CHEMICAL ABSTRACTS, vol. 121, no. 28, 1994 Columbus, Ohio, US; abstract no. 83374r, page 1111; column 2; XP002233213 & JP 00 601776 A (NIPPON SODA) 11 January 1994 (1994-01-11)
- CHEMICAL ABSTRACTS, vol. 127, no. 2, 1997 Columbus, Ohio, US; abstract no. 17644b, FOKS,H.: "STUDIES ON PYRAZINE DERIVATIVES.XXX" page 559; XP002233214 & ACTA POL. PHARM., vol. 54, no. 1, 1997, pages 55-62, POL.

## Description

### FIELD OF THE INVENTION

This invention relates to substituted aryl 1,4-pyrazine derivatives and processes for preparing them, pharmaceutical compositions containing them, and methods of using them to treat a disorder or condition which can be effected or facilitated by antagonizing a CRF receptor, including but not limited to disorders induced or facilitated by CRF, such as anxiety disorders, and depression and stress related disorders. Additionally this invention relates to the use of such compounds as probes for the localization of CRF₁ receptors in cells or tissues.

### BACKGROUND OF THE INVENTION

Corticotropin releasing factor (CRF) is a 41 amino acid peptide that is the primary physiological regulator of proopiomelanocortin (POMC) derived peptide secretion from the anterior pituitary gland [J. Rivier et al., Proc. Natl. Acad. Sci (USA) 80:4851 (1983); W. Vale et al., Science 213:1394 (1981)]. In addition to its endocrine role at the pituitary gland, immunohistochemical localization of CRF has demonstrated that the hormone has a broad extrahypothalamic distribution in the central nervous system and produces a wide spectrum of autonomic, electrophysiological and behavioral effects consistent with a neurotransmitter or neuromodulator role in the brain [W. Vale et al., Rec. Prog. Horm. Res. 39:245 (1983); .F. Koob, Persp. Behav. Med. 2:39 (1985); E.B. De Souza et al., J. Neurosci. 5:3189 (1985)]. There is also evidence that CRF plays a significant role in integrating the response in the immune system to physiological, psychological, and immunological stressors [J.E. Blalock, Physiological Reviews 69:1 (1989); J.E. Morley, Life Sci. 41:527 (1987)].

There is evidence that CRF has a role in psychiatric disorders and neurological diseases including depression, anxiety-related disorders and feeding disorders. A role for CRF has also been postulated in the etiology and pathophysiology of Alzheimer's disease, Parkinson's disease, Huntington's disease, progressive supranuclear palsy and amyotrophic lateral sclerosis, as they relate to the dysfunction of CRF neurons in the central nervous system [for a review, see: E.B. De Souze, Hosp. Practice 23:59 (1988)].

Anxiety disorders are a group of diseases, recognized in the art, that includes phobic disorders, anxiety states, post-traumatic stress disorder and atypical anxiety disorders [The Merck Manual of Diagnosis and Therapy, 16th edition (1992)]. Emotional stress is often a precipitating factor in anxiety disorders, and such disorders generally respond to medications that lower response to stress.

In affective disorder, or major depression, the concentration of CRF is significantly increased in the cerebral spinal fluid (CSF) of drug-free individuals [C.B. Nemeroff et al., Science 226:1342 (1984); C.M. Banki et al., Am. J. Psychiatry 144:873 (1987); R.D. France et al., Biol. Psychiatry 28:86 (1988); M. Arato et al., Biol. Psychiatry 25:355 (1989)]. Furthermore, the density of CRF receptors is significantly decreased in the frontal cortex of suicide victims, consistent with a hypersecretion of CRF [C.B. Memeroff et al., Arch. Gen. Psychiatry 45:577 (1988)]. In addition, there is a blunted adrenocorticotropin (ACTH) response to CRF (i.v. administered) observed in depressed patients [P.W. Gold et al., Am. J. Psychiatry 141:619 (1984); F. Holsboer et al., Psychoneuroendocrinology 9:147 (1984); P.W. Gold et al., New Engl. J. Med. 314:1129 (1986)]. Preclinical studies in rats and non-human primates provide additional support for the hypothesis that hypersecretion of CRF may be involved in the symptoms seen in human depression [R.M. Sapolsky, Arch. Gen. Psychiatry 46:1047 (1989)]. There is also preliminary evidence that tricyclic antidepressants can alter CRF levels and thus modulate the numbers of receptors in the brain [Grigoriadis et al., Neuropsychopharmacology 2:53 (1989)].

CRF has also been implicated in the etiology of anxiety-related disorders, and is known to produce anxiogenic effects in animals. Interactions between benzodiazepine/non-benzodiazepine anxiolytics and CRF have been demonstrated in a variety of behavioral anxiety models [D.R. Britton et al., Life Sci. 31:363 (1982); C.W. Berridge and A.J. Dunn Regul. Peptides 16:83 (1986)]. Preliminary studies using the putative CRF receptor antagonist α-helical ovine CRF (9-41) in a variety of behavioral paradigms demonstrates that the antagonist produces "anxiolytic-like" effects that are qualitatively similar to the benzodiazepines [C.W. Berridge and A.J. Dunn Horm. Behav. 21:393 (1987), Brain Research Reviews 15:71 (1990)].

Neurochemical, endocrine and receptor binding studies have all demonstrated interactions between CRF and benzodiazepine anxiolytics, providing further evidence for the involvement of CRF in these disorders. Chlodiazepoxide attenuates the "anxiogenic" effects of CRF both in the conflict test [K.T. Britton et al., Psychopharmacology 86:170 (1985); K.T. Britton et al., Psychopharmacology 94:306 (1988)] and in the acoustic startle test [N.R. Swerdlow et al., Psychopharmacology 88:147 (1986)] in rats. The benzodiazepine receptor antagonist Ro 15-1788, which was without behavioral activity alone in the operant conflict test, reversed the effects of CRF in a dose-dependent manner while the benzodiazepine inverse agonist FG 7142 enhanced the actions of CRF [K.T. Britton et al., Psychopharmacology 94:396 (1988)]. The mechanisms and sites of action through which conventional anxiolytics and antidepressants produce their therapeutic effects remain to be elucidated. Preliminary studies, examining the effects of a CRF₁ receptor antagonist peptide (a-helical CRF₉₋₄₁) in a variety of behavioral paradigms, have demonstrated that the CRF₁ antagonist produces "anxiolytic-like" effects qualitatively similar to the benzodiazepines [for a review, see: G.F. Koob and K.T. Britton, In: Corticotropin-Releasing Factor: Basic and Clinical Studies of a Neuropeptide, E.B. De Souza and C.B. Nemeroff eds., CRC Press p.221 (1990)].

The use of CRF₁ antagonists for the treatment of Syndrome X has also been described in U.S. Patent Application No. 09/696,822, filed October 26, 2000, and European Patent Application No. 003094414, filed October 26, 2000, which are also incorporated in their entireties herein by reference. Methods for using CRF₁ antagonists to treat congestive heart failure are described in U.S. Serial No. 09/248,073, filed February 10, 1999, now U.S. patent 6,043,260 (March 28, 2000) which is also incorporated herein in its entirety by reference.

CRF is known to have a broad extrahypothalmic distribution in the CNS, contributing therein to a wide spectrum of autonomic behavioral and physiological effects [see, e.g., Vale et al., 1983; Koob, 985; and E.B. De Souze et al., 1985]. For example, CRF concentrations are significantly increased in the cerebral spinal fluid of patients afflicted with affective disorder or major depression [see, e.g., Nemeroff et al., 1984; Banki et al., 1987; France et al., 1988; Arato et al., 1989]. Moreover, excessive levels of CRF are known to produce anxiogenic effects in animal models [see, e.g., Britton et al., 1982; Berridge and Dunn, 1986 and 1987], and CRF₁ antagonists are known to produce anxiolytic effects; accordingly, therapeutically effective amounts of compounds provided herein are, for example, determined by assessing the anxiolytic effects of varying amounts of the compounds in such animal models.

The following patents or patent applications disclose compounds as antagonists of CRF₁ receptors: WO0160806, W09735901, WO9829119, W09736886, WO9736898, and U.S. Patents Nos. 5872136, 5880140, and 5883105. The compounds are useful for treating CNS-related disorders, particularly affective disorders and acute and chronic neurological disorders.

### SUMMARY OF THE INVENTION

We have found that compounds of Formulae IV, V and VI as well as stereoisomers thereof, and pharmaceutically acceptable salts thereof, are CRF₁ antagonists and are useful in the treatment of disorders and diseases associated with CRF₁ receptors, including CNS-related disorders and diseases, particularly psychiatric disorders, affective disorders such as anxiety disorders, depression and stress related disorders ,and acute and chronic neurological disorders and diseases. The compounds are also useful in smoking cessation programs.

In another aspect, the present invention provides a novel compound of Formulae IV, V an VI or a stereoisomer thereof, or a pharmaceutically acceptable salt thereof, which are useful for the treatment of the disorders or diseases that are associated with CRF₁ receptors, or disorders the treatment of which can be effected or facilitated by antagonizing CRF, in a mammal, particularly in a human, such as social anxiety disorder; panic disorder; obsessive-compulsive disorder; anxiety with co-morbid depressive illness; affective disorder; anxiety; and depression.

In still another aspect, the present invention provides for the use of a compound of the invention for treatment of a disorder disclosed herein above in a mammal, particularly in a human.

In still another aspect, the present invention provides for a composition comprising a compound of the invention useful for treatment of a disorder disclosed herein above in a mammal, particularly in a human.

In still another aspect, the present invention provides for the use of a compound of the invention in a binding assay, wherein one or more of the compounds may be joined to a label, where the label can directly or indirectly provide a detectable signal. Various labels include radioisotopes, fluorescers, chemiluminescers, specific binding molecules, particles, e.g. magnetic particles, and the like.

In yet another aspect, the present invention relates to the use of the compounds of the invention (particularly labeled compounds of this invention) as probes for the localization of receptors in cells and tissues and as standards and reagents for use in determining the receptor-binding characteristics of test compounds.

Labeled compounds of the invention may be used for *in vitro* studies such as autoradiography of tissue sections or for *in vivo* methods, e.g. PET or SPECT scanning. Particularly, compounds of the invention are useful as standards and reagents in determining the ability of a potential pharmaceutical to bind to the CRF₁ receptor.

### DETAILED DESCRIPTION OF THE INVENTION

In a first aspect, this invention provides a compound of Formulae IV, V and VI.

Compounds provided herein can have one or more asymmetric centers or planes, and all chiral (enantiomeric and diasteteomeric) and racemic forms of the compound are included in the present invention. Many geometric isomers of olefins, C=N double bonds, and the like can also be present in the compounds, and all such stable isomers are contemplated in the present invention. Compounds of the invention are isolated in either the racemic form, or in the optically pure form, for example, by resolution of the racemic form by conventional methods such as crystallization in the presence of a resolving agent, or chromatography, using, for example, a chiral HPLC column, or synthesized by an asymmetric synthesis route enabling the preparation of enantiomerically enriched material. The present invention encompasses all possible tautomers of the compounds represented by Formulae IV, V and VI. The invention provides a compound of Formula IV 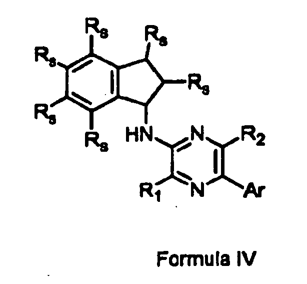 or a stereoisomer thereof, or a pharmaceutically acceptable salt thereof,
wherein in Formula IV,
Ar is selected from aryl, substituted aryl, heteroaryl, or substituted heteroaryl;
m is 0,1 or 2;
R₁ and R₂ are independently selected from halogen, -NO₂, -CN, -Rₐ, -ORₐ, -S(O)ₘRₐ, -NRₐRₐ, -C(O)NRₐRₐ, -C(S)NRₐRₐ -S(O)ₘNRₐRₐ, -NRₐS(O)ₘRₐ, - NRₐC(O)ORₐ, -OC(O)NRₐRₐ, -NRₐC(O)NRₐRₐ, -NRₐC(S)NRₐRₐ, -C(O)ORₐ, - C(S)ORₐ, or -OC(O)ORₐ;
Rₛ each is independently selected from halogen, -NO₂, -CN, -Rₐ, -ORₐ, - S(O)ₘRₐ, -NRₐRₐ, -C(O)NRₐRₐ, -C(S)NRₐRₐ -S(O)ₘNRₐRₐ, -NRₐS(O)ₘRₐ,-NRₐC(O)ORₐ, -OC(O)NRₐRₐ, -NRₐC(O)NRₐRₐ, -NRₐC(S)NRₐRₐ, -C(O)ORₐ, - C(S)ORₐ, or -OC(O)ORₐ;
Rₐ each is independently selected from H, alkyl, cycloalkyl, haloalkyl, aryl, heteroaryl, or heterocycloalkyl optionally substituted with 1 to 5 of Rₜ, -ORₜ, -S(O)ₘRₜ, NRₜRₜ, oxo (=O), thione (=S), phenyl, heteroaryl, or heterocycloalkyl where phenyl, heteroaryl, and heterocycloalkyl are optionally substituted with 1 to 5 independently taken from R_{T}; and
Rₜ each is independently selected from H, halogen, -NO₂, -NH₂, -OH, -SH, - CN, -C(O)NH₂, -C(O)-NHalkyl, -C(O)Nalkylalkyl, -Oalkyl, NHalkyl, Nalkylalkyl, - S(O)ₘalkyl, SO₂NH₂, SO₂NHalkyl and SO₂Nalkylalkyl, alkyl, cycloalkyl, haloalkyl, phenyl, benzyl, heteroaryl, or heterocycloalkyl where phenyl, benzyl heteroaryl and heterocycloalkyl may be optionally substituted with alkyl or halogen, wherein "substituted aryl" means an aryl group optionally substituted with 1-5 substituents independently selected from halogen, -NO₂, -CN, -Rₐ, -ORₐ, -S(O)ₘRₐ, -NRₐRₐ, -C(O)NRₐRₐ, -C(S)NRₐRₐ, -S(O)ₘNRₐRₐ, -NRₐS(O)ₘRₐ, NRₐC(O)ORₐ, -OC(O)NRₐRₐ -NRₐC(O)NRₐRₐ, -NRₐC(S)NRₐRₐ, -C(O)ORₐ, -C(S)ORₐ and -OC(O)ORₐ;
"heteroalyl" means a radical attachet via a ring carbon or nitrogen atom of a monocyclic aromatic ring containing five or six ring atoms consisting of carbon and 1, 2, 3 or 4 heteroatoms each selected from the group consisting of non-peroxide O, S, N, with appropriate bonding to satisfy valence requirements as well as a radical (attachment at either carbon or nitrogen) of a fused bicyclic heteroaromatic of about eight to ten ring atoms,
"substituted heteroaryl" means a heteroaryl group having 1-5 substituents independently selected from halogen, -NO₂, -CN, -Rₐ, -ORₐ, -S(O)ₘRₐ, -NRₐRₐ, -C(O)NRₐRₐ, -C(S)NRₐRₐ, -S(O)ₘNRₐRₐ, -NRₐS(O)ₘRₐ, NRₐC(O)ORₐ, -OC(O)NRₐRₐ -NRₐC(O)NRₐRₐ, -NRₐC(S)NRₐRₐ, -C(O)ORₐ, -C(S)ORₐ and -OC(O)ORₐ;
"alkyl" means both straight and branched chain moieties having from 1-10 carbon atoms optionally containing one or more double or triple bonds;
"cycloalkyl" means a monocyclic or bicyclic alkyl moiety having from 3-10 carbon atoms optionally containing 1 to 2 double bonds provided that the moiety is not aromatic;
"haloalkyl" means an alkyl moiety having from 1-10 carbon atoms and having 1 to (2v+1) independently selected halogen substituent(s) where v is the number of carbon atoms in the moiety;
"heterocycloalkyl", unless otherwise specified, means a 4 to 8 membered monocyclic ring or bicyclic ring, wherein at least one carbon atom is replaced with a hereromember selected from oxygen, nitrogen, -NH-, or -S(O)ₘ-, wherein m is zero, 1, or 2, optionally containing from one to three double bonds, provided that the molecule is not aromatic; and provided that ring attachment can occur at either a carbon or nitrogen atom;
"substituted heterocycloalkyl" is a heterocycloalkyl group having 1-5 substituents independently selected from halogen, -NO₂, -CN, -Rₐ, -ORₐ, -S(O)ₘRₐ, -NRₐRₐ, -C(O)NRₐRₐ, -C(S)NRₐRₐ, -S(O)ₘNRₐRₐ, -NRₐS(O)ₘRₐ, NRₐC(O)ORₐ, -OC(O)NRₐRₐ -NRₐC(O)NRₐRₐ, -NRₐC(S)NRₐRₐ, -C(O)ORₐ, -C(S)ORₐ and -OC(O)ORₐ;
and halogen is a group selected from -F-, -Cl-, -Br, and -I.

Still other preferred compounds of the invention include those of Formula V 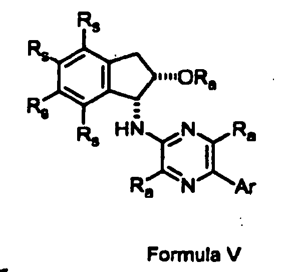 or a stereoisomer thereof, or a pharmaceutically acceptable salt thereof,
wherein in Formula V,
Ar is selected from aryl, substituted aryl, heteroaryl, or substituted heteroaryl;
m is 0,1 or 2;
Rₛ, each is independently selected from halogen, -NO₂, -CN, -Rₐ, -ORₐ, - S(O)ₘRₐ, -NRₐRₐ, -C(O)NRₐRₐ, -C(S)NRₐRₐ -S(O)ₘNRₐRₐ, -NRₐS(O)ₘRₐ, - NRₐC(O)ORₐ, -OC(O)NRₐRₐ, -NRₐC(O)NRₐRₐ, -NRₐC(S)NRₐRₐ, -C(O)ORₐ, - C(S)ORₐ, or -OC(O)ORₐ;
Rₐ each is independently selected from H, alkyl, cycloalkyl, haloalkyl, aryl, heteroaryl, or heterocycloalkyl optionally substituted with 1 to 5 of Rₐ, -ORₜ, -S(O)ₘRₜ, NRₜRₜ, oxo (=O), thione (=S), phenyl, heteroaryl, or heterocycloalkyl where phenyl, heteroaryl, and heterocycloalkyl are optionally substituted with 1 to 5 independently taken from Rₜ; and
Rₜ each is independently selected from H, halogen, -NO₂, -NH₂, -OH, -SH, - CN, -C(O)NH₂, -C(O)-NHalkyl, -C(O)Nalkylalkyl, -Oalkyl, NHalkyl, Nalkylalkyl, - S(O)ₘalkyl, SO₂NH₂, SO₂NHalkyl and SO₂Nalkylalkyl, alkyl, cycloalkyl, haloalkyl, phenyl, benzyl, heteroaryl, or heterocycloalkyl where phenyl, benzyl heteroaryl and heterocycloalkyl may be optionally substituted with alkyl or halogen, wherein "substituted aryl", "heteroaryl", "substituted heteroaryl", "alkyl", "cycloalkyl", "haloalkyl" and "heterocycloalkyl" are as defined above.

Still other preferred compounds of the invention include those of Formula VI 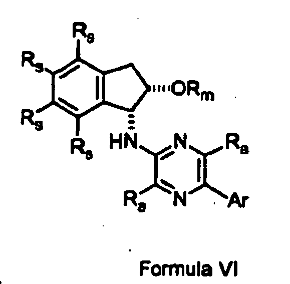 or a stereoisomer thereof, or a pharmaceutically acceptable salt thereof,
wherein in Formula VI,
Ar is selected from aryl, substituted aryl, heteroaryl, or substituted heteroaryl;
m is 0,1 or 2;
Rₐ each is independently selected from halogen, -NO₂, -CN, -Rₐ, -ORₐ, - S(O)ₘRₐ, -NRₐRₐ, -C(O)NRₐRₐ, -C(S)NRₐRₐ -S(O)ₘNRₐRₐ, -NRₐS(O)ₘRₐ, - NRₐC(O)ORₐ, -OC(O)NRₐRₐ, -NRₐC(O)NRₐRₐ, -NRₐC(S)NRₐRₐ, -C(O)ORₐ, - C(S)ORₐ, or -OC(O)ORₐ;
Rₐ each is independently selected from H, alkyl, cycloalkyl, haloalkyl, aryl, heteroaryl, or heterocycloalkyl optionally substituted with 1 to 5 of Rₜ, -ORₜ, -S(O)ₘRₜ, NRₜRₜ, oxo (=O), thione (=S), phenyl, heteroalkyl, or heterocycloalkyl where phenyl, heteroaryl, and heterocycloalkyl are optionally substituted with 1 to 5 independently taken from Rₜ;
Rₘ is C₁-C₆ alkyl substituted with from 1-2 of halogen, -NO₂, -NH₂, -OH, - SH, -CN, -C(O)NH₂, -C(O)-NHalkyl, C(O)Nalkylalkyl, -Oalkyl, NHalkyl, Nalkylalkyl, -S(O)ₘalkyl, SO₂NH₂, SO₂NHalkyl and SO₂Nalkylalkyl, oxo (=O), thione (=S), heterocycloalkyl, or substituted heterocycloalkyl; and
Rₜ each is independently selected from H, halogen, -NO₂, -NH₂, -OH, -SH, - CN, -C(O)NH₂, -C(O)-NHalkyl, -C(O)Nalkylalkyl, -Oalkyl, NHalkyl, Nalkylalkyl, - S(O)ₘalkyl, SO₂NH₂, SO₂NHalkyl and SO₂Nalkylalkyl, alkyl, cycloalkyl, haloalkyl, phenyl, benzyl, heteroaryl, or heterocycloalkyl where phenyl, benzyl heteroaryl and heterocycloalkyl may be optionally substituted with alkyl or halogen, wherein "substituted aryl", "heteroaryl", "substituted heteroaryl", "alkyl", "cycloalkyl", "haloalkyl", "heterocycloalkyl" and "substituted heterocycloalkyl", are as defined above.

Following are examples of particular compounds of the invention, with each compound being identified by both a chemical name and a structural formula immediately below the chemical name:
5-(2,4-dichlorophenyl)N-[(1R,2S)-2-ethoxy 2,3-dihydro-1H-inden-1-yl]-3,6-diethylpyrazin-2-amine 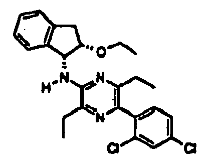
5-(2,4-dichlorophenyl)-3,6-diethyl-N-[(1R,2S)-2-methoxy-2,3-dihydro-1H-inden-1-yl]pyrazin-2-amine 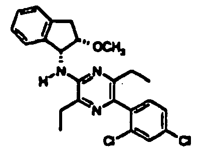
5-(2,4-dichlorophenyl)-3,6-diethyl-N-[(1R,2S)-2-isopropoxy-2,3-dihydro-1 H-inden-1-yl]pyrazin-2-amine 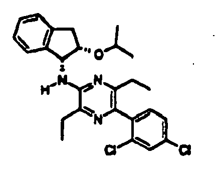
5-(2,4-dichlorophenyl)-3,6-diethyl-N-[(1S,2R)-2-methoxy-2,3-dihydro-1H-inden-1-yl]pyrazin-2-amine. 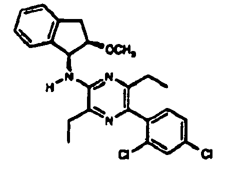
5-(2,4-dichlorophenyl)-3,6-diethyl-N-[(1S,2S)-2-methoxy-2,3-dihydro-1H-inden-1-yl]pyrazin-2-amine 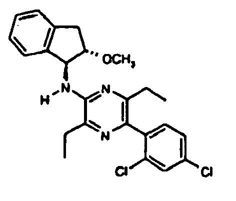
5-(2,4-dichlorophenyl)-3,6-diethyl-N-[(1R,2R)-2-methoxy-2,3-dihydro-1H-inden-1-yl]pyrazin-2-amine 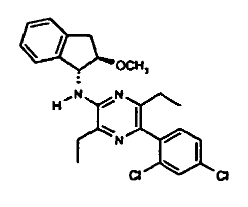
N-[(1 R,2S)-2-(cyclopropylmethoxy)-2,3-dihydro-1H-inden-1-yl]-5-(2,4-dichlorophenyl)-3,6-diethylpyrazin-2-amine. 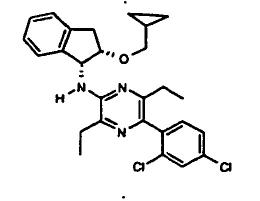
5-(2,4-dichlorophenyl)-3,6-diethyl-N-[(1R,2S)2-(prop-2-ynyloxy)-2,3-dihydro-1H-inden-1-yl]pyrazin-2-amine. 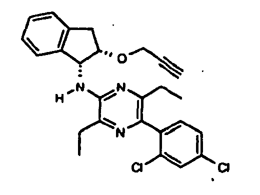
5-(2,4-dichlorophenyl)-3,6-diethyl-N-[(1R,2S)-2-(2-methoxyethoxy)-2,3-dihydro-1H-inden-1-yl]pyrazin-2-amine. 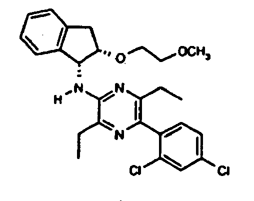
N-[(1R,2S)-2-(allyloxy)-2,3-dihydro-1H-inden-1-yl]-5-(2,4-dichlorophenyl)-3,6-diethylpyrazin-2-amine. 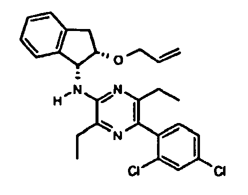
5-(2,4-dichlorophenyl)-3,6-diethyl-N-[(1R,2S)-2-(2-fluoroethoxy)-2,3-dihydro-1H-inden-1-yl]pyrazin-2-amine. 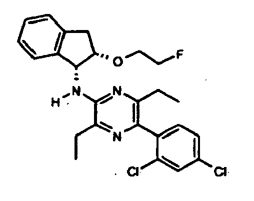
5-(2,4-dichlorophenyl)-3,6-diethyl-N-[(1R,2S)-2-propoxy-2,3-dihydro-1H-inden-1-yl]pyrazin-2-amine. 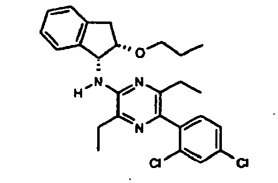
2-[((1R,2S)-1-{[5-(2,4-dichlorophenyl)-3,6-diethylpyrazin-2-yl]amino}-2,3-dihydro-1H-inden-2-yl)oxy]ethanol. 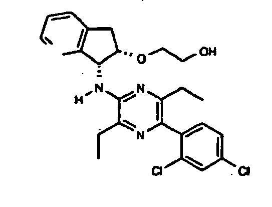
(1R,2S)-1-{[5-(2,4-dichlorophenyl)-3,6-diethylpyrazin-2-yl]amino}-2,3-dihydro-1H-inden-2-yl dimethylcarbamate. 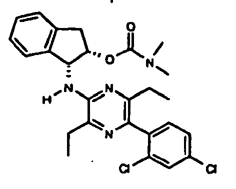
(1R,2S)-1-{[5-(2,4-dichlorophenyl)-3,6-diethylpyrazin-2-yl]amino}-2,3-dihydro-1H-inden-2-yl acetate. 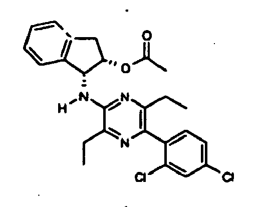
5-(2-chloro-4-methoxyphenyl)-N-[(1R,2S)-2-ethoxy-2,3-dihydro-1H-inden-1-yl]-3,6-diethylpyrazin-2-amine. 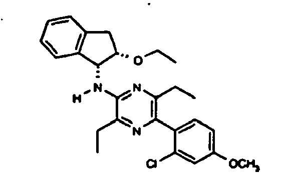
5-(2-chloro-4-methoxyphenyl)-3,6-diethyl-N-[(1R,2S)-2-(2-fluoroethoxy)-2,3-dihydro-1H-inden-1-yl]pyrazin-2-amine. 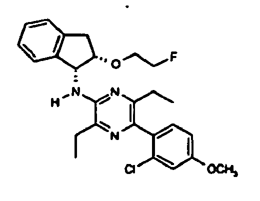
(1R,25)-1-{[5-(2-chloro-4-methoxyphenyl)-3,6-diethylpyrazin-2-yl]amino}-2,3-dihydro-1H-inden-2-yl acetate. 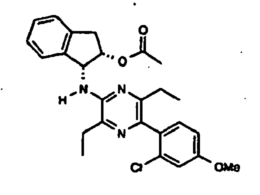
N-[(1R,2S)-2-ethoxy-2,3-dihydro-1H-inden-1-yl]-3,6-diethyl-5-(4-methoxy-2-methylphenyl)pyrazin-2-amine. 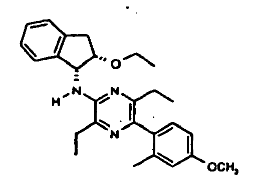
3,6-diethyl-N-[(1R,2S)-2-(2-fluoroethoxy)-2,3-dihydro-1H-inden-1-yl]-5-(4-methoxy-2-methylphenyl)pyrazin-2-amine. 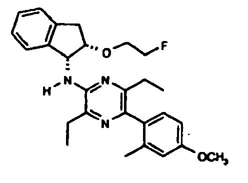
5-(2,4-dimethoxyphenyl)-N-[(1R,2S)-2-ethoxy-2,3-dihydro-1H-inden-1-yl]-3,6-diethylpyrazin-2-amine. 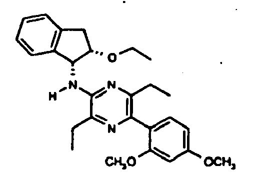
5-(2,4-dimethoxyphenyl)-3,6-diethyl-N-[(1R,2S)-2-(2-fluoroethoxy)-2,3-dihydro-1H-inden-1-yl]pyrazin-2-amine. 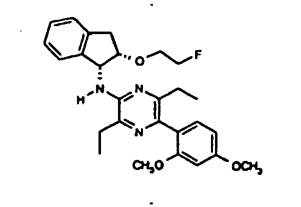
5-[2-chloro-4-(dimethylamino)phenyl]-N-[(1R,2S)-2-ethoxy-2,3-dihydro-1H-inden-1-yl]-3,6-diethylpyrazin-2-amine. 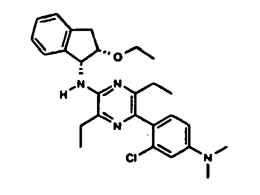
5-[2-chloro-4-(dimethylamino)phenyl]-3,6-diethyl-N-[(1R,2S)-2-(2-fluoroethoxy)-2,3-dihydro-1 H-inden-1-yl]pyrazin-2-amine. 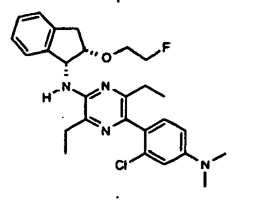
5-[6-(dimethylamino)-4-methylpyridin-3-yl]-N-[(1R,2S)-2-ethoxy-2,3-dihydro-1H-inden-1-yl]-3,6-diethylpyrazin-2-amine. 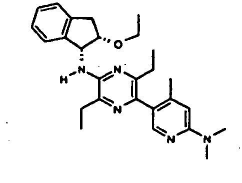
5-(2,4-dichlorophenyl)-N-(2,3-dihydro-1H-inden-1-yl)-3,6-dimethylpyrazin-2-amine 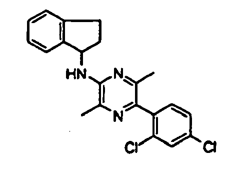
N-(2,3-dihydro-1 H-inden-1-yl)-5-(4-methoxy-2-methylphenyl)-3,6-dimethylpyrazin-2-amine 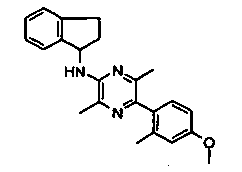
5-(2,4-dichlorophenyl)-N-[(1R,2S)-2-methoxy-2,3-dihydro-1H-inden-1-yl]-3,6-dimethylpyrazin-2-amine 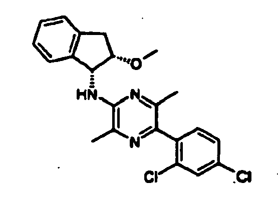
5-(2,4-dichlorophenyl)-N-[(1R,2S)-2-ethoxy-2,3-dihydro-1H-inden-1-yl]-3,6-dimethylpyrazin-2-amine 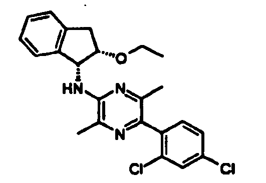
5-(2,4-dichlorophenyl)-N-[(1R,2S)-2-isopropoxy-2,3-dihydro-1H-inden-1-yl]-3,6-dimethylpyrazin-2-amine 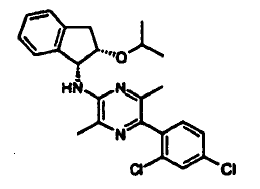
5-(2,4-dichlorophenyl)-3,6-diethyl-N-(6-methoxy-2,3-dihydro-1H-inden-1-yl)pyrazin-2-amine 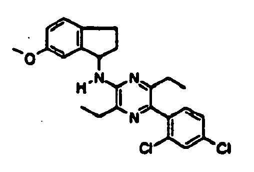
5-(2,4-dichlorophenyl)-3,6-diethyl-N-[(cis)-2-ethyl-6-methoxy-2,3-dihydro-1H-inden-1-yl]pyrazin-2-amine 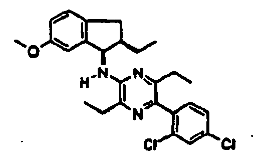
5-(2,4-chlorophenyl)-3,6-diethyl-N-(5-methoxy-2,3-hydro-1H-inden-1-yl)pyrazin-2-amine 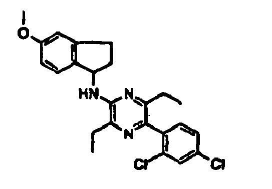
(+/-)-5-(2,4-dichlorophenyl)-3,6-diethyl-N-[cis-2-ethyl-2,3-dihydro-1H-inden-1-yl]pyrazin-2-amine. 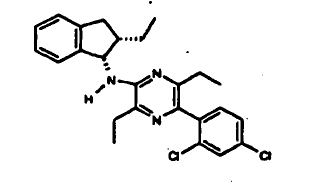
(+/-)-5-(2,4-dichlorophenyl)-3,6-diethyl-N-[trans-2-ethyl-2,3-dihydro-1H-inden-1-yl]pyrazin-2-amine. 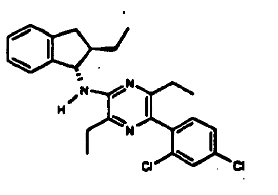
3,6-dicyclopropyl-5-(2,4-dichlorophenyl)-N-[(1R,2S)-2-ethoxy 2,3-dihydro-1H-inden-1-yl]pyrazin-2-amine 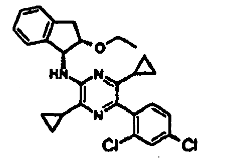
6-cyclopropyl-5-(2,4-dichlorophenyl)-N-[(1R,2S)-2-ethoxy-2,3-dihydro-1H-inden-1-yl]-3-methylpyrazin-2-amine 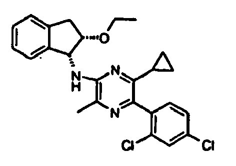
3-cyclopropyl-5-(2,4-dichlorophenyl)-N-[(1R,2S)-2-ethoxy-2,3-dihydro-1H-inden-1-yl]-6-methylpyrazin-2-amine 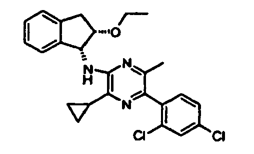
6-cyclopropyl-5-(2,4-dichlorophenyl)-N-[(1R,2S)-2-ethoxy-2,3-dihydro-1H-inden-1-yl]-3-ethylpyrazin-2-amine 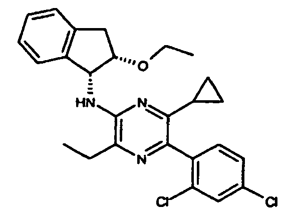
5-(2-chloro-4-methoxyphenyl)-6-cyclopropyl-N-[(1R,2S)-2-ethoxy-2,3-dihydro-1H-inden-1-yl]-3-ethylpyrazin-2-amine 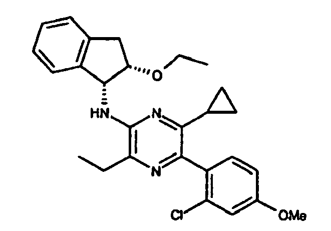
6-cyclopropyl-5-[6-(dimethylamino)-4-methylpyridin-3-yl]-N-[(1R,2S)-2-ethoxy-2,3-dihydro-1 H-inden-1-yl]-3-ethylpyrazin-2-amine 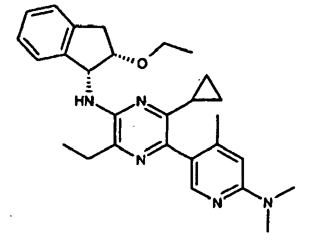
3-cyclopropyl-5-(2,4-dichlorophenyl)-N-[(1 R,2S)-2-ethoxy-2,3-dihydro- 1H-inden-1-yl]-6-ethylpyrazin-2-amine 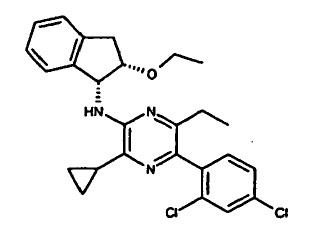
3-cyclopropyl-5-[6-(dimethylamino)-4-methylpyridin-3-yl]-N-[(1R,2S)-2-ethoxy-2,3-dihydro-1H-inden-1-yl]-6-ethylpyrazin-2-amine 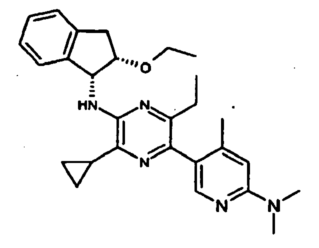
5-(2-chloro-4-methoxyphenyl)-3-cyclopropyl-N-[(1R,2S)-2-ethoxy-2,3-dihydro-1H-inden-1-yl]-6-ethylpyrazin-2-amine 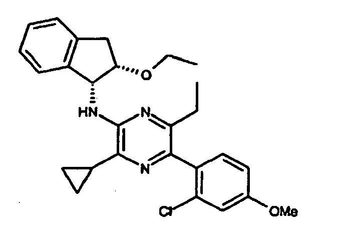
5-(2-chloro-4-methoxyphenyl)-3-cyclopropyl-N-[(1R,2S)-2-ethoxy-2,3-dihydro-1H-inden-1-yl]-6-ethylpyrazin-2-amine 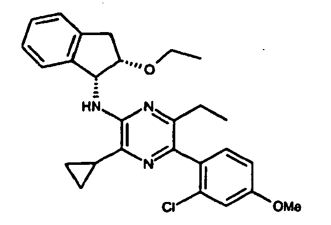
5-(2,4-dichlorophenyl)-N-[(1R,2S)-2-ethoxy-2,3-dihydro-1H-inden-1-yl]-6-ethyl-3-methylpyrazin-2-amine 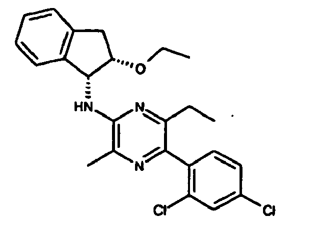
5-(2,4-dichlorophenyl)-N-[(1R,2S)-2-ethoxy-2,3-dihydro-1H-inden-1-yl]-3-ethyl-6-methylpyrazin-2-amine 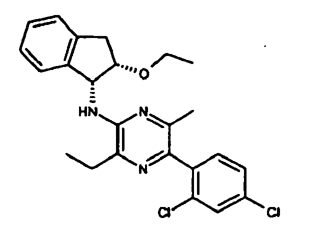
(1R,2R)-N¹-[3,6-dicyclopropyl-5-(2,4-dichlorophenyl)pyrazin-2-yl]-N²-ethyl-2,3-dihydro-1 H-indene-1,2-diamine 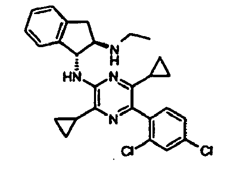
N-((1R,2R)-1-{[3,6-dicyclopropyl-5-(2,4-dichlorophenyl)pyrazin-2-yl]amino}-2,3-dihydro-1H-inden-2-yl)acetamide 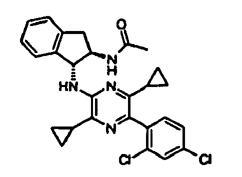
(1R,2R-N¹-[3,6-dicyclopropyl-5-(2,4-dichlorophenyl)pyrazin-2-yl]-N²-(2-methoxyethyl)-2,3-dihydro-1H-indene-1,2-diamine 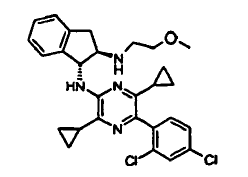
5-(2,4-dichlorophenyl)-N-[(1S,2R)-2-ethoxy-2,3-dihydro-1H-inden-1-yl]-3,6-diethylpyrazin-2-amine 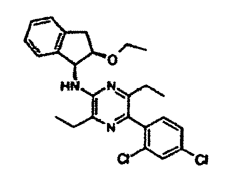
5-(2,4-dichlorophenyl)-N-[(1S,2S)-2-ethoxy-2,3-dihydro-1H-inden-1-yl]-3,6-diethylpyrazin-2-amine 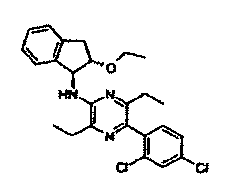
5-(2,4-dichlorophenyl)-3,6-diethyl-N-[(1S,2S)-2-(2-fluoroethoxy)-2,3-dihydro-1H-inden-1-yl]pyrazin-2-amine 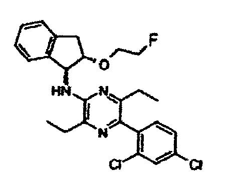
methyl 3-(2,4-dichlorophenyl)-6-{[(1R,2S)-2-ethoxy-2,3-dihydro-1H-inden-1-yl]amino}-5-methoxypyrazine-2-carboxylate 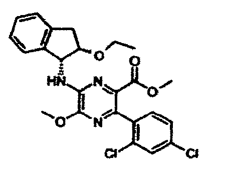
methyl 6- {[(1R,2S)-2-(acetyloxy)-2,3-dihydro-1H-inden-1-yl]amino}-3-(2,4-dichlorophenyl)-5-methoxypyrazine-2-carboxylate 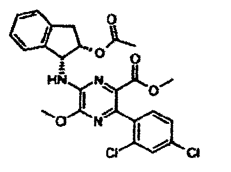
5-(2,4-dichlorophenyl)-N-[(1R,2S)-2-ethoxy-2,3-dihydro-1H-inden-1-yl]-6-methoxy-3-vinylpyrazin-2-amine 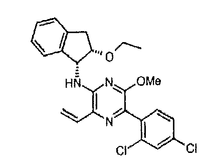
5-(2-chloro-4-methoxyphenyl)-3,6-diethyl-N-[(1R,2S)-2-(3-fluoropropoxy)-2,3-dihydro-1H-inden-1-yl]pyrazin-2-amine 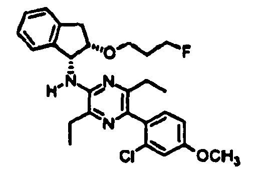
5-(2,4-dichlorophenyl)-3,6-diethyl-N-[(1R,2S)-2-(3-fluoropropoxy)-2,3-dihydro-1H-inden-1-yl]pyrazin-2-amine 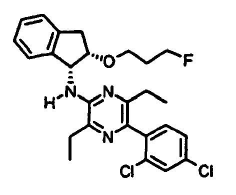
(1R,2S)-1-{[5-(2-chloro-4-methoxyphenyl)-3,6-diethylpyrazin-2-yl]amino}-2,3-dihydro-1H-inden-2-yl hydroxyacetate 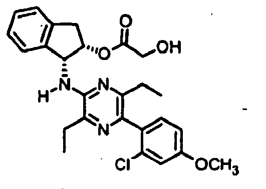
(1R,2S)-1-{[5-(2,4-dichlorophenyl)-3,6-diethylpyrazin-2-yl]amino}-2,3-dihydro-1H-inden-2-yl hydroxyacetate 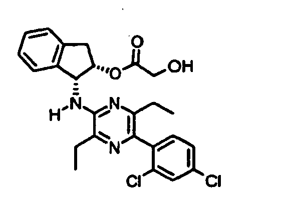
(1R,2S)-1-{[5-(2-chloro-4-methoxyphenyl)-3,6-diethylpyrazin-2-yl]amino} -2,3-dihydro-1H-inden-2-yl methoxyacetate 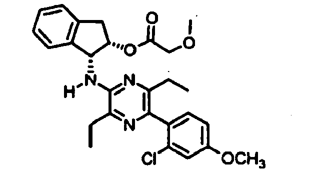
(1R,2S)-1-{[5-(2,4-dichlorophenyl)-3,6-diethylpyrazin-2-yl]amino}-2,3-dihydro-1H-inden-2-yl methoxyacetate 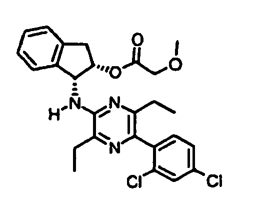
N-[(1R,2S)-2-(2-aminoethoxy)-2,3-dihydro-1H-inden-1-yl]-5-(2-chloro-4-methoxyphenyl)-3,6-diethylpyrazin-2-amine 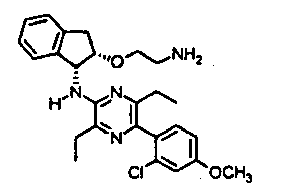
N-[(1R,2S)-2-(2-aminoethoxy)-2,3-dihydro-1H-inden-1-yl]-5-(2,4-dichlorophenyl)-3,6-diethylpyrazin-2-amine 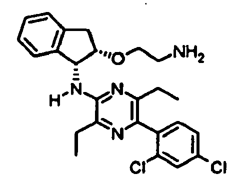
(1R,2S)-1-{[5-(2-chloro-4-methoxyphenyl)-3,6-diethylpyrazin-2-yl]amino}-2,3-dihydro-1H-inden-2-yl glycinate 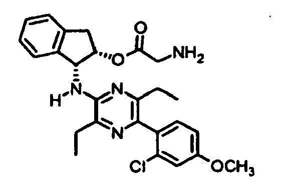
(1R,2S)-1-{[5-(2,4-dichlorophenyl)-3,6-diethylpyrazin-2-yl]amino}-2,3-dihydro-1H-inden-2-yl glycinate 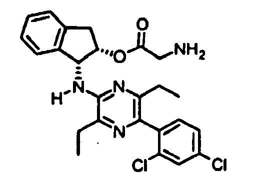
5-(2-chloro-4-methoxyphenyl)-3,6-diethyl-N-{(1R,2S)-2-[2-(methylamino)ethoxy]-2,3-dihydro-1H-inden-1-yl}pyrazin-2-amine 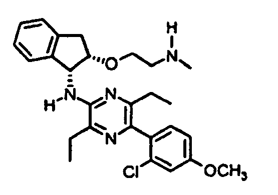
5-(2,4-dichlorophenyl)-3,6-diethyl-N-{(1R,2S)-2-[2-(methylamino)ethoxy]-2,3-dihydro-1H-inden-1-yl}pyrazin-2-amine 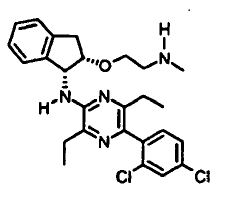
(1R,2S)-1-{[5-(2-chloro-4-methoxyphenyl)-3,6-diethylpyrazin-2-yl]amino}-2,3-dihydro-1H-inden-2-yl N-methylglycinate 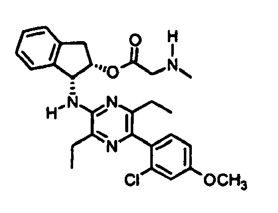
(1R,2S)-1-{[5-(2,4-dichlorophenyl)-3,6-diethylpyrazin-2-yl]amino}-2,3-dihydro-1H-inden-2-yl N-methylglycinate 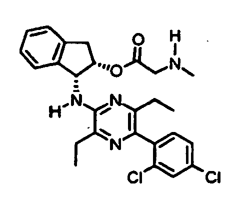
5-(2-chloro-4-methoxyphenyl)-N-{(1R,2S)-2-[2-(dimethylamino)ethoxy]-2,3-dihydro-1H-inden-1-yl}-3,6-diethylpyrazin-2-amine 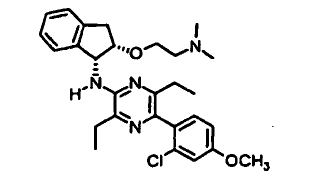
5-(2,4-dichlorophenyl)-N- {(1R,2S)-2-[2-(dimethylamino)ethoxy]-2,3-dihydro-1H-inden-1-yl}-3,6-diethylpyrazin-2-amine 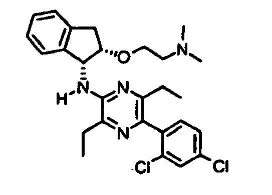
(1R,2S)-1-{[5-(2-chloro-4-methoxyphenyl)-3,6-diethylpyrazin-2-yl]amino}-2,3-dihydro-1H-inden-2-yl N,N-dimethylglycinate 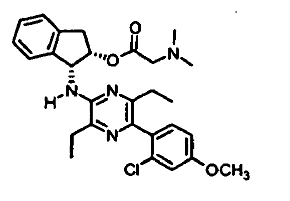
(1R,2S)-1-{[5-(2,4-dichlorophenyl)-3,6-diethylpyrazin-2-yl]amino}-2,3-dihydro-1H-inden-2-yl N,N-dimethylglycinate 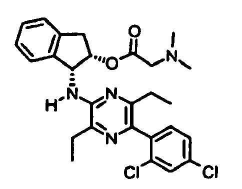
5-(2-chloro-4-methoxyphenyl)-3,6-diethyl-N-{(1R,2S)-2-(2-(methylamino)ethoxy]-2,3-dihydro-1H-inden-1-yl}pyrazin-2-amine 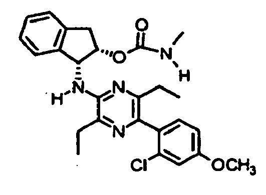
(1R,2S)-1-{[5-(2,4-dichlorophenyl)-3,6-diethylpyrazin-2-yl]amino}-2,3-dihydro-1H-inden-2-yl methylcarbamate 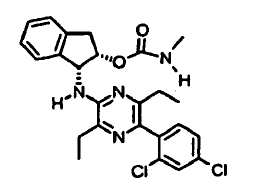
5-(4-chloro-2-methoxyphenyl)-3,6-diethyl-N-[(1R,2S)-2-(2-fluoroethoxy)-2,3-dihydro-1H-inden-1-yl]pyrazin-2-amine 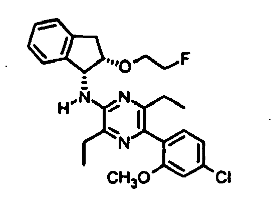
5-(3,5-dichloropyridin-2-yl)-3,6-diethyl-N-[(1R,2S)-2-(2-fluoroethoxy)-2,3-dihydro-1H-inden-1-yl]pyrazin-2-amine 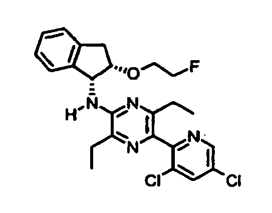
5-(3-chloro-5-methoxypyridin-2-yl)-3,6-diethyl-N-[(1R,2S)-2-(2-fluoroethoxy)-2,3-dihydro-1H-inden-1-yl]pyrazin-2-amine 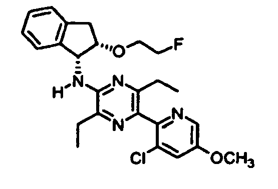
(1R,2S)-1-{[5-(4-chloro-2-methoxyphenyl)-3,6-diethylpyrazin-2-yl]amino}-2,3-dihydro-1H-inden-2-yl acetate 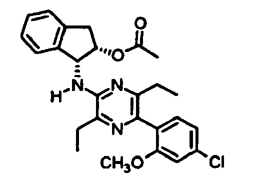
(1R,2S)-1-{[5-(3,5-dichloropyridin-2-yl)-3,6-diethylpyrazin-2-yl]amino}-2,3-dihydro-1H-inden-2-yl acetate 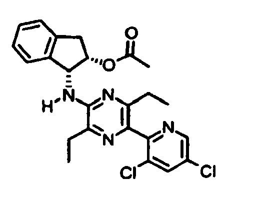
(1R,2S)-1-{[5-(3-chloro-5-methoxypyridin-2-yl)-3,6-diethylpyrazin-2-yl]amino}-2,3-dihydro-1H-inden-2-yl acetate 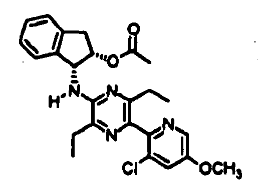
5-(4-chloro-2-methoxyphenyl)-3,6-diethyl-N-[(1R,2S)-2-(3-fluoropropoxy)-2,3-dihydro-1H-inden-1-yl]pyrazin-2-amine 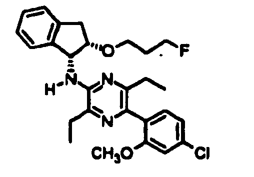
(1R,2S)-N-[5-(2,4-dichlorophenyl)-3,6-diethylpyrazin-2-yl]-N'-ethyl-2,3-dihydro-1H-indene-1,2-diamine 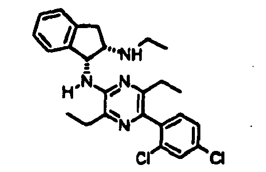
N-((1R,2S)-1-{[5-(2,4-dichlorophenyl)-3,6-diethylpyrazin-2-yl]amino}-2,3-dihydro-1 H-inden-2-yl)acetamide 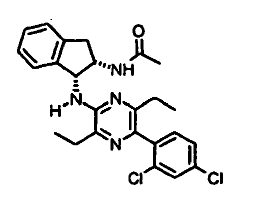
5-(2,4-dichlorophenyl)-N-[(1R,2S)-2-ethoxy-2,3-dihydro-1H-inden-1-yl]-3,6-dimethoxypyrazin-2-amine 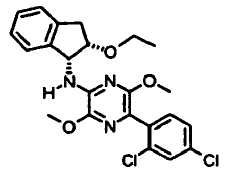
3-cyclopropyl-5-(2,4-dichlorophenyl)-N-[(1R,2S)-2-ethoxy-2,3-dihydro-1H-inden-1-yl]-6-methoxypyrazin-2-amine 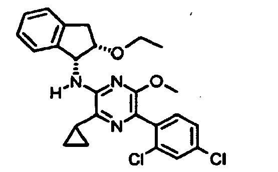
3-(2,4-dichlorophenyl)-6- {[(1R,2S)-2-ethoxy-2,3-dihydro-1H-inden-1-yl]amino}-5-ethylpyrazine-2-carboxamide 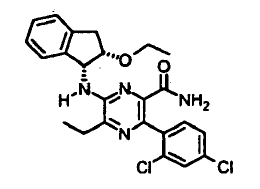
3-(2,4-dichlorophenyl)-6-{[(1R,2S)-2-ethoxy-2,3-dihydro-1H-inden-1-yl]amino}-5-methoxypyrazine-2-carboxamide 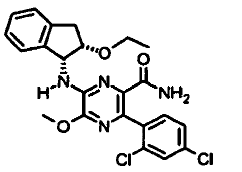
3-(2,4-dichlorophenyl)-6-{[(1R,2S)-2-ethoxy-2,3-dihydro-1H-inden-1-yl]amino}-5-(methylthio)pyrazine-2-carboxamide 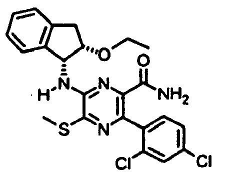
5-(2,4-dichlorophenyl)-N-[(1R,2S)-2-ethoxy-2,3-dihydro-1H-inden-1-yl]-6-ethyl-3-(methylthio)pyrazin-2-amine 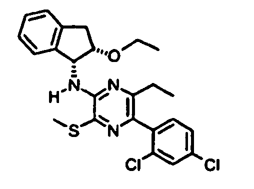
5-(2,4-dichlorophenyl)-N-[(1R,2S)-2-ethoxy-2,3-dihydro-1H-inden-1-yl]-6-ethyl-3-methoxypyrazin-2-amine 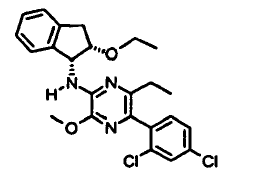
5-(2,4-dichlorophenyl)-N-[(1R,2S)-2-ethoxy-2,3-dihydro-1H-inden-1-yl]-3-ethyl-6-methoxypyrazin-2-amine 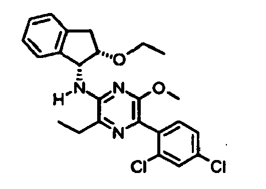
5-(2,4-dichlorophenyl)-N-[(1R,2S)-2-ethoxy-2,3-dihydro-1H-inden-1-yl]-3-ethyl-6-(methylthio)pyrazin-2-amine 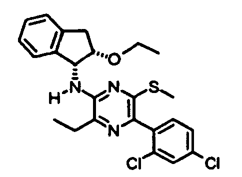
5-(2,4-dichlorophenyl)-N-[(1R,2S)-2-ethoxy-2,3-dihydro-1H-inden-1-yl]-3,6-bis(methylthio)pyrazin-2-amine 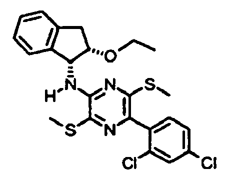
6-(2,4-dichlorophenyl)-3-{[(1R,2S)-2-ethoxy-2,3-dihydro-1H-inden-1-yl]amino}-5-ethylpyrazine-2-carboxamide 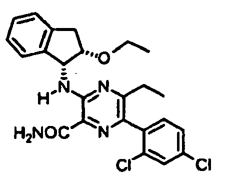
6-(2,4-dichlorophenyl)-3- {[(1R,2S)-2-ethoxy-2,3-dihydro-1H-inden-1-yl]amino }-5-methoxypyrazine-2-carboxamide 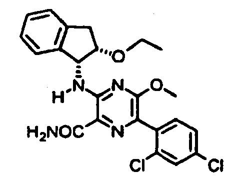
6-(2,4-dichlorophenyl)-3-{[(1R,25)-2-ethoxy-2,3-dihydro-1H-inden-1-yl]amino}-5-(methylthio)pyrazine-2-carboxamide 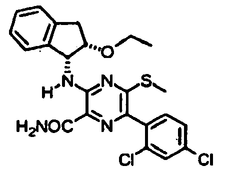
5-(2,4-dichlorophenyl)-N[(1R,2S)-2-ethoxy-2,3-dihydro-1H-inden-1-yl]-6-ethyl-N'-methylpyrazine-2,3-diamine 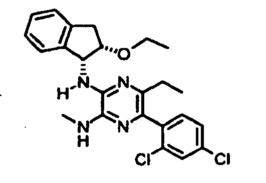
6-(2,4-dichlorophenyl)-N-[(1R,2S)-2-ethoxy-2,3-dihydro-1H-inden-1-yl]-N',N"-dimethylpyrazine-2,3,5-triamine 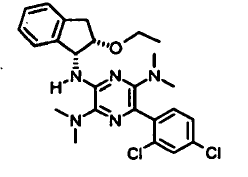
3-(2,4-dichlorophenyl)-N-[(1R,2S)-2-ethoxy-2,3-dihydro-1H-inden-1-yl]-5-ethyl-N'-methylpyrazine-2,6-diamine

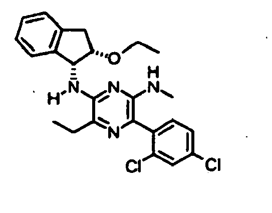

Compounds of the invention can be prepared using the reactions depicted in the following charts or variations thereof known to those skilled in the art. As illustrated in Chart A, the aminopyrazine A-II can be prepared from the suitably functionalized chloropyrazine **A-I** (see Chart C) by reaction with the appropriate heterocyclic or carbocyclic amine in the presence of a transition metal catalyst (e.g. palladium(II) acetate or tris(dibenzylideneacetone)dipalladium(0)), base (e.g. sodium or potassium tert-butoxide) in solvents such as but not limited to toluene, DMF, or dioxane. (for example see Buchwald, S.L. et al J. Org. Chem. 2000, 65, 1158.)*.* A variety of heterocyclic and carbocyclic amines are commercially available or can be synthesized by those skilled in the art. Halogenation of A-II can be accomplished by a number of methods well-known to those skilled in the art utilizing reagents such as N-chlorosuccinimide, N-bromosuccinimide, N-iodosuccinimide, bromine, iodine, pyridinium tribromide in solvents such as dichloromethane, acetic acid, DMF, etc, to give the halopyrazine **A-III**. Formation of the claimed compounds I is accomplished by a transition metal catalyzed coupling reaction **A-III** and an appropriate metalloaryl reagent such as aryl boronic acids (see for example Miyaura, N.; et al Chem. Rev. 1995, 95, 2457), aryl stannanes (see for example Mitchell, T.N. Synthesis 1992, 803), or aryl Grignards (see for example Miller, J.A. Tetrahedron Lett. 1998, 39, 7275). Alternatively, **A-I** can be coupled with a suitable metalloaryl reagent as described above to provide the arylpyrazine **A-IV**. Oxidation of the sterically less hindered nitrogen can be effected by using a variety of known oxidizing agents (eg, MCPBA, hydrogen peroxide), and the resulting N-oxide can be treated with phosphorous oxybromide to provide the bromopyrazine **A-V**. Reaction of the halogen with an amine as described above provides **I**.

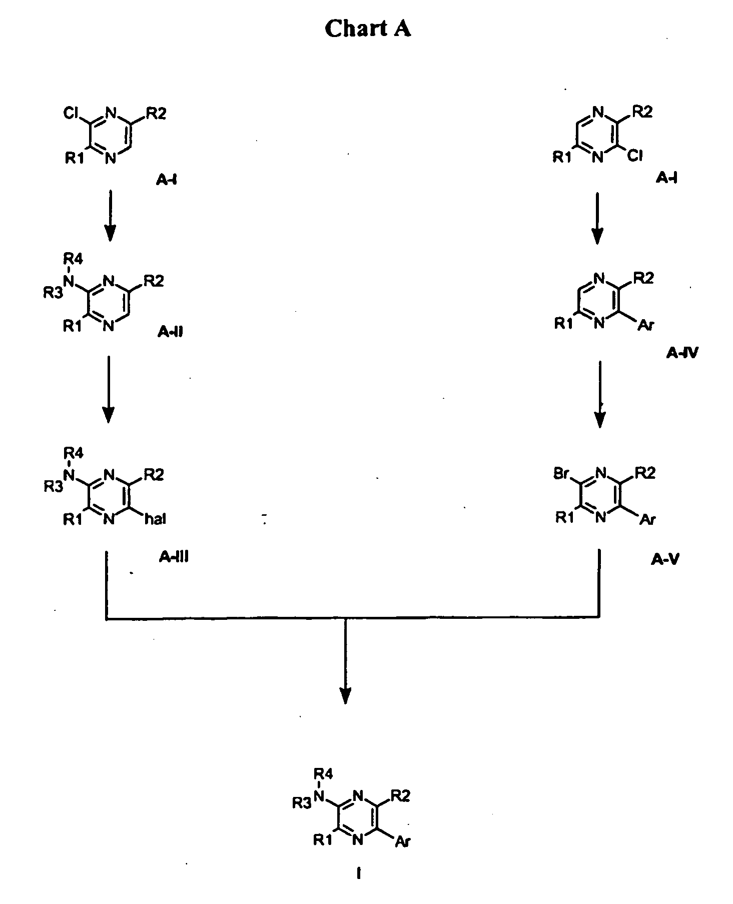

Another way of preparing the compounds of this invention is illustrated in Chart B. Dialkyl-dichloropyrazines **B-I** (see Chart C) can serve as the starting point for sequential displacement of one chlorine with the appropriate secondary amine (as described in Chart A) followed by biaryl formation with a suitable metalloaryl reagent (as described in Chart A) to afford **I**. In some instances, this sequence can also be conducted in the opposite order, i.e. biaryl formation followed by nucleophilic displacement by a secondary amine.

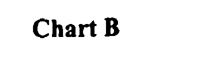

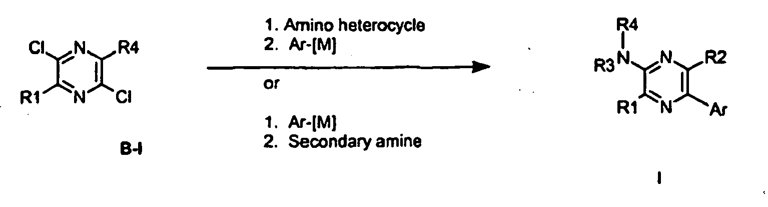

Chart C illustrates the preparation of mono- and dichloropyrazine **A-I** and **B-I** respectively when R1 and R2 are alkyl and the same. The reaction sequence shown below follows that described in Chemical and Pharmaceutical Bulletin of Japan, 1979,27,2027.

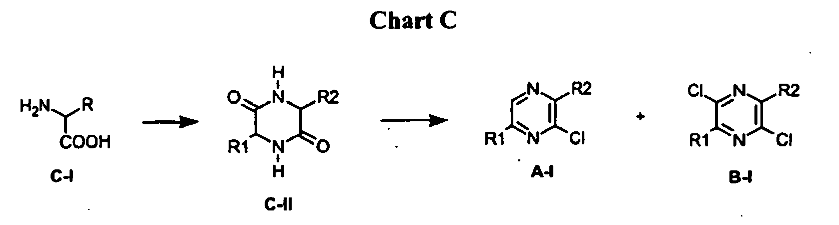

As illustrated in Chart D, treatment of A-V (depicted in Chart A) with an alkoxide or sodium or potassium salt of a thiol should afford compounds such as **D-1.** Alternatively, if direct alkoxide addition fails, palladium catalysis (see Buchwald, S.L. et al J. Am. Chem. Soc. 2001,123, 10770) or copper catalysis (see Fagan, P.J. et al J. Am. Chem. Soc. 2000, 122, 5043) of an alkoxide will provide the desired pyrazinyl aryl ether. Another literature method for forming aryl sulfur bonds is demonstrated by the work of Herradura et al. (see, Herradura, P.S. et al Org. Lett. 2000, 2, 2019).

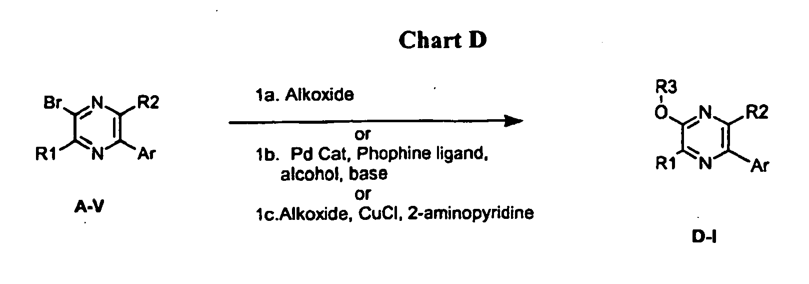

As illustrated in Chart E, treatment of A-V (depicted in Chart A) with a nucleophile such as an alkyl Grignard or alkyl lithium reagent would afford compounds such as **E-I**. Alternatively, treatment with an alkyl boronic acid (see Fu, G.C. et as J. Am. Chem. Soc. 2000,122, 4020.) under transition metal catalysis should also provide compounds like **E-I**.

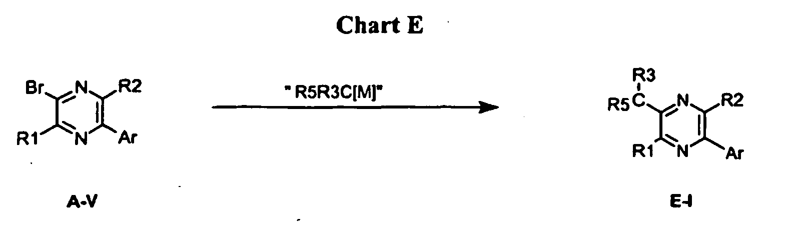

As illustrated in Chart F, reaction of an amine with a pyrazinyl chloride following the protocol of Buchwald et al (J. Org. Chem. 2000, 1158.) provides the desired aniline (**F-I**). Halogenation with NBS (or other suitable reagents as described above) and standard palladium mediated coupling provides the biaryl product **(F-III).** Alkylation with a base such as NaH and an alkyl halide affords the desired alkyl ethers (**F-IV**).

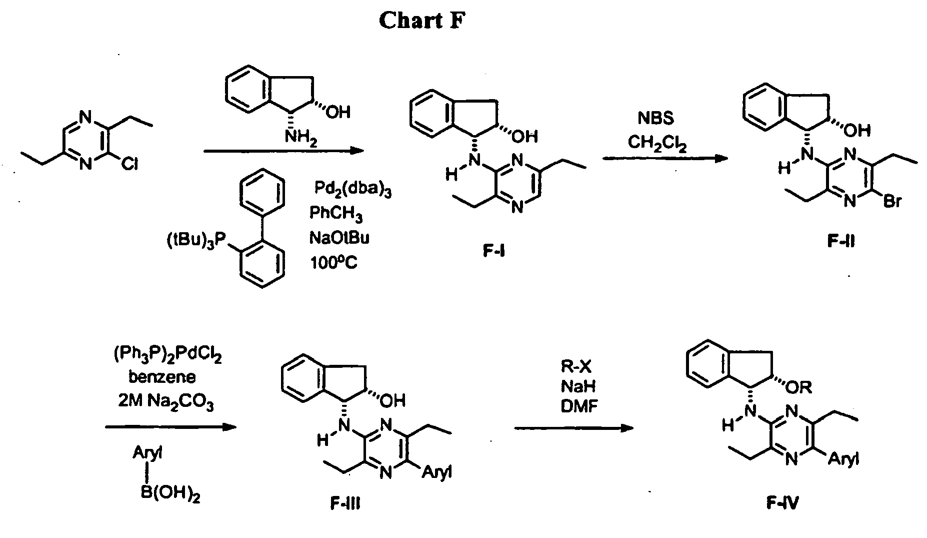

The preparation of unsymmetrically substituted pyrazines (R₁ # R₂) is shown in Chart G. The synthesis commences with the coupling of a suitably protected amino acid, such as **G-I**, to an N-protected amino acid, such as **G-II**, using methods known to those skilled in the art. The N-protecting group is removed from G-III to afford **G-IV**. Cyclization of **G-IV** to **G-V** and the conversion of **G-V** into the regioisomeric chloro-pyrazines **G-VI** and **G-VII** proceed through standard methods (see Chemical and Pharmaceutical Bulletin of Japan, 1979, 27,2027).

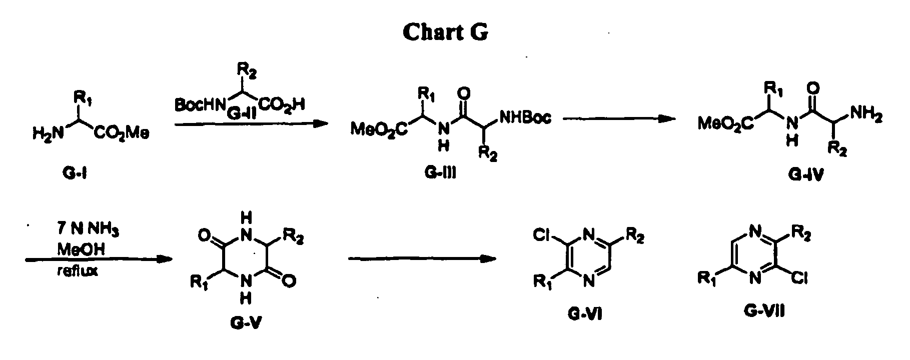

The present invention provides a pharmaceutical composition comprising a pharmaceutically acceptable carrier and a therapeutically effective amount of a compound of the invention useful for the treatment of a disorder described herein above in a mammal, particularly in a human.

In another aspect, the present invention provides a use of a compound of the invention for the preparation of a medicament for treating a disorder manifesting hypersecretion of CRF in a mammal.

In another aspect, the present invention provides a method of inhibiting the binding of CRF to a CRF₁ receptor, comprising contacting a compound of the invention with a solution comprising cells expressing the CRF₁ receptor, wherein the compound is present in the solution at a concentration sufficient to inhibit the binding of CRF to the CRF₁ receptor.

In another aspect, the present invention provides an article of manufacture comprising: a) a packaging material; b) a compound of the invention; and c) a label or package insert contained within said packaging material indicating that said compound is effective for treating a pre-selected disorder described herein above.

Compounds of the invention are useful for treating various disorders in a mammal, particularly in a human, such as social anxiety disorder, panic disorder; obsessive-compulsive disorder; anxiety with co-morbid depressive illness; affective disorder; anxiety; depression; irritable bowel syndrome; post-traumatic stress disorder; supranuclear palsy; immune suppression; gastrointestinal disease; anorexia nervosa or other feeding disorder; drug or alcohol withdrawal symptoms; substance abuse disorder (e.g., nicotine, cocaine, ethanol, opiates, or other drugs); inflammatory disorder, fertility problems; disorders the treatment of which can be effected or facilitated by antagonizing CRF, including but not limited to disorders induced or facilitated by CRF; a disorder selected from inflammatory disorders such as rheumatoid arthritis and osteoarthritis, pain, asthma, psoriasis and allergies; generalized anxiety disorder; panic, phobias, obsessive-compulsive disorder; post-traumatic stress disorder; sleep disorders induced by stress; pain perception such as fibromyalgia; mood disorders such as depression, including major depression, single episode depression, recurrent depression, child abuse induced depression, and postpartum depression; dysthemia; bipolar disorders; cyclothymia; fatigue syndrome; stress-induced headache; cancer, human immunodeficiency virus (HIV) infections; neurodegenerative diseases such as Alzheimer's disease, Parkinson's disease and Huntington's disease; gastrointestinal diseases such as ulcers, irritable bowel syndrome, Crohn's disease, spastic colon, diarrhea, and post operative ilius and colonic hypersensitivity associated by psychopathological disturbances or stress; eating disorders such as anorexia and bulimia nervosa; hemorrhagic stress; stress-induced psychotic episodes; euthyroid sick syndrome; syndrome of inappropriate antidiarrhetic hormone (ADH); obesity; infertility; head traumas; spinal cord trauma; ischemic neuronal damage (e.g., cerebral ischemia such as cerebral hippocampal ischemia); excitotoxic neuronal damage; epilepsy; cardiovascular and hear related disorders including hypertension, tachycardia and congestive heart failure; stroke; immune dysfunctions including stress induced immune dysfunctions (e.g., stress induced fevers, porcine stress syndrome, bovine shipping fever, equine paroxysmal fibrillation, and dysfunctions induced by confinement in chickens, sheering stress in sheep or human-animal interaction related stress in dogs); muscular spasms; urinary incontinence; senile dementia of the Alzheimer's type; multiinfarct dementia; amyotrophic lateral sclerosis; chemical dependencies and addictions (e.g., dependences on alcohol, cocaine, heroin, benzodiazepines, or other drugs); osteoporosis; psychosocial dwarfism and hypoglycemia.

Thus, in another aspect, the present invention provides use of a compound of the invention for treating a disorder described herein above in a mammal, particularly in a human.

Particular disorders that can be treated by a compound or use of the invention preferably include the following: affective disorder; anxiety; depression; irritable bowel syndrome; post-traumatic stress disorder; supranuclear palsy; obsessive-compulsive disorder; anxiety with co-morbid depressive illness; Alzheimer's disease; gastrointestinal disease; skin disorders (e.g., acne, psoriasis); anorexia nervosa; social anxiety disorder; bulimia nervosa or other feeding disorder; drug (e.g., dependencies on cocaine, heroin, benzodiazepines, nicotine or other drugs) or alcohol withdrawal symptoms; substance abuse disorder (e.g., nicotine, cocaine, ethanol, opiates, or other drugs); inflammatory disorder; disorders; the treatment of which can be effected or facilitated by antagonizing CRF, including but not limited to disorders induced or facilitated by CRF; or a disorder selected from inflammatory disorders such as rheumatoid arthritis and osteoarthritis, pain, asthma, psoriasis and allergies; generalized anxiety disorder; panic disorder; phobias; obsessive-compulsive disorder; sleep disorders induced by stress; pain perception such as fibromyalgia; mood disorders such as depression, including major depression, single episode depression, recurrent depression, child abuse induced depression, and postpartum depression; dysthymia; bipolar disorders; cyclothymia; fatigue syndrome; stress-induced headache; cancer; neurodegenerative diseases such as, Parkinson's disease and Huntington's disease; gastrointestinal diseases such as ulcers, Crohn's disease, spastic colon, diarrhea, and post operative ilius and colonic hypersensitivity associated by psychopathological disturbances or stress; stress-induced psychotic episodes; syndrome of inappropriate antidiarrhetic hormone (ADH); cardiovascular and hear related disorders including hypertension, tachycardia and congestive heart failure; stroke; senile dementia of the Alzheimer's type; multiinfarct dementia; amyotrophic lateral sclerosis.

Particular disorders that can be treated by a compound or use of the invention more preferably include the following: generalized anxiety disorder; social anxiety disorder; anxiety; obsessive-compulsive disorder; anxiety with co-morbid depressive illness; panic disorder; mood disorders such as depression, including major depression, single episode depression, recurrent depression, child abuse induced depression, and postpartum depression; bipolar disorders; post-traumatic stress disorder; substance abuse disorder (e.g., nicotine, cocaine, ethanol, opiates, or other drugs); inflammatory disorders such as rheumatoid arthritis and osteoarthritis; gastrointestinal diseases such as irritable bowel syndrome, ulcers, Crohn's disease, spastic colon, diarrhea, and post operative ilius and colonic hypersensitivity associated by psychopathological disturbances or stress; inflammatory disorder; and skin disorders such as acne and psoriasis.

Particular disorders that can be treated by a compound or use of the invention even more preferably include the following: generalized anxiety disorder; social anxiety disorder, anxiety; obsessive-compulsive disorder, anxiety with co-morbid depressive illness; panic disorder, mood disorders such as depression, including major depression, single episode depression, recurrent depression, child abuse induced depression, and postpartum depression; bipolar disorders; and post-traumatic stress disorder.

A compound of this invention can be administered to treat these abnormalities in a mammal or human by means that produce contact of the active agent with the agent's site of action in the body of the mammal or human. The compounds can be administered by any conventional means available for use in conjunction with pharmaceuticals either as individual therapeutic agent or in combination of therapeutic agents. It can be administered alone, but will generally be administered with a pharmaceutical carrier selected on the basis of the chosen route of administration and standard pharmaceutical practice.

The dosage administered will vary depending on the use and known factors such as pharmacodynamic character of the particular agent, and its mode and route of administration; the recipient's age, weight, and health; nature and extent of symptoms; kind of concurrent treatment; frequency of treatment; and desired effect.

For use in the treatment of said diseases or conditions, a compound of this invention can be orally administered at a dosage of the active ingredient of 0.002 to 200 mg/kg of body weight. Ordinarily, a dose of 0.01 to 10 mg/kg in divided doses one to four times a day, or in sustained release formulation will be effective in obtaining the desired pharmacological effect.

Dosage forms (compositions) suitable for administration contain from about 1 mg to about 100 mg of active ingredient per unit. In these pharmaceutical compositions, the active ingredient will ordinarily be present in an amount of about 0.5 to 95% by weight based on the total weight of the composition.

The active ingredient can be administered orally in solid dosage forms, such as capsules, tablets and powders; or in liquid forms such as elixirs, syrups, and/or suspensions. The compounds of this invention can also be administered parenterally in sterile liquid dose formulations. Gelatin capsules can be used to contain the active ingredient and a suitable carrier such as but not limited to lactose, starch, magnesium stearate, steric acid, or cellulose derivatives. Similar diluents can be used to make compressed tablets. Both tablets and capsules can be manufactured as sustained release products to provide for continuous release of medication over a period of time. Compressed tablets can be sugar coated or film-coated to mask any unpleasant taste, or used to protect the active ingredients from the atmosphere, or to allow selective disintegration of the tablet in the gastrointestinal tract. Liquid dose forms for oral administration can contain coloring or flavoring agents to increase patient acceptance. In general, water, pharmaceutically acceptable oils, saline, aqueous dextrose (glucose), and related sugar solutions and glycols, such as propylene glycol or polyethylene glycol, are suitable carriers for parenteral solutions. Solutions for parenteral administration preferably contain a water soluble salt of the active ingredient, suitable stabilizing agents, and if necessary, butter substances. Antioxidizing agents, such as sodium bisulfite, sodium sulfite, or ascorbic acid, either alone or in combination, are suitable stabilizing agents. Also used are citric acid and its salts, and EDTA. In addition, parenteral solutions can contain preservatives such as benzalkonium chloride, methyl- or propyl-paraben, and chlorobutanol. Suitable pharmaceutical carriers are described in "Remington's Pharmaceutical Sciences", A. Osol, a standard reference in the field.

Useful pharmaceutical dosage forms or administration of the compounds of this invention can be illustrated as follows: (1) Capsules. A large number of units capsules are prepared by filling standard two-piece hard gelatin capsules each with 100 mg of powdered active ingredient, 150 mg lactose, 50 mg cellulose, and 6 mg magnesium stearate. (2) Soft Gelatin Capsules. A mixture of active ingredient in a digestible oil such as soybean, cottonseed oil, or olive oil is prepared and injected by means of a positive displacement was pumped into gelatin to form soft gelatin capsules containing 100 mg of the active ingredient. The capsules were washed and dried. (3) Tablets. A large number of tablets are prepared by conventional procedures so that the dosage unit was 100 mg active ingredient, 0.2 mg of colloidal silicon dioxide, 5 mg of magnesium stearate, 275 mg of microcrystalline cellulose, 11 mg of starch, and 98.8 mg lactose. Appropriate coatings may be applied to increase palatability or delayed adsorption.

The compounds of this invention may also be used as reagents or standards in the biochemical study of neurological function, dysfunction, and disease.

### DEFINITIONS AND CONVENTIONS

The term "substituted aryl" means an aryl group optionally substituted with 1-5 substituents independently selected from halogen, -NO₂, -CN, -Rₐ, -ORₐ, -S(O)ₘRₐ, - NRₐRₐ, -C(O)NRₐRₐ, -C(S)NRₐRₐ -S(O)ₘNRₐRₐ, -NRₐS(O)ₘRₐ, -NRₐC(O)ORₐ, - OC(O)NRₐRₐ, -NRaC(O)NRₐRₐ, -NRₐC(S)NRₐRₐ, -C(O)ORₐ, -C(S)ORₐ, and - OC(O)ORa;

The term "aryl cycloalkyl" means a bicyclic ring system containing 8 to 14 carbon atoms wherein one ring is aryl and the other ring is fused to the aryl ring and may be fully or partially saturated in the portion of the ring fused to the aryl ring, provided that either ring may act as a point of attachment;

The term "substituted aryl cycloalkyl" means an aryl cycloalkyl group having 1-5 substituents independently selected from halogen, -NO₂,-CN, -Rₐ, -ORₐ, - S(O)ₘRₐ, -NRₐRₐ, -C(O)NRₐRₐ, -C(S)NRₐRₐ -S(O)ₘNRₐRₐ, -NRₐS(O)ₘRₐ, - NRₐC(O)ORₐ, -OC(O)NRₐRₐ, -NRₐC(O)NRₐRₐ, -NRₐC(S)NRₐRₐ, -C(O)ORₐ, - C(S)ORₐ, and -OC(O)ORₐ;

The term "heteroaryl cycloalkyl" means a bicyclic ring system containing 8 to 14 atoms, wherein one ring is heteroaryl and the other ring is fused to the heteroaryl ring and may be fully or partially saturated in the portion of the ring fused to the heteroaryl ring, provided that either ring may act as a point of attachment;

The term "substituted heteroaryl cycloalkyl" means a heteroaryl cycloalkyl group having 1-5 substituents independently selected from halogen, -NO₂, -CN, -Rₐ, - ORₐ, -S(O)ₘRₐ, -NRₐRₐ, -C(O)NRₐRₐ, -C(S)NRₐRₐ -S(O)ₘNRₐRₐ, -NRₐS(O)ₘRₐ, - NRₐC(O)ORₐ, -OC(O)NRₐRₐ, -NRₐC(O)NRₐRₐ, -NRₐC(S)NRₐRₐ, -C(O)ORₐ, - C(S)ORₐ, and -OC(O)ORₐ;

The term "aryl heterocycloalkyl" means a bicyclic ring system containing 8 to 14 atoms, wherein one ring is aryl and the other ring is heterocycloalkyl, provided that either ring may act as a point of attachment;

The term "substituted aryl heterocycloalkyl" means an aryl heterocycloalkyl group having 1-5 substituents independently selected from halogen, -NO₂, -CN, -Rₐ, - ORₐ, -S(O)ₘRₐ, -NRₐRₐ, -C(O)NRₐRₐ, -C(S)NRₐRₐ -S(O)ₘNRₐRₐ, -NRₐS(O)ₘRₐ, - NRₐC(O)ORₐ, -OC(O)NRₐRₐ, -NRₐC(O)NRₐRₐ, -NRₐC(S)NRₐRₐ, -C(O)ORₐ, - C(S)ORₐ, and -OC(O)ORₐ;

The term "heteroaryl heterocycloalkyl" means a bicyclic ring system containing 8 to 14 atoms, wherein one ring is heteroaryl and the other ring is heterocycloalkyl, provided that either ring may act as a point of attachment;

The term "substituted heteroaryl heterocycloalkyl" means an heteroaryl heterocycloalkyl group having 1-5 substituents independently selected from halogen, - NO₂, -CN, -Rₐ, -ORₐ, -S(O)ₘRₐ, -NRₐRₐ, -C(O)NRₐRₐ, -C(S)NRₐRₐ -S(O)ₘNRₐRₐ, - NRₐS(O)ₘRa,-NRₐC(O)ORₐ, -OC(O)NRₐRₐ, -NRₐC(O)NRₐRₐ, -NRₐC(S)NRₐRₐ, - C(O)ORₐ, -C(S)ORₐ, and -OC(O)ORₐ;

The term "heteroaryl" means a radical attached via a ring carbon or nitrogen atom of a monocyclic aromatic ring containing five or six ring atoms consisting of carbon and 1, 2, 3, or 4 heteroatoms each selected from the group consisting of non-peroxide O, S, N, with appropriate bonding to satisfy valence requirements as well as a radical (attachment at either carbon or nitrogen) of a fused bicyclic heteroaromatic of about eight to ten ring atoms, and includes radicals such as thienyl, benzothienyl, pyridyl, thiazolyl, quinolyl, pyrazinyl, pyrimidyl, imidazolyl, furanyl, benzofuranyl, benzothiazolyl, isothiazolyl, benzisothiazolyl, benzisoxazolyl, benzimidazolyl, indolyl, and benzoxazolyl, pyrazolyl, triazolyl, tetrazolyl, isoxazolyl, oxazolyl, pyrrolyl, isoquinolinyl, cinnolinyl, indazolyl, indolizinyl, phthalazinyl, pydridazinyl, triazinyl, isoindolyl, purinyl, oxadiazolyl, furazanyl, benzofurazanyl, benzothiophenyl, benzothiazolyl, quinazolinyl, quinoxalinyl, naphthridinyl, and furopyridinyl;

The term "substituted heteroaryl" means a heteroarylgroup having 1-5 substituents independently selected from halogen, -NO₂, -CN, -Rₐ, -ORₐ, -S(O)ₘRₐ, - NRₐRₐ, -C(O)NRₐRₐ, -C(S)NRₐRₐ -S(O)ₘNRₐRₐ, -NRₐS(O)ₘRₐ, -NRₐC(O)ORₐ, - OC(O)NRₐRₐ, -NRₐC(O)NRₐRₐ, -NRₐC(S)NRₐRₐ, -C(O)ORₐ, -C(S)ORₐ, and - OC(O)ORₐ

The term "heterocycloalkyl", unless otherwise specified, means a 4 to 8 membered monocyclic ring or bicyclic ring, wherein at least one carbon atom is replaced with a heteromember selected from oxygen, nitrogen, -NH-, or -S(O)ₘ-wherein m is zero, 1, or 2, optionally containing from one to three double bonds, provided that the molecule is not aromatic; and provided that ring attachment can occur at either a carbon or nitrogen atom; Heterocycloalkyl includes tetrahydrofuranyl, tetrahydropyranyl, morpholinyl, pyrrolidinyl, piperidinyl, piperazinyl, [2.2.1]-azabicyclic rings, [2.2.2]-azabicyclic rings, [3.3.1]-azabicyclic rings, quinuclidinyl, azetidinyl, azetidinonyl, oxindolyl, dihydroimidazolyl, and pyrrolidinonyl

The term "substituted heterocycloalkyl" is a heterocycloalkyl group having 1-5 substituents independently selected from halogen, -NO₂, -CN, -Rₐ, -ORₐ, -S(O)ₘRₐ, - NRₐRₐ, -C(O)NRₐRₐ, -C(S)NRₐRₐ -S(O)ₘNRₐRₐ, -NRₐS(O)ₘRₐ, -NRₐC(O)ORₐ, - OC(O)NRₐRₐ, -NRₐC(O)NRₐRₐ, -NRₐC(S)NRₐRₐ, -C(O)ORₐ, -C(S)ORₐ, and - OC(O)ORₐ;

Halogen is a group selected from -F, -Cl, -Br, and -I;

The term "alkyl" means both straight and branched chain moieties having from 1-10 carbon atoms optionally containing one or more double or triple bonds;

The term "cycloalkyl" means a monocyclic or bicyclic alkyl moiety, having from 3-10 carbon atoms optionally containing 1 to 2 double bonds provided that the moiety is not aromatic;

The term "haloalkyl" means an alkyl moiety having from 1-10 carbon atoms and having 1 to (2v+1) independently selected halogen substituent(s) where v is the number of carbon atoms in the moiety;

The term "pharmaceutically acceptable salt" refers to a salt prepared from pharmaceutically acceptable non- toxic acids, including inorganic acids and organic acids. Suitable non- toxic acids include inorganic and organic acids of basic residues such as amines, for example, acetic, benzenesulfonic, benzoic, amphorsulfonic, citric, ethenesulfonic, fumaric, gluconic, glutamic, hydrobromic, hydrochloric, isethionic, lactic, maleic, malic, mandelic, methanesulfonic, mucic, nitric, pamoic, pantothenic, phosphoric, succinic, sulfuric, barbaric acid, p-toluenesulfonic and the like; and alkali or organic salts of acidic residues such as carboxylic acids, for example, alkali and alkaline earth metal salts derived from the following bases: sodium hydride, sodium hydroxide, potassium hydroxide, calcium hydroxide, aluminum hydroxide, lithium hydroxide, magnesium hydroxide, zinc hydroxide, ammonia, trimethylammonia, triethylammonia, ethylenediamine, lysine, arginine, ornithine, choline, N,N'-dibenzylethylenediamine,chloroprocaine, diethanolamine, procaine, n-benzylphenethylamine, diethylamine, piperazine, tris(hydroxymethyl)-aminomethane, tetramethylammonium hydroxide, and the like. Pharmaceutically acceptable salts of the compounds of the invention can be prepared by reacting the free acid or base forms of these compounds with a stoichiometric amount of the appropriate base or acid in water or in an organic solvent, or in a mixture of the two; generally, nonaqueous media like ether, ethyl acetate, ethanol, isopropanol, or acetonitrile are preferred. Lists of suitable salts are found in Remin ton's Pharmaceutical Sciences, 17th ea., Mack Publishing Company, Easton, PA, 1985, p. 1418, the disclosure of which is hereby incorporated by reference.

The term "therapeutically effective amount" of a compound of this invention means an amount effective to antagonize abnormal level of CRF or treat the symptoms of affective disorder, anxiety, depression, or other disorders described herein above, in a host.

The term "compound of the invention" means a compound of Formulae IV, V, or VI, a stereoisomer thereof, or a pharmaceutically acceptable salt thereof.

### PREPARATIONS AND EXAMPLES

The invention is illustrated further by the following examples and preparations, which are not to be construed as limiting the invention in scope or spirit to the specific procedures described in them.

### EXAMPLE A.

### CRF₁ Receptor Binding Assay for the Evaluation of Biological Activity

The following is a description of the isolation of rat brain membranes for use in the standard binding assay as well as a description of the binding assay itself. It is based on a modified protocol described by De Souza (De Souza, 1987).

To prepare brain membranes for binding assays, rat frontal cortex is homogenized in 10 mL of ice cold tissue buffer (50 mM HEPES buffer pH 7.0, containing 10 mM MgCl₂, 2 mM EGTA, 1 µg/ml aprotinin, 1 µg/ml leupeptin and 1 µg/ml pepstatin). The homogenate is centrifuged at 48,000 x g for 10 min. and the resulting pellet rehomogenized in 10 mL of tissue buffer. Following an additional centrifugation at 48,000 x g for 10 min., the pellet is resuspended to a protein concentration of 300µg/mL.

Binding assays are performed in 96 well plates at a final volume of 300 µL. The assays are initiated by the addition of 150 µL membrane suspension to 150 µL of assay buffer containing ¹²⁵I-ovine-CRF (final concentration 150 pM) and various concentrations of inhibitors. The assay buffer is the same as described above for membrane preparation with the addition of 0.1% ovalbumin and 0.15 mM bacitracin. Radioligand binding is terminated after 2 hours at room temperature by filtration through Packard GF/C unifilter plates (presoaked with 0.3% polyethyleneimine) using a Packard cell harvestor. Filters are washed three times with ice cold phosphate buffered saline pH 7.0 containing 0.01% Triton X-100. Filters are assessed for radioactivity in a Packard TopCount. Nonspecific binding is determined in the presence of excess (10 µM α-helical CRF.

Alternatively, tissues and cells that naturally express CRF receptors, such as IMR-32 human neuroblastoma cells (ATCC; Hogg et al., 1996), can be employed in binding assays analogous to those described above.

IC₅₀ values are calculated using standard methods known in the art, such as with the non-linear curve fitting program RS/1 (BBN Software Products Corp., Cambridge, MA). A compound is considered to be active if it has an IC₅₀ value of less than about 10 micromolar (µM) for the inhibition of CRF₁ receptors. The binding affinity of the compounds of Formula I expressed as IC₅₀ values generally ranges from about 0.5 nanomolar to about 10 micromolar. Preferred compounds of Formula I exhibit IC₅₀ of 1 micromolar or less, more preferred compounds of Formula I exhibit IC₅₀ of less than 100 nanomolar or less, still more preferred compounds of Formula I exhibit IC₅₀ of less than 10 nanomolar or less.

### EXAMPLE B.

### Inhibition of CRF-Stimulated Adenylate Cyclase Activity

Inhibition of CRF-stimulated adenylate cyclase activity can be performed as previously described [G. Battaglia et al., Synapse 1:572 (1987)]. Briefly, assays are carried out at 37°C for 10 min in 200 mL of buffer containing 100 mM Tris-HCl (pH 7.4 at 37 °C), 10 mM MgCl₂, 0.4 mM EGTA, 0.1% BSA, 1 mM isobutylmethylxanthine (IBMX), 250 units/mL phosphocreatine kinase, 5 mM creatine phosphate, 100 mM guanosine 5'-triphosphate, 100 nM o-CRF, antagonist peptides (various concentrations) and 0.8 mg original wet weight tissue (approximately 40-60 mg protein). Reactions are initiated by the addition of 1 mM ATP/[³²P]ATP (approximately 2-4 mCi/tube) and terminated by the addition of 100 mL of 50 mM Tris-HCl, 45 mM ATP and 2% sodium dodecyl sulfate. In order to monitor the recovery of cAMP, 1 mL of [³H]cAMP (approximately 40,000 dpm) is added to each tube prior to separation. The separation of [³²P]cAMP from [³²P]ATP is performed by sequential elution over Dowex and alumina columns.

Alternatively, adenylate cyclase activity can be assessed in a 96-well format utilizing the Adenylyl Cyclase Activation FlashPlate Assay from NEN Life Sciences according to the protocols provided. Briefly, a fixed amount of radiolabeled cAMP is added to 96-well plates that are precoated with anti-cyclic AMP antibody. Cells or tissues are added and stimulated in the presence or absence of inhibitors. Unlabeled cAMP produced by the cells will displace the radiolabeled cAMP from the antibody. The bound radiolabeled cAMP produces a light signal that can be detected using a microplate scintillation counter such as the Packard TopCount. Increasing amounts of unlabeled cAMP results in a decrease of detectable signal over a set incubation time (2-24 hours).

### Preparation 1

### (1R,2S)-1-[(3,6-diethylpyrazin-2-yl)amino]-2,3-dihydro-1H-inden-2-ol

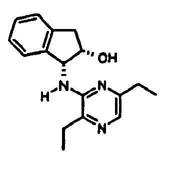

A solution of 3-chloro-2,5-diethylpyrazine (171 mg, 1.0 mmol), (1R,2S)-(+)-cis-1-amino-2-indanol (298 mg, 2.0 mmol), tris(dibenzylideneacetone)dipalladium (0) (28 mg, 0.03 mmol), and 2-(di-tertbutylphosphino)biphenyl (18 mg, 0.06 mmol) in toluene (2.0 mL) was purged with nitrogen and treated with sodium tertbutoxide (135 mg, 1.4 mmol). The resulting brown suspension was heated to 100 °C for 2 hours. At this time, the reaction was quenched with a saturated water solution of NaHCO₃ and extracted twice with ethyl acetate (20 mL). The combined organics were washed with brine (15 mL), dried over MgSO₄, filtered, and concentrated to give a black solid. This material was purified by biotage MPLC (40 g column, 25% ethyl acetate/heptane) to afford 184 mg (65%) of the title compound as a light purple solid. IR (diffuse reflectance) 3435, 3241, 2962, 2935, 2912, 2873, 1581, 1547, 1500, 1453, 1184, 1163, 1047, 744, 733 cm⁻¹ ; OAMS supporting ions at: ESI+ 384.0; MS (CI) *m*/*z* 284 (MH⁺);
HRMS (FAB) calcd for C₁₇H₂₁N₃O+H₁ 284.1763, found 284.1754. [α]²⁵_{D}= 12(*c* 0.55, methylene chloride); Anal. Calcd for C₁₇H₂₁N₃O: C, 72.06; H, 7.47; N, 14.83. Found: C, 72.15; H, 7.53; N, 14.42.

### Preparation 2

### (1R,2S)-1-[(5-bromo-3,6-diethylpyrazin-2-yl)amino]-2,3-dihydro-1H-inden-2-ol

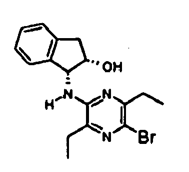

A solution of (1R,2S)-1-[(3,6-diethylpyrazin-2-yl)amino]-2,3-dihydro-1H-inden-2-ol (1.94 g, 6.8 mmol) in dichloromethane (35 mL) was purged with nitrogen and cooled to 0 °C. This light purple/grey homogenous solution was treated with a single portion of N-bromosuccinimide (1.34 g, 7.5 mmol) instantly becoming a lighter color. The reaction was warmed to room temperature for 15 minutes, transferred to a separatory funnel, diluted with 100 mL additional dichloromethane, washed twice with water (75 mL), and once with brine (75 mL). The organics were dried over MgSO₄ filtered, and concentrated to give a golden oil. This material was purified by biotage MPLC (90 g column, 15% ethyl acetate/heptane) to afford 2.24 g (90%) of the title compound as a pale yellow solid. IR (diffuse reflectance) 3416, 3355, 2969, 2941, 1555, 1538, 1482, 1385, 1378, 1295, 1285, 1200, 1181, 1043, 733 cm⁻¹; OAMS supporting ions at: ESI+ 361.9; MS (EI) *m*/*z* 361 (M⁺); [α]²⁵_{D} = -35 (c 0.53, methylene chloride); Anal. Calcd for C₁₇H₂₀BrN₃O: C, 56.36; H, 5.56; N, 11.60. Found: C, 56.46; H, 5.59; N, 11.50.

### Preparation 3

### (1R,2S)-1-{[5-(2,4-dichlorophenyl)-3,6-diethylpyrazin-2-yl]amino}-2,3-dihydro-1H-inden-2-ol

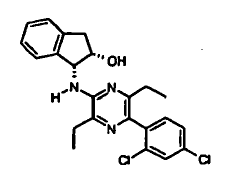

A solution of (1R,2S)-1-[(5-bromo-3,6-diethylpyrazin-2-yl)amino]-2,3-dihydro-1H-inden-2-ol (1.48 g, 4.12 mmol) and 2,4-dichlorophenyl boronic acid (858 mg, 4.5 mmol) in benzene (41 mL) and 2M sodium carbonate (7 mL) was purged with nitrogen and treated with a single portion of bistriphenylphosphinepalladium(II)chloride (287 mg, 0.41 mmol). The resulting golden 2-phase solution was heated to 80 °C gradually darkening in color. After 16 hours, the reaction was cooled to room temperature, transferred to a separatory funnel, and washed with water (50 mL). The aqueous layer was extracted twice with ethyl acetate (75 mL) and the combined organics were washed once with brine (75 mL), dried over MgSO₄, filtered, and concentrated to give 2.36 g of a brown syrup. This material was purified by biotage MPLC (90 g column, 20% ethyl acetate/heptane) to afford 1.25 g (71%) of the title compound as a tan solid. OAMS supporting ions at:
ESI+ 428.0; HRMS (FAB) calcd for C₂₃H₂₃CL₂N₃O +H₁ 428.1296, found 428.1292.

### Example 1

### The preparation of 5-(2,4-dichlorophenyl)-N-[(1R,2S)-2-ethoxy-2,3-dihydro-1H-inden-1-yl]-3,6-diethylpyrazin-2-amine

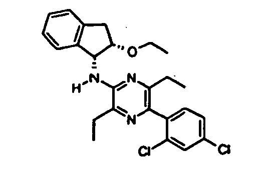

A solution of sodium hydride (60% oil dispersion, 26 mg, 0.65 mmol) was suspended in DMF (1.5 mL), purged with nitrogen, and treated with a single portion of (1R,2S)-1-{[5-(2,4-dichlorophenyl)-3,6-diethylpyrazin-2-yl]amino}-2,3-dihydro-1H-inden-2-ol (214 mg, 0.5 mmol) with copious gas evolution. The resulting green/golden solution was stirred for 5 minutes at room temperature and treated with iodoethane (88 µL, 1.1 mmol). After 3 hours, the reaction was poured into 20 mL 1:1 water/brine and extracted twice with ethyl acetate (20 mL). The combined organics were washed once with brine, dried over MgSO₄, filtered, and concentrated to give 389 mg of a golden oil. This material was purified by LC (18 g silica gel, 7% ethyl acetate/heptane) to afford 157 mg (69%) of the title compound as a pale yellow syrup. IR (liq.) 3446, 2972, 2935, 2896, 2874, 1565, 1552, 1498, 1470, 1391, 1206, 1184, 1101, 1091, 1080 cm⁻¹; OAMS supporting ions at: ESI+ 456.1; MS (EI) *m*/*z* 455 (M⁺); [α]²⁵_{D} = -94 (c 0.38, methylene chloride); Anal. Calcd for C₂₅H₂₇C₁₂N₃O: C, 65.79; H, 5.96; N, 9.21. Found: C, 66.06; H, 6.10; N, 9.17.

### Example 2

### The preparation of 5-(2,4-dichlorophenyl)-3,6-diethyl-N-[(1R,2S)-2-methoxy-2,3-dihydro- 1H-inden-1-yl]pyrazin-2-amine

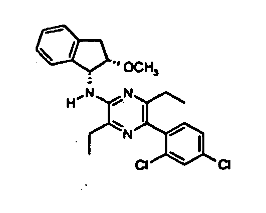

Following the procedure for the preparation of 5-(2,4-dichlorophenyl)-N-[(1R,2S)-2-ethoxy-2,3-dihydro-1H-inden-1-yl]-3,6-diethylpyrazin-2-amine but substituting iodomethane provided the title compound as colorless syrup. OAMS supporting ions at: ESI+ 442.0; MS (EI) *m*/*z* 441 (M⁺); HRMS (FAB) calcd for C₂₄H₂₅CL₂N₃O +H₁ 442.1453, found 442.1456.

### Example 3

### The preparation of 5-(2,4-dichlorophenyl)-3,6-diethyl-N-[(1R,2S)-2-isopropoxy-2,3-dihydro-1H-inden-1-yl]pyrazin-2-amine

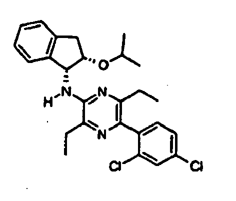

Following the procedure for the preparation of 5-(2,4-dichlorophenyl)-N-[(1R,2S)-2-ethoxy-2,3-dihydro- H-inden-1-yl]-3,6-diethylpyrazin-2-amine but substituting 2-iodopropane (20 equivalents iodide and sodium hydride with portion-wise addition) provided the title compound as a light yellow syrup. IR (liq.) 2971, 2935, 1564, 1552, 1497, 1471, 1392, 1379, 1368, 1206, 1177, 1140, 1123, 1101, 1061 cm⁻¹; OAMS. supporting ions at: ESI+ 470.2; MS (EI) *m*/*z* 469 (M⁺); HRMS (FAB) calcd for C₂₆H₂₉CL₂N₃O +H₁ 470.1766, found 470.1773. [α]²⁵_{D} = -93 (c 0.33, methylene chloride); Anal. Calcd for C₂₆H₂₉Cl₂N₃O: C, 66.38; H, 6.21; N, 8.93. Found: C, 66.53; H, 6.19; N, 8.89.

### Preparation 4

### Preparation of (1S,2R)-1-[(3,6-diethylpyrazin-2-yl)amino]-2,3-dihydro-1H-inden-2-ol

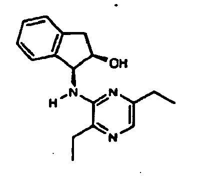

Following the procedure for the preparation of (1R,2S)-1-[(3,6-diethylpyrazin-2-yl)amino]-2,3-dihydro-1H-inden-2-ol but substituting (1S,2R)-(-)-cis-1-amino-2-indanol and making non-critical variations provided the title compound as a light purple solid. MS (ESI+) for C₁₇H₂₁N₃O *m*/*z* 284.0 (M+H)⁺.

### Preparation 5

### Preparation of (1S,2R)-1-[(5-bromo-3,6-diethylpyrazin-2-yl)amino]-2,3-dihydro-1H-inden-2-ol

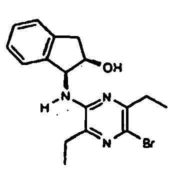

Following the procedure for the preparation of (1R,2S)-1-[(5-bromo-3,6-diethylpyrazin-2-yl)amino]-2,3-dihydro-1H-inden-2-ol but substituting (1S,2R)-1-[(3,6-diethylpyrazin-2-yl)amino]-2,3-dihydro-1H-inden-2-ol and making non-critical variations provided the title compound as a pale yellow solid. MS (ESI+) for C₁₇H₂₀BrN₃O *m*/*z* 361.9 (M+H)⁺.

### Preparation 6

### Preparation of (1S,2R)-1-{[5-(2,4-dichlorophenyl)-3,6-diethylpyrazin-2-yl]amino} - 2,3-dihydro-1H-inden-2-ol.

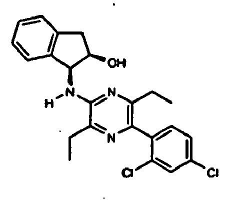

Following the procedure for the preparation of (1R,2S)-1-{[5-(2,4-dichlorophenyl)-3,6-diethylpyrazin-2-yl]amino}-2,3-dihydro-1H-inden-2-ol but substituting (1S,2R)-1-[(5-bromo-3,6-diethylpyrazin-2-yl)amino]-2,3-dihydro-1H-inden-2-ol and making non-critical variations provided the title compound as a tan solid. IR (diffuse reflectance) 3439, 2964, 1568, 1551, 1500, 1469, 1391, 1373, 1204, 1183, 1102, 1048, 819, 748, 741 cm⁻¹; OAMS supporting ions at: ESI+ 427.9; MS (EI) *m*/*z* 427 (M⁺); HRMS (FAB) calcd for C₂₃H₂₃CL₂N₃O +H₁, 428.1296, found 428.1286. Anal. Calcd for C₂₃H₂;Cl₂N₃O: C, 64.49; H, 5.41; N, 9.81. Found: C, 64.43; H, 5.54; N, 9.42.

### Example 4

### Preparation of 5-(2,4-dichlorophenyl)-3,6-diethyl-N-[(1S,2R)-2-methoxy-2,3-dihydro-1 H-inden-1-yl]pyrazin-2-amine.

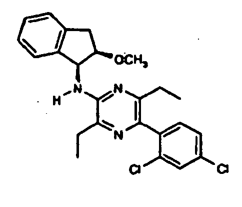

Following the procedure for the preparation of 5-(2,4-dichlorophenyl)-N-[(1R,2S)-2-ethoxy-2,3-dihydro-1H-inden-1-yl]-3,6-diethylpyrazin-2-amine but substituting (1S,2R)-1-{[5-(2,4-dichlorophenyl)-3,6-diethylpyrazin-2-yl]amino}-2,3-dihydro-1H-inden-2-ol and iodomethane and making non-critical variations provided the title compound as a colorless syrup. IR (liq.) 3447, 2972, 2934, 2907, 2877, 1589, 1565, 1552, 1498, 1471, 1391, 1375, 1196, 1177, 1093 cm⁻¹; OAMS supporting ions at: ESI+ 442.0; MS (EI) *m*/*z* 441 (M⁺); [α]²⁵_{D} = 55 (c 0.51, methanol); Anal. Calcd for C₂₄H₂₅Cl₂N₃O: C, 65.16; H, 5.70; N, 9.50. Found: C, 65.20; H, 5.63; N, 9.43.

### Preparation 7

### Preparation of (1S,2S)-1-{[5-(2,4-dichlorophenyl)-3,6-diethylpyrazin-2-yl]amino}-2,3-dihydro-1H-inden-2-yl 4-nitrobenzoate.

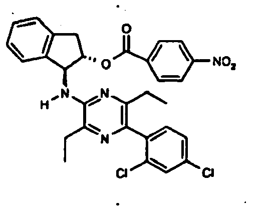

A solution of (1S,2R)-1-{[5-(2,4-dichlorophenyl)-3,6-diethylpyrazin-2-yl]amino)-2,3-dihydro-1H-inden-2-ol (321 mg, 0.75 mmol), 4-nitrobenzoic acid (552 mg, 3.3 mmol), and triphenylphosphine (964 mg, 3.7 mmol) in benzene (19 mL) was purged with nitrogen and cooled to 0°C. The resulting yellow suspension was treated with diethylazodicarboxylate (0.58 mL, 3.7 mmol) becoming lighter in color with gradual warming to RT. After 18 hours, the volatiles were removed under reduced pressure with the resulting crude residue being absorbed on 6 g silica gel and purified by Biotage MPLC (40 g column, 15% ethyl acetate/heptane) to afford the title compound as a light yellow solid. IR (diffuse reflectance) 1725, 1568, 1550, 1527, 1498, 1467, 1392, 1372, 1349, 1320, 1273, 1119, 1103, 836, 719 cm⁻¹; OAMS supporting ions at: ESI+ 576.8; MS (CI) *m*/*z* 577(MH⁺); HRMS (FAB) calcd for C₃₀H₂₆CL₂N₄O₄ +H₁ 577.1409, found 577.1417.

### Preparation 8

### Preparation of (1S,2S)-1-{[5-(2,4-dichlorophenyl)-3,6-diethylpyrazin-2-yl]amino}-2,3-dihydro-1H-inden-2-ol.

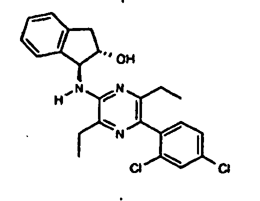

A solution of (1S,2S)-1-([5-(2,4-dichlorophenyl)-3,6-diethylpyrazin-2-yl]arnino)-2,3-dihydro-1H-inden-2-yl 4-nitrobenzoate (369 mg, 0.64 mmol) in methanol (3 mL) and tetrahydrofuran (6 mL) was treated with a 1M solution of lithium hydroxide (5 mL, 5 mmol). After 16 hrs, the volatiles were removed under reduced pressure and the aqueous residue was diluted with 1M sodium hydroxide (10 mL) and extracted twice with ethyl acetate (20 mL). The combined organics were washed once with brine (20 mL), dried over MgSO₄, filtered, and concentrated to give 302 mg of a yellow syrup. This material was purified by Biotage MPLC (40 g column, 20% ethyl acetate/heptane) to afford 250 mg (91%) of the title compound as a white solid. IR (diffuse reflectance) 3325, 2976, 2936, 2900, 2876, 1571, 1550, 1503, 1471, 1449, 1435, 1399, 861, 819, 753 cm⁻¹; OAMS supporting ions at: ESI+ 427.9; MS (EI) *m*/*z* 427 (M⁺); [α]²⁵_{D} =-108 (c 0.58, methylene chloride); Anal. Calcd for C₂₃H₂₃Cl₂N₃O: C, 64.49; H, 5.41; N, 9.81; Cl, 16.55.
Found: C, 64.27; H, 5.46; N, 9.58.

### Example 5 and 6

### Preparation of 5-(2,4-dichlorophenyl)-3,6-diethyl-N-[(1S,2S)-2-methoxy-2,3-dihydro-1 H-inden-1-yl]pyrazin-2-amine and 5-(2,4-dichlorophenyl)-3,6-diethyl-N-[(1S,2S)-2-methoxy-2,3-dihydro-1H-inden-1-yl]-N-methylpyrazin-2-amine.

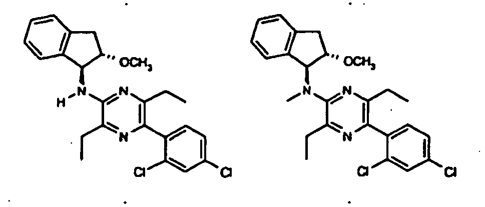

Following the procedure for the preparation of 5-(2,4-dichlorophenyl)-N-[(1R,2S)-2-ethoxy-2,3-dihydro-1H-inden-1-yl]-3,6-diethylpyrazin-2-amine but substituting (1S,2S)-1-{[5-(2,4-dichlorophenyl)-3,6-diethylpyrazin-2-yl]amino}-2,3-dihydro-1H-inden-2-ol, and iodomethane and making non-critical variations provided the title compounds. Analytical data for 5-(2,4-dichlorophenyl)-3,6-diethyl-N-[(1S,2S)-2-methoxy-2,3-dihydro-1H-inden-1-yl]pyrazin-2-amine; IR (diffuse reflectance) 2965, 1569, 1551, 1500, 1468, 1397, 1393, 1372, 1203, 1189, 1106, 988, 838, 819, 748 cm⁻¹; OAMS supporting ions at: ESI+ 442.1; MS (EI) m/z 441 (M⁺); Anal. Calcd for C₂₄H₂₅Cl₂N₃O: C, 65.16; H, 5.70; N, 9.50; Cl, 16.03. Found: C, 65.14; H, 5.90; N, 9.32. Analytical data for 5-(2,4-dichlorophenyl)-3,6-diethyl-N-[(1S,2S)-2-methoxy-2,3-dihydro-1H-inden-1-yl]-N-methylpyrazin-2-amine; OAMS supporting ions at: ESI+ 455.8; HRMS (FAB) calcd for C₂₅H₂₇CL₂N₃O +H₁ 456.1609, found 456.1601.

### Preparation 9

### Preparation of (1R,2R)-1-{[5-(2,4-dichlorophenyl)-3,6-diethylpyrazin-2-yl]amino} - 2,3-dihydro-1H-ioden-2-yl 4-nitrobenzoate.

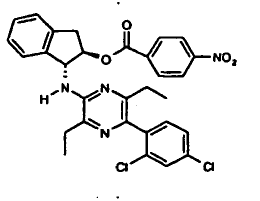

Following the procedure for the preparation of (1S,2S)-1-{[5-(2,4-dichlorophenyl)-3,6-diethylpyrazin-2-yl]amino}-2,3-dihydro-1H-inden-2-yl 4-nitrobenzoate but substituting (1R,2S)-1-[(3,6-diethylpyrazin-2-yl)amino]-2,3-dihydro-1H-inden-2-01 and making non-critical variations provided the title compound as a yellow solid. IR (diffuse reflectance) 1725, 1568, 1550, 1527, 1498, 1467, 1392, 1372, 1349, 1320, 1273, 1119, 1103, 1014, 719 cm⁻¹; OAMS supporting ions at: ESI+ 577.1; MS (CI) *m*/*z* 577 (MH⁺); HRMS (FAB) calcd for C₃₀H₂₆CL₂N₄O₄ +H₁, 577.1409, found 577.1393; [α]²⁵_{D} =-118 (c 0.62, methylene chloride).

### Preparation 10

### Preparation of (1R,2R)-1-{[5-(2,4-dichlorophenyl)-3,6-diethylpyrazin-2-yl]amino}-2,3-dihydro-1H-inden-2-ol.

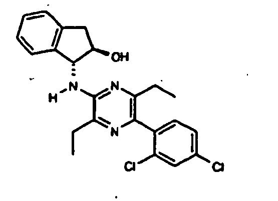

Following the procedure for the preparation of (1S,2S)-1-([5-(2,4-dichlorophenyl)-3,6-diethylpyrazin-2-yl]amino}-2,3-dihydro-1H-inden-2-ol but substituting (1R,2R)-1- {[5-(2,4-dichlorophenyl)-3,6-diethylpyrazin-2-yl]amino}-2,3-dihydro-1H-inden-2-yl 4-nitrobenzoate and making non-critical variations provided the title compound as an off-white solid. IR (diffuse reflectance) 3326, 2976, 1572, 1550, 1503, 1472, 1449, 1435, 1399, 1358, 1198, 1074, 862, 819, 753 cm⁻¹; OAMS supporting ions at: ESI+ 428.1; MS (EI) *m*/*z* 427 (M⁺); HRMS (FAB) calcd for C₂₃H₂₃CL₂N₃O +H₁ 428.1296, found 428.1295. [α]²⁵_{D} = 112 (c 0.58, methylene chloride).
Anal. Calcd for C₂₃H₂₃Cl₂N₃O: C, 64.49; H, 5.41; N, 9.81. Found: C, 64.60; H, 5.34; N, 9.75.

### Example 7 and 8

### Preparation of 5-(2,4-dichlorophenyl)-3,6-diethyl-N-[(1R,2R)-2-methoxy-2,3-dihydro-1H-inden-1-yl]pyrazin-2-amine and 5-(2,4-dichlorophenyl)-3,6-diethyl-N-[(1R,2R)-2-methoxy-2,3-dihydro-1H-inden-1-yl]-N-methylpyrazin-2-amine.

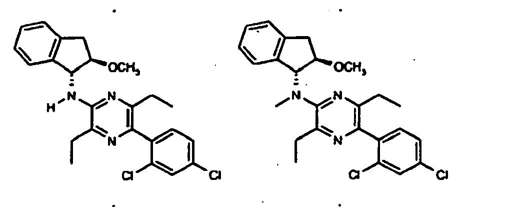

Following the procedure for the preparation of 5-(2,4-dichlorophenyl)-N-[(1R,2S)-2-ethoxy-2,3-dihydro-1H-inden-1-yl]-3,6-diethylpyrazin-2-amine but substituting (1R,2R)-1-{[5-(2,4-dichlorophenyl)-3,6-diethylpyrazin-2-yl]amino}-2,3-dihydro-1H-inden-2-ol and iodomethane and making non-critical variations provided the title compounds. Analytical data for 5-(2,4-dichlorophenyl)-3,6-diethyl-N-[(1R,2R)-2-methoxy-2,3-dihydro-1H-inden-1-yl]pyrazin-2-amine: IR (diffuse reflectance) 3382, 2963, 2933, 1568, 1550, 1500, 1469, 1396, 1371, 1203, 1189, 1105, 988, 838, 748 cm⁻¹; OAMS supporting ions at: ESI+ 441.1; MS (EI) *m*/*z* 441 (M⁺); HRMS (FAB) calcd for C₂₄H₂₅CL₂N₃O+H₁ 442.1453, found 442.1455. [α]²⁵_{D} = -76 (c 0.64, methylene chloride). Analytical data for 5-(2,4-dichlorophenyl)-3,6-diethyl-N-[(1R,2R)-2-methoxy-2,3-dihydro-1H-inden-1-yl]-N-methylpyrazin-2-amine: OAMS supporting ions at: ESI+ 456.1.

### Example 9

### Preparation of N-[(1R,2S)-2-(cyclopropylmethoxy)-2,3-dihydro-1H-inden-1-yl]-5-(2,4-dichlorophenyl)-3,6-diethylpyrazin-2-amine.

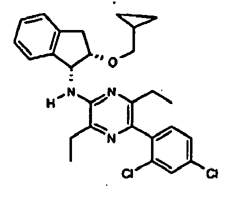

Following the procedure for the preparation of 5-(2,4-dichlorophenyl)-N-[(1R,2S)-2-ethoxy-2,3-dihydro- 1H-inden-1-yl]-3,6-diethylpyrazin-2-amine but substituting (bromomethyl)cyclopropane and making non-critical variations provided the title compound as a yellow amorphous solid. IR (liq.) 3442, 2971, 2935, 2874, 1565, 1552, 1498, 1471, 1393, 1206, 1184, 1101, 1077, 1021, 740 cm⁻¹; OAMS supporting ions at: ESI+ 481.8; MS (EI) *m*/*z* 481 (M⁺); HRMS (FAB) calcd for C₂₇H₂₉CL₂N₃O +H₁, 482.1766, found 482.1775. [α]²⁵_{D} = -65 (c 0.73, methylene chloride); Anal. Calcd for C₂₇H₂₉Cl₂N₃O: C, 67.22; H, 6.06; N, 8.71. Found: C, 67.15; H, 6.08; N, 8.66.

### Example 10

### Preparation of 5-(2,4-dichlorophenyl)-3,6-diethyl-N-[(1R,2S)-2-(prop-2-ynyloxy)-2,3-dihydro-1H-inden-1-yl]pyrazin-2-amine.

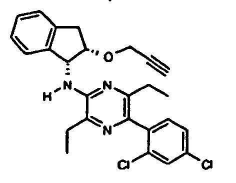

Following the procedure for the preparation of 5-(2,4-dichlorophenyl)-N-[(1R,2S)-2-ethoxy-2,3-dihydro-1H-inden-1-yl]-3,6-diethylpyrazin-2-amine but substituting propargylbromide and making non-critical variations provided the title compound as a white solid. IR (diffuse reflectance) 3451, 3293, 2964, 2932, 1563, 1493, 1469, 1390, 1204, 1184, 1087, 1076, 820, 742, 646 cm⁻¹; OAMS supporting ions at: ESI+ 465.8; MS (EI) *m*/*z* 465 (M⁺); HRMS (FAB) calcd for C₂₆H₂₅CL₂N₃O+H₁ 466.1453, found 466.1455. [α]²⁵_{D} = -56 (c 0.83, methylene chloride).
Anal. Calcd for C₂₆H₂₅Cl₂N₃O: C, 66.96; H, 5.40; N, 9.01. Found: C, 66.96; H, 5.49; N, 8.93.

### Example 11

### Preparation of 5-(2,4-dichlorophenyl)-3,6-diethyl-N-[(1R,2S)-2-(2-methoxyethoxy)-2,3-dihydro-1H-inden-1-yl]pyrazin-2-amine.

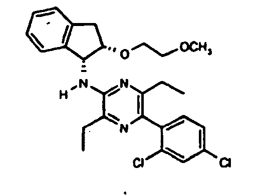

Following the procedure for the preparation of 5-(2,4-dichlorophenyl)-N-[(1R,2S)-2-ethoxy-2,3-dihydro-1H-inden-1-yl]-3,6-diethylpyrazin-2-amine but substituting 2-bromoethylmethylether and making non-critical variations provided the title compound as a light yellow syrup. IR (liq.) 3418, 2971, 2934, 2875, 1565, 1552, 1498,1471, 1392, 1376, 1201, 1183, 1132, 1100, 748 cm⁻¹; OAMS supporting ions at: ESI+ 485.8; MS (CI) *m*/*z* 486 (MH⁺); [α]²⁵_{D} =102 (c 0.57, methylene chloride); Anal. Calcd for C₂₆H₂₉Cl₂N₃O₂: C, 64.20; H, 6.01; N, 8.64. Found: C, 63.92; H, 6.14; N, 8.53.

### Example 12

### Preparation of N-[(1R,2S)-2-(allyloxy)-2,3-dihydro-1H-inden-1-yl]-5-(2,4-dichlorophenyl)-3,6-diethylpyrazin-2-amine.

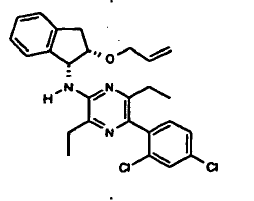

Following the procedure for the preparation of 5-(2,4-dichlorophenyl)-N-[(1R,2S)-2-ethoxy-2,3-dihydro-1H-inden-1-yl]-3,6-diethylpyrazin-2-amine but substituting allybromide and making non-critical variations provided the title compound as a light yellow semi-solid. IR (liq.) 3446, 2972, 2935, 2874, 1565, 1552, 1497, 1471, 1392, 1206, 1181, 1101, 1087, 1078, 744 cm⁻¹; OAMS supporting ions at: ESI+ 467.9; MS (EI) *m*/*z* 467 (M⁺); [α]²⁵_{D} = -73 (c 0.79, methylene chloride); Anal. Calcd for C₂₆H₂₇Cl₂N₃O: C, 66.67; H, 5.81; N, 8.97. Found: C, 66.49; H, 5.87; N, 8.91.

### Example 13

### Preparation of 5-(2,4-dichlorophenyl)-3,6-diethyl-N-[(1R,2S)-2-(2-fluoroethoxy)-2,3-dihydro-1H-inden-1-yl]pyrazin-2-amine.

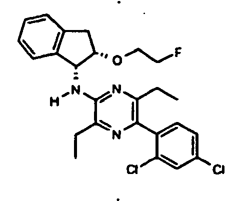

Following the procedure for the preparation of 5-(2,4-dichlorophenyl)-N-[(1R,2S)-2-ethoxy-2,3-dihydro-1H-inden-1-yl]-3,6-diethylpyrazin-2-amine but substituting 1-bromo-2-fluoroethane and making non-critical variations provided the title compound as a light yellow amorphous solid. IR (diffuse reflectance) 2972, 2934, 1564, 1551, 1498, 1468, 1392, 1377, 1206, 1182, 1123, 1102, 1046, 826, 750 cm⁻¹; OAMS supporting ions at: ESI+ 473.9; MS (EI) *m*/*z* 473 (M⁺); [α]²⁵_{D} = -87 (c 0.77, methylene chloride); Anal. Calcd for C₂₅H₂₆Cl₂FN₃O: C, 63.30; H, 5.52; N, 8.86. Found: C, 63.10; H, 5.60; N, 8.79.

### Example 14

### Preparation of 5-(2,4-dichlorophenyl)-3,6-diethyl-N-[(1R,2S)-2-propoxy-2,3-dihydro-1H-inden-1-yl]pyrazin-2-amine.

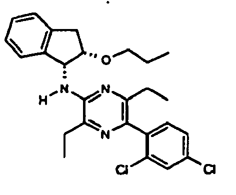

Following the procedure for the preparation of 5-(2,4-dichlorophenyl)-N-[(1 R,2S)-2-ethoxy-2,3-dihydro-1H-inden-1-yl]-3,6-diethylpyrazin-2-amine but substituting 1-iodopropane and making non-critical variations provided the title compound as a light yellow syrup. IR (liq.) 3446, 2967, 2935, 2875, 1564, 1552, 1497, 1470, 1392, 1206, 1184, 1101, 1091, 1082, 748 cm⁻¹; OAMS supporting ions at: ESI+ 469.9; MS (EI) *m*/*z* 469 (M⁺); [α]²⁵_{D}=-98 (c 0.91, methylene chloride); Anal. Calcd for C₂₆H₂₉Cl₂N₃O: C, 66.38; H, 6.21; N, 8.93. Found: C, 66.53; H, 6.15; N, 9.05.

### Example 15

### Preparation of 2-[((1R,2S)-1-{[5-(2,4-dichlorophenyl)-3,6-diethylpyrazin-2-yl]amino}-2,3-dihydro-1H-inden-2-yl)oxy]ethanol.

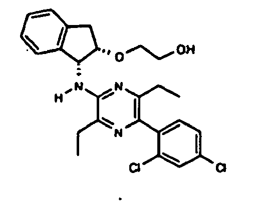

Following the procedure for the preparation of 5-(2,4-dichlorophenyl)-N-[(1R,2S)-2-ethoxy-2,3-dihydro-1H-inden-1-yl]-3,6-diethylpyrazin-2-amine but substituting 2-(bromoethoxy)-tertbutyldimethylsilane and making non-critical variations provided N-[(1R,2S)-27(2-{[tert-butyl(dimethyl)silyl]oxy}ethoxyl)-2,3-dihydro-1H-inden-1-yl]-5-(2,4-dichlorophenyl)-3,6-diethylpyrazin-2-amine. The crude product was treated with 5% hydrochloric acid in ethanol for 18 hrs followed by removal of the volatiles under reduced pressure. The resulting residue was basified with IN sodium hydroxide and extracted twice with ethyl acetate. The combined organics were washed once with brine, dried over MgSO₄, filtered, and concentrated to give a tan syrup. This material was purified by Biotage MPLC (40 g column, 25% ethyl acetate/heptane) to afford 67 mg of the title compound as a white solid. IR (diffuse reflectance) 2969, 2933, 1567, 1551, 1498, 1468, 1392, 1373, 1206, 1182, 1101, 1079, 1061, 1046, 749 cm⁻¹; OAMS supporting ions at: ESI+ 472.2; MS (EI) *m*/*z* 471 (M⁺); HRMS (FAB) calcd for C₂₅H₂₇CL₂N₃O₂ +H₁ 472.1558, found 472.1560. [α]²⁵_{D} = -73 (c 0.50, methylene chloride); Anal. Calcd for C₂₅H₂₇Cl₂N₃O₂: C, 63.56; H, 5.76; N, 8.89. Found: C, 63.23; H, 5.91; N, 8.75.

### Example 16

### Preparation of (1R,2S)-1-{[5-(2,4-dichlorophenyl)-3,6-diethylpyrazin-2-yl]amino}-2,3-dihydro-1H-inden-2-yl dimethylcarbamate.

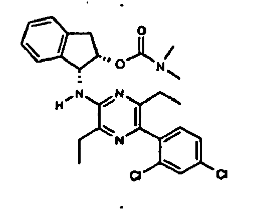

Following the procedure for the preparation of 5-(2,4-dichlorophenyl)-N-[(1R,2S)-2-ethoxy-2,3-dihydro- 1H-inden-1-yl]-3,6-diethylpyrazin-2-amine but substituting dimethylcarbamylchloride and making non-critical variations provided the title compound as a white solid. IR (diffuse reflectance) 3374, 2965, 1694, 1568, 1551, 1503, 1469, 1397, 1354, 1218, 1205, 1182, 765, 759, 751 cm⁻¹; OAMS supporting ions at: ESI+ 499.1; MS (EI) *m*/*z* 498 (M⁺); HRMS (FAB) calcd for C₂₆H₂₈CL₂N₄O₂ +H₁ 499.1667, found 499.1684. [α]²⁵_{D} = 63 (c 0.71, methylene chloride); Anal. Calcd for C₂₆H₂₈Cl₂N₄O₂: C, 62.53; H, 5.65; N, 11.22. Found: C, 62.37; H, 5.74; N, 11.09.

### Example 17

### Preparation of (1R,2S)-1 -{[5-(2,4-dichlorophenyl)-3,6-diethylpyrazin-2-yl]amino}-2,3-dihydro-1H-inden-2-yl acetate.

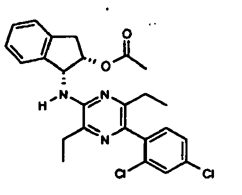

A solution of (1R,2S)-1-{[5-(2,4-dichlorophenyl)-3,6-diethylpyrazin-2-yl]amino}-2,3-dihydro-1H-inden-2-ol (214 mg, 0.5 mmol) and pyridine (44 µL, 0.55 mmol) in dichloromethane (5 mL) was purged with nitrogen and cooled to 0°C. The light yellow homogenous solution was treated with acetyl chloride (34 µL, 0.48 mmol) with no visible change. The reaction mixture was gradually warmed to RT. After 16 hrs, the volatiles were removed under reduced pressure and the resulting residue was absorbed on 4 g silica gel and purified by Biotage MPLC (40 g column, 15% ethyl acetate/heptane) to afford the title compound as an off-white solid. IR (diffuse reflectance) 2971, 2934, 1743, 1568, 1550, 1498, 1467, 1393, 1372, 1238, 1207, 1177, 1101, 1036, 751 cm⁻¹; OAMS supporting ions at: ESI+ 470.1; MS (EI) *m*/*z* 469 (M⁺); HRMS (FAB) calcd for C₂₅H₂₅CL₂N₃O₂ +H₁ 470.1402, found 470.1404. [α]²⁵_{D} = 73 (c 0.60, methylene chloride); Anal. Calcd for C₂₅H₂₅Cl₂N₃O₂: C, 63.83; H, 5.36; N, 8.93. Found: C, 63.45; H, 5.40; N, 8.79.

### Preparation 11

### Preparation of (1R,2S)-1-([5-(2-chloro-4-methoxyphenyl)-3,6-diethylpyrazin-2-yl]amino}-2,3-dihydro-1H-inden-2-ol

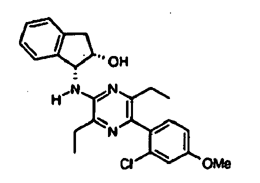

Following the procedure for the preparation of (1R,2S)-1-{[5-(2,4-dichlorophenyl)-3,6-diethylpyrazin-2-yl]amino}-2,3-dihydro-1H-inden-2-ol but substituting 2-chloro-4-methoxyphenylboronic acid and making non-critical variations provided the title compound as an off-white amorphous solid. IR (diffuse reflectance) 2969, 2934, 1604, 1568, 1550, 1482, 1448, 1439, 1392, 1287, 1228, 1203, 1180, 1045, 740 cm⁻¹; OAMS supporting ions at: ESI+ 423.9; MS (CI) *m*/*z* 424 (MH⁺); HRMS (FAB) calcd for C₂₄H₂₆CLN₃O₂ +H₁ 424.1792, found 424.1789. Anal. Calcd for C₂₄H₂₆ClN₃O₂: C, 68.00; H, 6.18; N, 9.91. Found: C, 67.86; H, 6.29; N, 9.79.

### Example 18

### Preparation of 5-(2-chloro-4-methoxyphenyl)-N-[(1R,25)-2-ethoxy-2,3-dihydro-1H-inden-1-yl]-3,6-diethylpyrazin-2-amine.

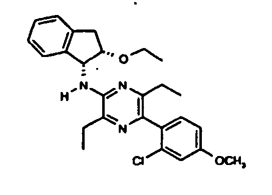

Following the procedure for the preparation of 5-(2,4-dichlorophenyl)-N-[(1R,2S)-2-ethoxy-2,3-dihydro-1H-inden-1-yl]-3,6-diethylpyrazin-2-amine but substituting (1R,2S)-1-{[5-(2-chloro-4-methoxyphenyl)-3,6-diethylpyrazin-2-yl]amino}-2,3-dihydro-1H-inden-2-ol and making non-critical variations provided the title compound as a pale yellow semi-solid. IR (liq.) 2971, 2935, 2875, 1606, 1565, 1480, 1440, 1391, 1287, 1230, 1206, 1183, 1091, 1079, 1044 cm⁻¹; OAMS supporting ions at: ESI+ 452.1; MS (EI) *m*/*z* 451 (M⁺); MS (FAB) calcd for C₂₆H₃₀CLN₃O₂ +H₁ 452.2104, found 452.2100. [α]²⁵_{D} = -91 (c 0.39, methylene chloride); Anal. Calcd for C₂₆H₃₀ClN₃O₂: C, 69.09; H, 6.69; N, 9.30. Found: C, 69.04; H, 6.74; N, 9.30.

### Example 19

### Preparation of 5-(2-chloro-4-methoxyphenyl)-3,6-diethyl-N-[(1R,2S)-2-(2-fluoroethoxy)-2,3-dihydro-1H-inden-1-yl]pyrazin-2-amine.

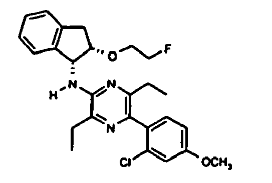

Following the procedure for the preparation of 5-(2,4-dichlorophenyl)-N-[(1R,2S)-2-ethoxy-2,3-dihydro-1H-inden-1-yl]-3,6-diethylpyrazin-2-amine but substituting (1R,2S)-1-{[5-(2-chloro-4-methoxyphenyl)-3,6-diethylpyrazin-2-yl]amino}-2,3-dihydro-1H-inden-2-ol and 1-bromo-2-fluoroethane and making non-critical variations provided the title compound as a pale yellow semi-solid. IR (diffuse reflectance) 2970, 1604, 1564, 1481, 1439, 1391, 1286, 1228, 1204, 1182, 1123, 1109, 1044, 876, 750 cm⁻¹ ; OAMS supporting ions at: ESI+ 470.1; MS (EI) *m*/*z* 469 (M⁺); HRMS (FAB) calcd for C₂₆H₂₉CLFN₃O₂ +H₁ 470.2010, found 470.2013. [α]²⁵_{D} = -82 (c 0.39, methylene chloride); Anal. Calcd for C₂₆H₂₉ClFN₃O₂: C, 66.45; H, 6.22; N, 8.94. Found: C, 66.41; H, 6.37; N, 8.84.

### Example 20

### Preparation of (1R,2S)-1-{[5-(2-chloro-4-methoxyphenyl)-3,6-diethylpyrazin-2-yl]amino}-2,3-dihydro-1H-inden-2-yl acetate.

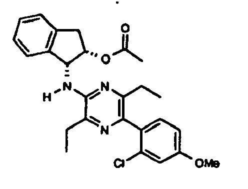

Following the procedure for the preparation of (1R,2S)-1-{[S-(2,4-dichlorophenyl)-3,6-diethylpyrazin-2-yl]amino}-2,3-dihydro-1H-inden-2-yl acetate but substituting (1R,2S)-1-{[5-(2-chloro-4-methoxyphenyl)-3,6-diethylpyrazin-2-yl]amino}-2,3-dihydro-1H-inden-2-ol and making non-critical variations provided the title compound as a light yellow semi-solid. IR (diffuse reflectance) 2969, 2933, 1742, 1604, 1568, 1551, 1482, 1439, 1393, 1372, 1286, 1232, 1204,1176, 1037 cm⁻¹; OAMS supporting ions at: ESI+ 465.2; MS (EI) *m*/*z* 465 (M⁺); [α]²⁵_{D} = 79 (c 0.80, methylene chloride); Anal. Calcd for C₂₆H₂₈ClN₃O₃: C, 67.02; H, 6.06; N, 9.02. Found: C, 66.99; H, 6.18; N, 8.92.

### Preparation 12

### Preparation of (1R,2S)-1-{[3,6-diethyl-5-(4-methoxy-2-methylphenyl)pyrazin-2-yl]amino}-2,3-dihydro-1H-inden-2-ol.

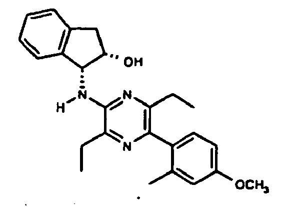

Following the procedure for the preparation of (1R,2S)-1-{[5-(2,4-dichlorophenyl)-3,6-diethylpyrazin-2-yl]amino}-2,3-dihydro-1H-inden-2-ol but substituting 2-methyl-4-methoxyphenylboronic acid and making non-critical variations provided the title compound as a peach colored foam. IR (diffuse reflectance) 3439, 2968, 2933, 2875, 1608, 1564, 1482, 1391, 1294, 1279, 1242, I204, 1175, 1050, 742 cm⁻¹; OAMS supporting ions at: ESI+ 404.0 & ESI- 402.1; MS (EI) *m*/*z-*403 (M⁺); HRMS (FAB) calcd for C₂₅H₂₉N₃O₂+H₁ 404.2338, found 404.2324. Anal. Calcd for C₂₅H₂₉N₃O₂: C, 74.41; H, 7.24; N, 10.41. Found: C, 74.05; H, 7.44; N, 10.28.

### Example 21

### Preparation of N-[(1R,2S)-2-ethoxy-2,3-dihydro-1H-inden-1-yl]-3,6-diethyl-5-(4-methoxy-2-methylphenyl)pyrazin-2-amine.

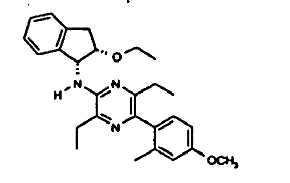

Following the procedure for the preparation of 5-(2,4-dichlorophenyl)-N-[(1R,2S)-2-ethoxy-2,3-dihydro-1H-inden-1-yl]-3,6-diethylpyrazin-2-amine but substituting (1R,2S)-1-{[3,6-diethyl-5-(4-methoxy-2-methylphenyl)pyrazin-2-yl]amino}-2,3-dihydro-1H-inden-2-ol and making non-critical variations provided the title compound as a light yellow semi-solid. IR (liq.) 2971, 2934, 1609, 1562, 1480, 1391, 1294, 1243,1206, 1184, 1171, 1161, 1119,1091, 1054 cm⁻¹; OAMS supporting ions at: ESI+ 432.1; MS (EI) *m*/*z* 431 (M⁺); HRMS (FAB) calcd for C₂₇H₃₃N₃O₂ +H₁ 432.2651, found 432.2650. Anal. Calcd for C₂₇H₃₃N₃O₂: C, 75.14; H, 7.71; N, 9.74. Found: C, 74.86; H, 7.72; N, 9.67.

### Example 22

### Preparation of 3,6-diethyl-N-[(1R,2S)-2-(2-fluoroethoxy)-2,3-dihydro-1H-inden-1-yl]-5-(4-methoxy-2-methylphenyl)pyrazin-2-amine.

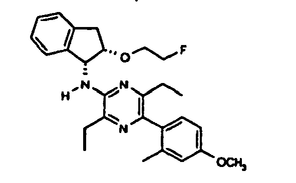

Following the procedure for the preparation of 5-(2,4-dichlorophenyl)-N-[(1R,2S)-2-ethoxy-2,3-dihydro-1H-inden-1-yl]-3,6-diethylpyrazin-2-amine but substituting (1R,2S)-1-{[3,6-diethyl-5-(4-methoxy-2-methylphenyl)pyrazin-2-yl]amino-2,3-dihydro-1H-inden-2-ol and 1-bromo-2-fluoroethane and making non-critical variations provided the title compound as a pale yellow semi-solid. IR (liq.) 2969, 2935, 1609, 1563, 1482, 1391, 1294, 1243, 1206, 1182, 1172, 1159, 1124, 1110, 1047 cm⁻¹; OAMS supporting ions at: ESI+ 450.0; MS (CI) *m*/*z* 450 (MH⁺); HRMS (FAB) calcd for C₂₇H₃₂FN₃O₂ +H₁ 450.2556, found 450.2544. Anal. Calcd for C₂₇H₃₂FN₃O₂: C, 72.14; H, 7.17; N, 9.35. Found: C, 72.24; H, 7.20; N, 9.33.

### Preparation 13

### Preparation of (1R,2S)-1-{[5-(2,4-dimethoxyphenyl)-3,6-diethylpyrazin-2-yl]amino}-2,3-dihydro-1H-inden-2-ol.

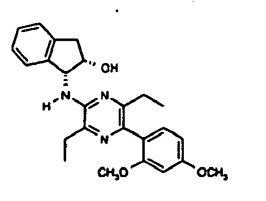

Following the procedure for the preparation of (1R,2S)-1-{[S-(2,4-dichlorophenyl)-3,6-diethylpyrazin-2-yl]amino}-2,3-dihydro-1H-inden-2-ol but substituting 2,4-dimethoxyphenylboronic acid and making non-critical variations provided the title compound as an off white foam. IR (diffuse reflectance) 2966, 2934, 1610, 1567, 1483, 1391, 1303, 1280, 1253, 1208, 1159, 1128, 1046, 1034, 741 cm⁻¹; OAMS supporting ions at: ESI+ 420.1; MS (EI) *m*/*z* 419 (M⁺); HRMS (FAB) calcd for C₂₅H₂₉N₃O₃ +H₁ 420.2287, found 420.2278.

### Example 23

### Preparation of 5-(2,4-dimethoxyphenyl)-N-[(1R,2S)-2-ethoxy-2,3-dihydro-1H-inden-1-yl]-3,6-diethylpyrazin-2-amine.

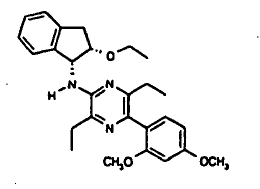

Following the procedure for the preparation of 5-(2,4-dichlorophenyl)-N-[(1R,2S)-2-ethoxy-2,3-dihydro-1 H-inden-1-yl]-3,6-diethylpyrazin-2-amine but substituting (1R,2S)-1-{[5-(2,4-dimethoxyphenyl)-3,6-diethylpyrain-2-yl]amino}-2,3-dihydro-1H-inden-2-ol and making non-critical variations provided the title compound as a white solid, m.p. 120-121 °C (uncorrected). IR (diffuse reflectance) 2965, 2935, 1615, 1566, 1480, 1390, 1305, 1256, 1215, 1209, 1183, 1165, 1040, 825, 747 cm⁻¹; OAMS supporting ions at: ESI+ 448.1; MS (EI) *m*/*z* 447 (M⁺); HRMS (FAB) calcd for C₂₇H₃₃N₃O₃ +H₁ 448.2600, found 448.2600. Anal. Calcd for C₂₇H₃₃N₃O₃: C, 72.46; H, 7.43; N, 9.39. Found: C, 72.46; H, 7.59; N, 9.39.

### Example 24

### Preparation of 5-(2,4-dimethoxyphenyl)-3,6-diethyl-N-[(1R,2S)-2-(2-fluoroethoxy)-2,3-dihydro-1H-inden-1-yl]pyrazin-2-amine.

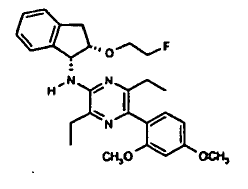

Following the procedure for the preparation of 5-(2,4-dichlorophenyl)-N-[(1 R,2S)-2-ethoxy-2,3-dihydro-1H-inden-1-yl]-3,6-diethylpyrazin-2-amine but substituting (1R,2S)-1-{[5-(2,4-dimethoxyphenyl)-3,6-diethylpyrazin-2-yl]amino}-2,3-dihydro-1H-inden-2-ol and 1-bromo-2-fluoroethane and making non-critical variations provided the title compound as a white solid. IR (diffuse reflectance) 2964, 2935, 1614, 1567, 1480, 1392, 1305, 1256, 1215, 1209, 1165, 1158, 1040, 825, 747 cm⁻¹; OAMS supporting ions at: ESI+ 466.2; MS (EI) *m*/*z* 465 (M⁺); Anal. Calcd for C₂₇H₃₂FN₃O₃: C, 69.66; H, 6.93; N, 9.03. Found: C, 69.61; H, 7.02; N, 8.98.

### Preparation 14

### Preparation of (1R,2S)-1-({5-[2-chloro-4-(dimethylamino)phenyl]-3,6-diethylpyrazin-2-yl}amino)-2,3-dihydro-1H-inden-2-ol.

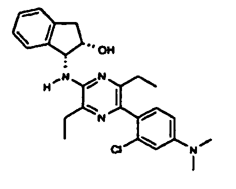

Following the procedure for the preparation of (1R,2S)-1-{[5-(2,4-dichlorophenyl)-3,6-diethylpyrazin-2-yl]amino}-2,3-dihydro-1H-inden-2-ol but substituting 2-chloro-4-dimethylaminophenylboronic acid and making non-critical variations provided the title compound as a pale yellow amorphous solid. IR (diffuse reflectance) 2969, 2932, 1607, 1567, 1550, 1486, 1443, 1391, 1353, 1225, 1207, 1177, 1043, 961, 740 cm⁻¹; OAMS ESI+ 437.2; MS (EI) *m*/*z* 436 (M⁺); HRMS (FAB) calcd for C₂₃H₂₉CLN₄O +H₁ 437.2108, found 437.2089. [α]²⁵_{D} = -7 (*c* 0.70, methylene chloride); Anal. Calcd for C₂₅H₂₉ClN₄O: C, 68.72; H, 6.69; N, 12.82. Found: C, 68.48; H, 6.84; N, 12.66.

### Example 25

### Preparation of 5-[2-chloro-4-(dimethylamino)phenyl]-N-[(1R,2S)-2-ethoxy-2,3-dihydro-1H-inden-1-yl]-3,6-diethylpyrazin-2-amine.

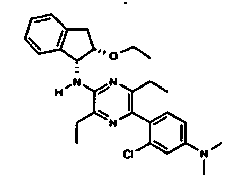

Following the procedure for the preparation of 5-(2,4-dichlorophenyl)-N-[(1R,2S)-2-ethoxy-2,3-dihydro-1H-inden-1-yl]-3,6-diethylpyrazin-2-amine but substituting (1R,2S)-1-((5-[2-chloro-4-(dimethylamino)phenyl]-3,6-diethylpyrazin-2-yl}amino)-2,3-dihydro-1H-inden-2-ol and making non-critical variations provided the title compound as a pale yellow amorphous solid. IR (diffuse reflectance) 2971, 2932, 2890, 2875, 1608, 1563, 1482, 1442, 1389, 1366, 1352, 1207, 1175, 1090, 961 cm⁻¹; OAMS supporting ions at: ESI+ 465.2; MS (EI) *m*/*z* 464 (M⁺); HRMS (FAB) calcd for C₂₇H₃₃CLN₄O+H₁ 465.2421, found 465.2406. Anal. Calcd for C₂₇H₃₃ClN₄O: C, 69.74; H, 7.15; N, 12.05. Found: C, 69.68; H, 7.16; N, 11.99.

### Example 26

### Preparation of 5-[2-chloro-4-(dimethylamino)phenyl]-3,6-diethyl-N-[(1R,2S)-2-(2-fluoroethoxy)-2,3-dihydro- H-inden-1-yl]pyrazin-2-amine.

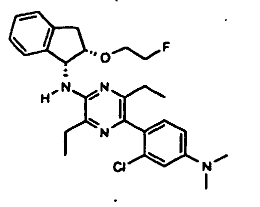

Following the procedure for the preparation of 5-(2,4-dichlorophenyl)-N-[(1 R,2S)-2-ethoxy-2,3-dihydro-1 H-inden-1-yl]-3,6-diethylpyrazin-2-amine but substituting (1R,2S)-1-({5-[2-chloro-4-(dimethylamino)phenyl]-3,6-diethylpyrazin-2-yl}amino)-2,3-dihydro-1H-inden-2-ol and 1-bromo-2-fluoroethane and making non-critical variations provided the title compound as a light yellow amorphous solid. IR (diffuse reflectance) 2969, 2932, 2907, 2877, 1608, 1563, 1545, 1483, 1447, 1392, 1353, 1207, 1177, 1123, 1042 cm⁻¹; OAMS supporting ions at: ESI+ 483.2; MS (EI) *m*/*z* 482 (M⁺); HRMS (FAB) calcd for C₂₇H₃₂CLFN₄O +H₁ 483.2327, found 483.2324. [a]²⁵_{D} = -79 (c 0.52, methylene chloride); Anal. Calcd for C₂₇H₃₂ClFN₄O: C, 67.14; H, 6.68; N, 11.60. Found: C, 67.19; H, 6.90; N, 11.63.

### Preparation 15

### Preparation of (1R,2S)-1-({5-[6-(dimethylamino)-4-methylpyridin-3-yl]-3,6-diethylpyrazin-2-yl}amino)-2,3-dihydro-1H-inden-2-ol.

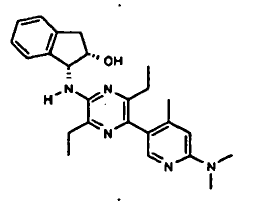

Following the procedure for the preparation of (1R,2S)-1-{[5-(2,4-dichlorophenyl)-3,6-diethylpyrazin-2-yl]amino}-2,3-dihydro-1H-inden-2-ol but substituting 6-(dimethylamino)-4-methylpyridin-3-ylboronic acid and making non-critical variations provided the title compound as a light yellow amorphous solid. IR (diffuse reflectance) 3448, 2962, 2954, 2933, 1611, 1567, 1520, 1482, 1392, 1376, 1174, 1160, 1046, 741, 737 cm⁻¹; OAMS supporting ions at: ESI+ 418.3; MS (EI) m/z 417 (M⁺); HRMS (FAB) calcd for C₂₅H₃₁N₅O +H₁ 418.2607, found 418.2611. Anal. Calcd for C₂₅H₃₁N₅O: C, 71.91; H, 7.48; N, 16.77. Found: C, 71.59; H, 7.57; N, 16.40.

### Example 27

### Preparation of 5-[6-(dimethylamino)-4-methylpyridin-3-yl]-N-[(1R,2S)-2-ethoxy-2,3-dihydro-1 H-inden-1=yl]-3,6-diethylpyrazin-2-amine.

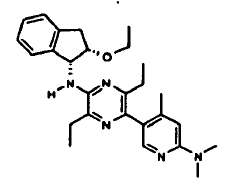

Following the procedure for the preparation of 5-(2,4-dichlorophenyl)-N-[(1R,2S)-2-ethoxy-2,3-dihydro-1H-inden-1-yl]-3,6-diethylpyrazin-2-amine but substituting (1R,2S)-1-({5-[6-(dimethylamino)-4-methylpyridin-3-yl]-3,6-diethylpyrazin-2-yl}amino)-2,3-dihydro-1H-inden-2-ol and making non-critical variations provided the title compound as a light golden amorphous solid. IR (diffuse reflectance) 3446, 2969, 2931, 2893, 2874, 1604, 1562, 1517, 1487, 1392, 1372, 1208, 1174, 1123, 1089 cm⁻¹; OAMS supporting ions at: ESI+ 446.3; MS (EI) *m*/*z* 445 (M⁺); HRMS (FAB) calcd for C₂₇H₃₅N₅O+H₁ 446.2920, found 446.2915. [α]²⁵_{D} = -89 (c 0.47, methylene chloride); Anal. Calcd for C₂₇H₃₅N₅O: C, 72.78; H, 7.92; N, 15.72. Found: C, 72.58; H, 7.94; N, 15.59.

### Preparation 16

### Preparation of N-(2,3-dihydro-1H-inden-1-yl)-3,6-dimethylpyrazin-2-amine

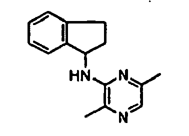

Following the procedure for the preparation of (1R,2S)-1-[(3,6-diethylpyrazin-2-yl)amino]-2,3-dihydro-1H-inden-2-ol but substituting 1-Aminoindane and 3-chloro-2,5-dimethylpyrazine, and making non-critical variations provided the title compound as a oil: ¹H NMR (CDCl₃) δ 1.88, 2.35, 2.41, 2.77, 2.94, 3.03, 4.55, 5.78, 7.3, 7.64; MS (ESI+) for C₁₅H₁₇N₃ *m*/*z* 240.30 (M+H)⁺.

### Preparation 17

### Preparation of 5-bromo-N-(2,3-dihydro-1H-inden-1-yl)-3,6-dimethylpyrazin-2-amine

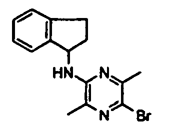

Following the procedure for the preparation of (1R,2S)-1-[(5-bromo-3,6-diethylpyrazin-2-yl)amino]-2,3-dihydro-1H-inden-2-ol but substituting N-(2,3-dihydro-1H-inden-1-yl)-3,6-dimethylpyrazin-2-amine and making non-critical variations provided the title compound as a oil: ¹H NMR (CDCl₃) δ 1.86, 2.26, 2.53, 2.70, 2.93, 3.03, 4.50, 5.72, 7.28; ¹³C NMR (CDCl₃) δ 19.23, 23.21, 30.25, 34.56, 56.45, 124.05, 124.81, 124.97, 126.78, 128.03, 136.98, 143.71, 144.09, 148.36, 151.15; MS (EST+) for C₁₅H₁₆N₃Br₁ *m*/*z* 319.20 (M+H)⁺.

### Example 28

### Preparation of 5-(2,4-dichlorophenyl)-N-(2,3-dihydro-1H-inden-1-yl)-3,6-dimethylpyrazin-2-amine

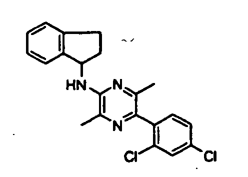

Following the procedure for the preparation of (1R,2S)-1-{[5-(2,4-dichlorophenyl)-3,6-diethylpyrazin-2-yl]amino}-2,3-dihydro-1H-inden-2-ol but substituting 5-bromo-N-(2,3-dihydro-1H-inden-1-yt)-3,6-dimethylpyrazin-2-amine and making non-critical variations provided the title compound as a oil: ¹H NMR (CDCl₃) 81.94, 2.29, 2.39, 2.80, 2.96, 3.08, 4.64, 5.83, 7.33, 7.51; MS (ESI+) for C₂₁H₁₉C1₂N₃ *m*/*z* 385.30 (M+H)⁺.

### Example 29

### Preparation of N-(2,3-dihydro-1H-inden-1-yl)-5-(4-methoxy-2-methylphenyl)-3,6-dimethylpyrazin-2-amine

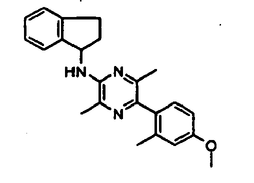

Following the procedure for the preparation of (1R,2S)-1-{[5-(2,4-dichlorophenyl)-3,6-diethylpyrazin-2-yl]amino}-2,3-dihydro-1H-inden-2-ol but substituting 5-bromo-N-(2,3-dihydro-1 H-inden-1-yl)-3,6-dimethylpyrazin-2-amine and 2-methyl-4-methoxy phenyl boronic acid, and making non-critical variations provided the title compound as a oil: ¹H NMR (CDCl₃) δ 1.92, 2.17, 2.26, 2.39, 2.80, 2.98, 3.07, 3.85, 4.54, 5.82, 6.84, 7.15, 7.30, 7.41; MS (ESI+) for C₂₃H₂₅N₃O₁ *m*/*z* 360.4 (M+H)⁺.

### Preparation 18

### Preparation of (1R,2S)-1-[(3,6-dimethylpyrazin-2-yl)amino]-2,3-dihydro-1H-inden-2-ol

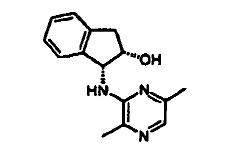

Following the procedure for the preparation of (1R,2S)-1-[(3,6-diethylpyrazin-2-yl)amino]-2,3-dihydro-1H-inden-2-ol but substituting 3-chloro-2,5-dimethylpyrazine and making non-critical variations provided the title compound as a oil: ¹H NMR (300 MHz, CDCl₃) δ) 7.68, 7.32-7.28, 5.59, 4.80, 3.27, 3.09, 2.92, 2.38; HRMS (FAB) calcd for C₁₅H₁₇N₃O+H 256.1450, found 256.1460.

### Preparation 19

### Preparation of (1R,2S)-1-[(5-bromo-3,6-dimethylpyrazin-2-yl)arnino]-2,3-dihydro-1H-inden-2-ol

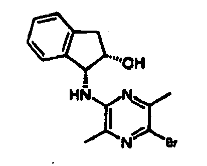

Following the procedure for the preparation of (1R,2S)1-[(5-bromo-3,6-diethylpyrazin-2-yl)amino]-2,3-dihydro-1H-inden-2-ol but substituting (1R,2S)-1-[(3,6-dimethylpyrazin-2-yl)amino]-2,3-dihydro-1H-inden-2-ol and making non-critical variations provided the title compound as a oil: ¹H NMR (300 MHz, CDCl₃) δ) 7.32-7.28, 5.58, 4.92, 4.77, 3.28, 3.07, 2.51, 2.38, 2.28; HRMS (FAB) calcd for C₁₅H₁₆BrN₃O+H 334.0555, found 334.0557. Anal. Calcd for C₁₅ s H₁₆ Br N₃ O: C, 53.91; H, 4.83; N, 12.57. Found: C, 53.55; H, 4.88; N, 12.27.

### Preparation 20

### Preparation of (1R,2S)-1-{[5-(2,4-dichlorophenyl)-3,6-dimethylpyrazin-2-yl]amino}-2,3-dihydro-1H-inden-2-ol

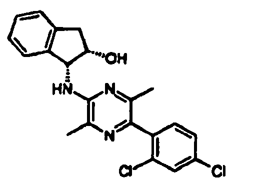

Following the procedure for the preparation of (1R,2S)-1-{[5-(2,4-dichlorophenyl)-3,6-diethylpyrazin-2-yl]amino}-2,3-dihydro-1H-inden-2-ol but substituting (1R,2S)-1-[(5-bromo-3,6-dimethylpyrazin-2-yl)amino]-2,3-dihydro-1H-inden-2-ol and making non-critical variations provided the title compound as a oil: ¹H NMR (300 MHz, CDCl₃) δ) 7.51, 7.36-7.28, 5.66, 4.98, 4.82, 3.25, 3.11, 2.75, 2.42, 2.26; HRMS (FAB) calcd for C₂₁H₁₉Cl₂N₃O+H 400.0983, found 400.0995.
Anal. Calcd for C₂₁ H₁₉ Cl₂ N₃ O: C, 63.01; H, 4.78; N, 10.50. Found: C, 62.86; H, 4.80; N, 10.39.

### Example 30

### Preparation of 5-(2,4-dichlorophenyl)-N-[(1R,2S)2-methoxy-2,3-dihydro-1H-inden-1-yl]-3,6-dimethylpyrazin-2-amine

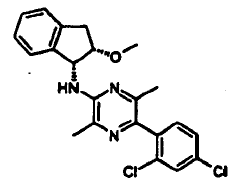

Following the procedure for the preparation of 5-(2,4-dichlorophenyl)-N-[(1 R,2S)-2-ethoxy-2,3-dihydro-1H-inden-1-yl]-3,6-diethylpyrazin-2-amine but substituting (1R,2S)-1-{[5-(2;4-dichlorophenyl)-3,6-dimethylpyrazin-2-yl]amino}-2,3-dihydro-1H-inden-2-ol and methyl iodide, and making non-critical variations provided the title compound as a oil: ¹H NMR (300 MHz, CDCl₃) δ) 7.51, 7.43, 7.37-7.25, 5.86, 5.40, 4.28, 3.43, 3.23, 3.06, 2.42, 2.25; HRMS (FAB) calcd for C₂₂H₂₁Cl₂N₃O+H 414.1140, found 414.1143.
Anal. Calcd for C₂₂H₂₁ Cl₂ N₃ O: C, 63.78; H, 5.11; N, 10.14. Found: C, 63.86; H, 5.21; N, 10.07.

### Example 31

### Preparation of 5-(2,4-dichlorophenyl)-N-[(1R,2S)-2-ethoxy-2,3-dihydro-1H-inden-1-yl]-3,6-dimethylpyrazin-2-amine

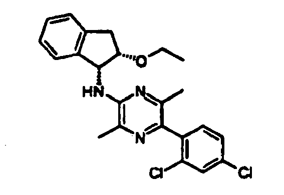

Following the procedure for the preparation of 5-(2,4-dichlorophenyl)-N-[(1R,2S)-2-ethoxy-2,3-dihydro-1H-inden-1-yl]-3,6-diethylpyrazin-2-amine but substituting (1R2S)-1-{[5-(2,4-dichlorophenyl)-3,6-dimethylpyrazin-2-yl]amino}-2,3-dihydro-1H-inden-2-ol and making non-critical variations provided the title compound as a oil: ¹H NMR (300 MHz, CDCl₃) δ); 7.50-7.48, 7.32-7.28, 5.81, 5.48, 4.38, 3.70, 3.52, 3.14, 2.41, 2.26, 1.20; HRMS (FAB) calcd for C₂₃H₂₃Cl₂N₃O+H 428.1296, found 428.1288.
Anal. Calcd for C₂₃ H₂₃ Cl₂ N₃ O: C, 64.49; H, 5.41; N, 9.8. Found: C, 64.49; H, 5.48; N, 9.74.

### Example 32

### Preparation of 5-(2,4-dichlorophenyl)-N-[(1R,2S)-2-isopropoxy-2,3-dihydro-1H-inden-1-yl]-3,6-dimethylpyrazin-2-amine.

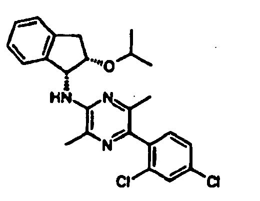

Following the procedure for the preparation of 5-(2,4-dichlorophenyl)-N-[(1R,2S)-2-ethoxy-2,3-dihydro-1H-inden-1-yl]-3,6-diethylpyrazin-2-amine but substituting (1R,2S)-1-{[5-(2,4-dichlorophenyl)-3,6-dimethylpyrazin-2-yl]amino}-2,3-dihydro-1 H-inden-2-ol and 2-iodopropane, and making non-critical variations provided the title compound as a oil: ¹H NMR (300 MHz, CDCl₃) δ) 7.50-7.46, 7.34-7.26, 5.76, 5.43, 4.47, 3.72, 3.14-3.11, 2.41, 2.26, 1.20, 1.12; HRMS (FAB) calcd for C₂₄H₂₅Cl₂N₃O+H 442.1453, found 442.1441. Anal. Calcd for C₂₄ H₂₅ Cl₂ N₃ **O:** C, 65.16; H, 5.70; N, 9.50. Found: C, 65.33; H, 5.83; N, 9.34.

### Preparation 21

### Preparation of 6-methoxyindan-1-amine

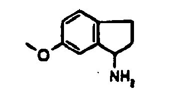

Following the procedure for the preparation of 4,5,6,7-tetrahydro-1-benzofuran-4-amine but substituting 8-methoxy-3,4-dihydronaphthalen-1(2H)-one and making non-critical variations provided the title compound as a oil: ¹H NMR (CDCl₃) δ 1.72. 2.54, 2.74, 2.90, 3.83, 4.34, 6.78, 6.91, 7.13; MS (FAB) m/z (rel. intensity) 164 (MH+, 17), 308 (8), 164 (17), 163 (7), 162 (19), 148 (18), 147 (99), 146 (18), 145 (9), 121 (11), 115 (8). HRMS (FAB) calcd for C₁₀H₁₃NO+H 164.1075, found 164.1071.

### Preparation 22

### Preparation of 3,6-diethyl-N-(6-methoxy-2,3-dihydro-1H-inden-1-yl)pymzin-2-amine

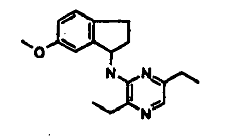

Following the procedure for the preparation of (1R,2S)-1-[(3,6-diethylpyrazin-2-yl)amino]-2,3-dihydro-1H-inden-2-ol but substituting 6-methoxyindan-1-amine and making non-critical variations provided the title compound as a oil: ¹H NMR (CDCl₃) δ 1.31,1.87, 2.59, 2.67, 2.75, 2.87, 2.95, 3.79, 4.55, 5.75, 6.83, 7.19, 7.70; HRMS (FAB) calcd for C₁₈H₂₃N₃O+H 298.1919, found 298.1909. Anal. Calcd for C₁₈ H₂₃ N₃ **O:** C, 72.70; H, 7.80; N, 14.13. Found: C, 72.19; H, 7.76; N, 13.84.

### Preparation 23

### Preparation of 5-bmmo-3,6-diethyl-N-(6-methoxy-2,3-dihydro-1H-inden-1-yl)pyrazin-2-amine

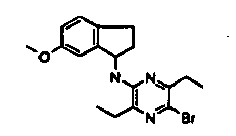

Following the procedure for the preparation of (1R,2S)-1-[(5-bromo-3,6-diethylpyrazin-2-yl)amino]-2,3-dihydro-1H-inden-2-ol but substituting 3,6-diethyl-N-(6-methoxy-2,3-dihydro-1H-inden-1-yl)pyrazin-2-amine and making non-critical variations provided the title compound as a oil: ¹H NMR (CDCl₃) δ 1.27-1.31, 1.86, 2.58, 2.75, 2.83, 2.85, 2.97, 3.80, 4.54, 5.67, 6.83-6.88, 7.20; HRMS (FAB) calcd for C₁₈H₂₂BrN₃O+H 376.1025, found 376.1020. Anal. Calcd forC₁₈ H₂₂ Br N₃ O: C, 57.45; H, 5.89; N, 11.17; Br, 21.23. Found: C, 56.15; H, 5.65; N, 10.83.

### Example 33

### Preparation of 5-(2,4-dichlorophenyl)-3,6-diethyl-N-(6-methoxy-2,3-dihydro-1H-inden-1-yl)pyrazin-2-amine

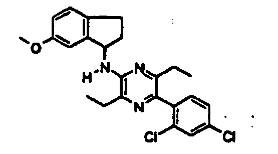

Following the procedure for the preparation of (1R,2S)-1-{[5-(2,4-dichlorophenyl)-3,6-diethylpyrazin-2-yl]amino}-2,3-dihydro-1H-inden-2-ol but substituting 5-bromo-3,6-diethyl-N-(6-methoxy-2,3-dihydro-1H-inden-1-yl)pyrazin-2-amine, ethylene glycol dimethyl ether and tetrakis(triphenylphosphine) palladium and making non-critical variations provided the title compound as a oil: ¹H NMR (CDCl₃) δ 1.21, 1.29, 1.94, 2.51, 2.67, 2.80, 2.92, 3.01, 3.81, 4.69, 5.80, 6.86, 6.95, 7.22, 7.29-7.36, 7.51; HRMS (FAB) calcd for C₂₄H₂₅Cl₂N₃O+H 442.1453, found 442.1443.

### Preparation 24

### Preparation of 2-ethyl-6-methoxyindan-1-one

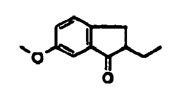

Following the procedure for the preparation of 5-propyl-6,7-dihydro-1-benzothiophen-4(5H)-one but substituting 6-methoxy 1-tetralone and ethyl iodide, and making non-critical variations provided the title compound as a oil: ¹H NMR (CDCl₃) δ 1.02, 1.55, 1.99, 2.68, 2.77, 3.27, 3.86, 7.21, 7.37; HRMS (FAB) calcd forC₁₂H₁₄O₂+H 191.1072, found 191.1075.

### Preparation 25

### Preparation of 2-ethyl-6-methoxy-2,3-dihydro-1H-inden-1-one oxime

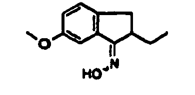

Following the procedure for the preparation of(4E)-2,3-dihydro-4H-chromen-4-one oxime but substituting 2-ethyl-6-methoxyindan-1-one and making non-critical variations provided the title compound as a oil: HRMS (FAB) calcd for C₁₂H₁₅NO₂+H 206.1181, found 206.1175. Anal. Calcd for C₁₂ H₁₅ N O₂: C, 70.22; H, 7.37; N, 6.82. Found: C, 69.90; H, 7.48; N, 6.64.

### Preparation 26

### Preparation of cis-2-ethyl-6-methoxy-2,3-dihydro-1H-inden-1-amine

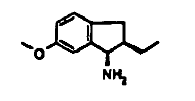

Following the procedure for the preparation of 6,7-dihydro-5H-cyclopenta[b]pyridin-5-amine but substituting 2-ethyl-6-methoxy-2,3-dihydro-1H-inden-1-one oxime and making non-critical variations provided the title compound as a oil: ¹H NMR (CDCl₃) δ 1.03, 1.39, 2.30, 2.63, 2.86, 3.82, 4.26, 6.77, 7.12;

### Preparation 27

### Preparation of 3,6-diethyl-N-[(cis)-2-ethyl-6-methoxy-2,3-dihydro-1H-inden-1-yl]pyrazin-2-amine

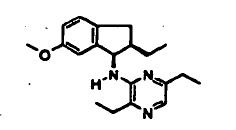

Following the procedure for the preparation of (1R,2S)-1-[(3,6-diethylpyrazin-2-yl)amino]-2,3-dihydro-1H-inden-2-ol but substituting cis-2-ethyl-6-methoxy-2,3-dihydro-1H-inden-1-amine and making non-critical variations provided the title compound as a oil: ¹H NMR (CDCl₃) δ 0.93, 1.27-1.34, 2.58, 2.68, 3.00, 3.79, 4.44, 5.83, 6.80, 6.92, 7.17, 7.68; HRMS (EI) calcd for C₂₀H₂₇N₃O 325.2154, found 325.2157.

### Preparation 28

### Preparation of 5-bromo-3,6-diethyl-N-[(cis)-2-ethyl-6-methoxy-2,3-dihydro-1H-inden-1-yl]pyrazin-2-amine

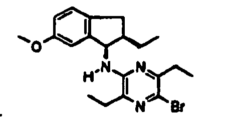

Following the procedure for the preparation of (1R,2S)-1-[(5-bromo-3,6-diethylpyrazin-2-yl)amino]-2,3-dihydro- 1H-inden-2-ol but substituting 3,6-diethyl-N-[(cis)-2-ethyl-6-methoxy-2,3-dihydro-1H-inden-1-yl]pyrazin-2-amine and making non-critical variations provided the title compound as a oil: ¹H NMR (CDCl₃) δ 0.93, 1.21-1.34, 2.57, 2.73, 2.82, 3.00, 3.79, 4.41, 5.76, 6.82, 7.17; HRMS (FAB) calcd for C₂₀H₂₆BrN₃O+H 404.1338, found 404.1317. Anal. Calcd for C₂₀ H₂₆ Br N₃ **O:** C, 59.41; H, 6.48; N, 10.39; Br, 19.76. Found: C, 59.05; H, 6.42; N, 10.13.

### Example 34

### Preparation of 5-(2,4-dichlorophenyl)-3,6-diethyl-N-[(cis)-2-ethyl-6-methoxy-2,3-dihydro-1H-inden-1-yl]pyrazin-2-amine

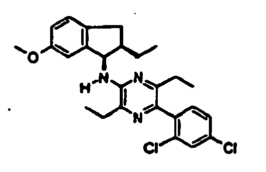

Following the procedure for the preparation of (1R,2S)-1-{[5-(2,4-dichlorophenyl)-3,6-diethylpyrazin-2-yl]amino}-2,3-dihydro-1H-inden-2-ol but substituting 5-bromo-3,6-diethyl-N-[(cis).2-ethyl-6-methoxy-2,3-dihydro-1H-inden-1-yl]pyrazin-2-amine, ethylene glycol dimethyl ether and tetrakis(triphenylphosphine) palladium and making non-critical variations provided the title compound as a oil: ¹H NMR (CDCl₃) δ 0.97, 1.22, 1.28, 2.52, 2.65, 2.75, 3.04, 3.81, 4.57, 5.87, 6.84, 6.98, 7.18, 7.34, 7.50; HRMS (FAB) calcd for C₂₆H₂₉Cl₂N₃O+H 470.1765, found 470.1747

### Preparation 29

### Preparation of 5-methoxyindan-1-one oxime

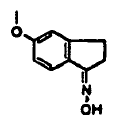

Following the procedure for the preparation of (4E)-2,3-dihydro-4H-chromen-4-one oxime but substituting 5-methoxyindan-1-one and making non-critical variations provided the title compound as a oil: HRMS (EI) calcd for C₁₀H₁₁O₂ 177.0790, found 177.0783. Anal. Calcd for C₁₀ H₁₁ N O₂: C, 67.78; H, 6.26; N, 7.90. Found: C, 67.73; H, 6.34; N, 7.77.

### Preparation 30

### Preparation of 5-methoxyindan-1-amine

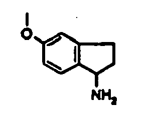

Following the procedure for the preparation of 6,7-dihydro-5H-cyclopenta[b]pyridin-5-amine but substituting 5-methoxyindan-1-one oxime and making non-critical variations provided the title compound as a oil: ¹H NMR (CDCl₃) δ 1.73, 2.75, 2.80_{,} 2.94, 3.82, 4.34, 6.80, 7.25; HRMS (FAB) calcd for C₁₀H₁₁NO+H 162.0919, found 162.0922.

### Preparation 31

### Preparation of 3,6-diethyl-N-(5-methoxy-2,3-dihydro-1H-inden-1 -yl)pyrazin-2-amine

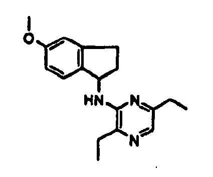

Following the procedure for the preparation of (1R,2S)-1-[(3,6-diethylpyrazin-2-yl)amino]-2,3-dihydro-1H-inden-2-ol but substituting 5-methoxyindan-1-amine and making non-critical variations provided the title compound as a oil: ¹H NMR (CDCl₃) δ 1.31, 1.89, 2.58, 2.67, 2.73, 2.91, 3.00, 3.83, 4.51, 5.69, 6.79, 6.85, 7.26, 7.69; ¹³C NMR (CDCl₃) δ 11.08, 13.97, 26.04, 28.55, 30.84, 35.35, 55.79, 55.87,110.39, 113.15, 125.21, 130.10, 137.12, 140.93, 145.91, 151.82, 153.69, 160.25; HRMS (FAB) calcd for C₁₈H₂₃N₃O+H 298.1919, found 298.1900. Anal. Calcd for C₁₈ H₂₃ N₃ O: C, 72.70; H, 7.80; N, 14.13. Found: C, 72.51; H, 8.00; N, 13.82.

### Preparation 32

### Preparation of 5-bromo-3,6-diethyl-N-(5-methoxy-2,3-dihydro-1H-inden-1 - yl)pyrazin-2-amine

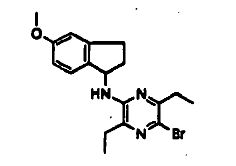

Following the procedure for the preparation of (1R,2S)-1-[(5-bromo-3,6-diethylpyrazin-2-yl)amino]-2,3-dihydro-1H-inden-2-ol but substituting 3,6-diethyl-N-(5-methoxy-2,3-dihydro-1H-inden-1-yl)pyrazin-2-amine and making non-critical variations provided the title compound as a oil: ¹H NMR (CDCl₃) δ 1.29, 1.89, 2.56, 2.70, 2.83, 2.87, 3.00, 3.83, 4.49, 5.61, 6.81, 6.85, 7.23; HRMS (FAB) calcd for C₁₈H₂₂BrN₃O+H 376.1025, found 376.1008.

### Example 35

### Preparation of 5-(2,4-dichlorophenyl)-3,6-diethyl-N-(5-methoxy-2.3-dihydro-1H-inden-1-yl)pyrazin-2-amine

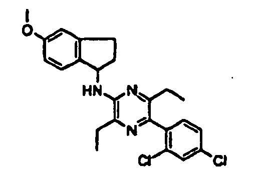

Following the procedure for the preparation of (1R,2S)-1-{[5-(2,4-dichlorophenyl)-3,6-diethylpyrazin-2-yl]amino}-2,3-dihydro-1H-inden-2-ol but substituting 5-bromo-3,6-diethyl-N-(5-methoxy-2,3-dihydro-1H-inden-1-yl)pyrazin-2-amine, ethylene glycol dimethyl ether and tetrakis(triphenylphosphine) palladium and making non-critical variations provided the title compound as a oil: ¹H NMR (CDCl₃) δ) 1.28, 1.95, 2.51, 2.65, 2.80, 2.95, 3.02, 3.85, 4.14, 4.65, 5.73, 6.82, 6.87, 7.28-7.35, 7.5; HRMS (FAB) calcd for C₂₄H₂₅Cl₂N₃O+H 442.1453, found 442.1450.

### Preparation 33

### Preparation of (+/-)-2-ethyl-2,3-dihydro-1H-inden-1-one

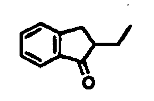

Following the procedure for the preparation of 5-propyl-6,7-dihydro-1-benzothiophen-4(5H)-one but substituting 1-tetralone and ethyl iodide, and making non-critical variations provided the title compound as a colorless mobile oil. IR (liq.) 2963, 2933, 2875, 2860, 1712, 1610, 1588, 1475, 1464, 1327, 1296, 1278, 1205, 749, 718 cm⁻¹; MS (EI) *m*/*z* 160 (M⁺); Anal. Calcd for C₁₁H₁₂O: C, 82.46; H, 7.55. Found: C, 82.16; H, 7.57.

### Preparation 34

### Preparation of 2-ethyl-2,3-dihydro-1H-inden-1-one oxime

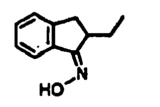

Following the procedure for the preparation of (4E)-2,3-dihydro-4H-chromen-4-one oxime but substituting (+/-)-2-ethyl-2,3-dihydro-1H-inden-1-one and making non-critical variations provided the title compound as a colorless syrup. MS (ESI+) for *m*/*z* 176.1 (M+H)⁺.

### Preparation 35

### Preparation of 2-ethylindan-1-amine

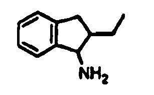

Following the procedure for the preparation of 6,7-dihydro-5H-cyclopenta[b]pyridin-5-amine but substituting 2-ethyl-2,3-dihydrn-1H-inden-1-one oxime and making non-critical variations provided the title compound as a colorless oil.

### Preparation 36

### Preparation of 3,6-diethyl-N-(2-ethyl-2,3-dihydro-1H-inden-1-yl)pyrazin-2-amine

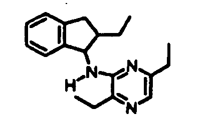

Following the procedure for the preparation of (1R,2S)-1-[(3,6-diethylpyrazin-2-yl)amino]-2,3-dihydro-1H-inden-2-ol but substituting 2-ethylindan-1-amine and making non-critical variations provided the title compound as a yellow oil. MS (ESI+) for *m*/*z* 296.2 (M+H)⁺.

### Preparation 37 and 38

### Preparation of (+/-)-5-bromo-3,6-diethyl-N-[cis-2-ethyl-2,3-dihydro-1H-inden-1-yl]pyrazin-2-amine and (+/-)-5-bromo-3,6-diethyl-N-[trans-2-ethyl-2,3-dihydro-1H-inden-1-yl]pyrazin-2-amine

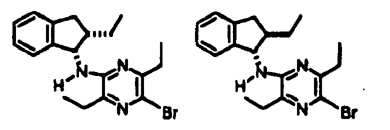

Following the procedure for the preparation of (1R,2S)-1-[(5-bromo-3,6-diethylpyrazin-2-yl)amino]-2,3-dihydro-1H-inden-2-ol but substituting 3,6-diethyl-N-(2-ethyl-2,3-dihydro-1H-inden-1-yl)pyrazin-2-amine and making non-critical variations provided the title compounds. Analytical data for (+/-)-5-bromo-3,6-diethyl-N-[cis-2-ethyl-2,3-dihydro-1H-inden-1-yl]pyrazin-2-amine: IR (liq.) 3435, 2967, 2935, 2874, 1560, 1539, 1479, 1460, 1446, 1416, 1390, 1243, 1177, 751, 731 cm⁻¹; MS (EI) *m*/*z* 373 (M⁺); Anal. Calcd for C₁₉H₂₄BrN₃: C, 60.97; H, 6.46; N, 11.23. Found: C, 61.01; H, 6.53; N, 11.22. Analytical data for (+/-)-5-bromo-3,6-diethyl-N-[trans-2-ethyl-2,3-dihydro-1H-inden-1-yl]pyrazin-2-amine: IR (liq.) 2967, 2935, 2874, 2856, 1561, 1537, 1478, 1460, 1447, 1416, 1388, 1186, 1176, 1164, 746 cm⁻¹; OAMS supporting ions at: ESI+ 373.8; MS (EI) *m*/*z* 373 (M⁺); Anal. Calcd for C₁₉H₂₄BrN₃: C, 60.97; H, 6.46; N, 11.23. Found: C, 60.97; H, 6.51; N, 11.16.

### Example 36

### Preparation of (+/-)-5-(2,4-dichlorophenyl)-3,6-diethyl-N-[cis-2-ethyl-2,3-dihydro-1H-inden-1-yl]pyrazin-2-amine.

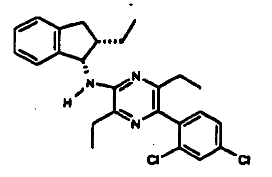

Following the procedure for the preparation of (1R,2S)-1-{[5-(2,4-dichlorophenyl)-3,6-diethylpyrazin-2-yl]amino}-2,3-dihydro-1H-inden-2-ol but substituting (+/-)-5-bromo-3,6-diethyl-N-[cis-2-ethyl-2,3-dihydro-1H-inden-1-yl]pyrazin-2-amine and making non-critical variations provided the title compound as a light yellow semi-solid. IR (liq.) 3452, 2965, 2934, 2874, 1589, 1566, 1551, 1495, 1470, 1392, 1377, 1203, 1175, 1101, 752 cm⁻¹; OAMS supporting ions at: ESI+ 439.8; HRMS (FAB) calcd for C₂₅H₂₇CL₂N₃ +H₁ 440.1660, found 440.1648. Anal. Calcd for C₂₅H₂₇Cl₂N₃: C, 68.18; H, 6.18; N,9.54. Found: C, 68.33; H, 6.36; N, 9.30.

### Example 37

### Preparation of (+/-)-5-(2,4-dichlorophenyl)-3,6-diethyl-N-[trans-2-ethyl-2,3-dihydro-1H-inden-1-yl]pyrazin-2-amine.

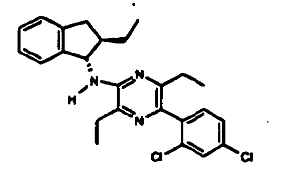

Following the procedure for the preparation of (1R,2S)-1-{[5-(2,4-dichlorophenyl)-3,6-diethylpyrazin-2-yl]amino}-2,3-dihydro-1H-inden-2-ol but substituting (+1-)-5-bromo-3,6-diethyl-N-[trans-2-ethyl-2,3-dihydro-1H-inden-1-yl]pyrazin-2-amine and making non-critical variations provided the title compound as a light yellow semi-solid. 1R (liq.) 3443, 2965, 2934, 2874, 1588, 1566,1551, 1497,1470,1390,1203, 1186, 1173, 1101, 747 cm⁻¹; OAMS supporting ions at: ESI+440.0;MS(EI) *m*/*z* 439 (M⁺); Anal. Calcd for C₂₅H₂₇Cl₂N₃: C, 68.18; H, 6.18; N, 9.54. Found: C, 68.11; H, 6.17; N, 9.29.

### Preparation 39

### methyl amino(cyclopropyl)acetate hydrochloride

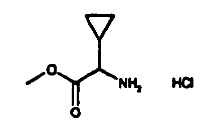

To a solution of cyclopropyl glycine (0.50 g, 4.3 mmol) in ice cold MeOH (4.3 mL) was added dropwise SOCl₂ (1.3 g, 0.8 mL, 11 mmol). The ensuing mixture was stirred at 0 °C for 10 min then allowed to warm to rt. Stir at rt overnight, concentrate and dry at rt/0.5 mmHg to provide 0.60 g (85%) of methyl amino(cyclopropyl)acetate hydrochloride as a solid: ¹H NMR (DMSO-d6) δ 0.51-0.69, 1.05-1.17, 3.36-3.39, 3.75, 8.69.

### Preparation 40

### methyl 2-aminobutanoate hydrochloride

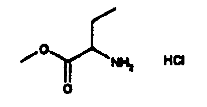

Following the procedure for the preparation of methyl amino(cyclopropyl)acetate hydrochloride but substituting alpha-aminobutyric acid and making non-critical variations provided the title compound as a solid: MS (ESI+) for C₅H₁₁NO₂ *m*/*z* 235 (2M+H)⁺.

### Preparation 41

### methyl {[N-(tert-butoxycarbonyl)-L-alanyl]amino}(cyclopropyl)acetate

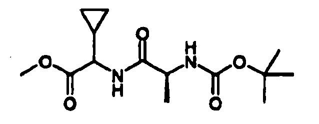

To an ice cold solution of methyl amino(cyclopropyl)acetate hydrochloride (0.25 g, 1.5 mmol), N-Boc-L-alanine-OH (0.34 g, 1.5 mmol) in DMF (7.5 mL) containing diisopropylethyl amine (0.58 g, 0.8 mL, 4.5 mmol) was added O-(7-azabenzotnazol-1-yl)-N,N,N,N-tetramethyluronium hexafluorophosphate (HATU) (0.57 g, 1.5 mmol). After stirring at rt for 2 days, the mixture was diluted with EtOAc and sequentially washed with sat. aq. NaHCO₃ and sat. aq. NaCl. The organic extracts were dried over MgSO₄, filtered and concentrated. Purify by biotage MPLC (90 g column, 25-30% ethyl acetate/hexanes) to afford 0.39 g (78%) methyl {[N-(tert-butoxycarbonyl)-L-alanyl]amino}(cyclopropyl)acetate as a solid: ¹H NMR (CDCl₃) δ 0.39-0.62, 1.28-1.39, 1.47, 3.77, 4.03-4.19, 4.99, 6.66; MS (ESI+) for C₁₄H₂₄N₂O₅ *m*/*z* 301 (M+H)⁺.

### Preparation 42

### methyl ({2-[(tert-butoxycarbonyl)amino]butanoyl}amino)(cyclopropyl)acetate

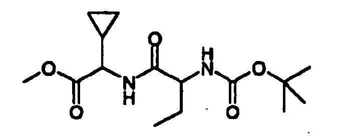

Following the procedure for the preparation of methyl {[N-(tert-butoxycarbonyl)-L-alanyl]amino}(cyclopropyl)acetate but substituting N-Boc-alpha-aminobutyric acid and making non-critical variations provided the title compound as a solid: ¹H NMR (CDCl₃) δ 0.40-0.62, 0.94-0.99, 1.09-1.12, 1.46, 1.63-2.06, 3.77, 4.04, 5.02, 6.54.

### Preparation 43

### methyl N-{2-[(tort-butoxycarbonyl)amino]butanoyl)alaninate

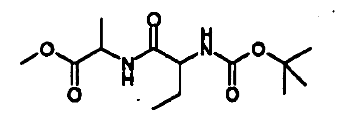

Following the procedure for the preparation of methyl {[N-(tert-butoxycarbonyl)-L-alanyl]amino}(cyclopropyl)acetate but substituting N-Boc-alpha-aminobutyric acid and methyl 2-aminobutanoate hydrochloride and making non-critical variations provided the title compound as a solid: ¹H NMR (CDCl₃) δ 0.82-0.91, 1.24-1.38,1.52, 1.68-2.05, 3.75, 4.20, 4.52-4.59, 5.05, 6.69.

### Preparation 44

### methyl (L-alanylamino)(cyclopropyl)acetate hydrochloride

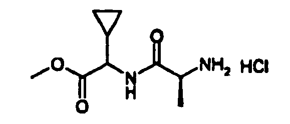

A solution of methyl {[N-(tert-butoxycarbonyl)-L-alanyl]amino}(cyclopmpyl)-acetate (72 mg, 0.24 mmol) in 4 N HCl in dioxane (1 mL) was stirred at rt for 45 min. Concentrate to afford 57 mg (100%) of methyl (L-alanylamino)(cyclopropyl)acetate hydrochloride as a solid: ¹H NMR (DMSO-d6) δ 0.42-0.60,1.21-1.26, 1.68, 3.65-3.91, 4.60, 8.17; MS (ESI+) for C₈H₁₂N₂O₂ *m*/*z* 201 (M+H)⁺.

### Preparation 45

### methyl [(2-aminobutanoyl)amino](cyclopropyl)acetate hydrochloride

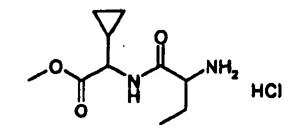

Following the procedure for the preparation of methyl (L-alanylamino)(cyclopropyl)acetate hydrochloride but substituting methyl ({2-[(tert-butoxycarbonyl)amino]butanoyl}amino)(cyclopropyl)acetate and making non-critical variations provided the title compound as a solid: ¹H NMR (DMSO-d6) δ 0.29-0.40, 0.88-0.94, 1.12, 1.37, 1.75-1.82, 3.57, 3.62-3.79, 8.29.

### Preparation 46

### methyl N-(2-aminobutanoyl)alaninate hydrochloride

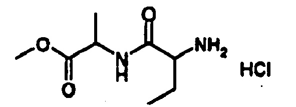

Following the procedure for the preparation of methyl (L-alanylamino)(cyclopropyl)acetate hydrochloride but substituting methyl N-{2-[(tert-butoxycarbonyl)amino]butanoyl}alaninate and making non-critical variations provided the title compound as a solid: ¹H NMR (DMSO-d6) δ 0.86-0.92, 1.39-1.41, 1.62-1..82, 3.64, 3.92, 4.18-4.23, 8.36. 8.92.

### Preparation 47

### 3,6-dicyclopropylpiperazine-2,5-dione

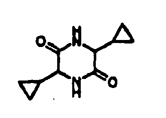

Following the procedure for the preparation of 3,6-diethylpiperazine-2,5-dione but substituting cyclopropyl glycine (prepared as described in patent US 6,191,306) and making non-critical variations provided the title compound as a solid. ¹H NMR (DMSO-d6) δ 0.26-0.52, 1.04-1.12, 3.15-3.18, 3.24-3.27, 3.33, 8.06, 8.19.

### Preparation 48

### 3-cyclopropyl-6-methylpiperazine-2,5-dione

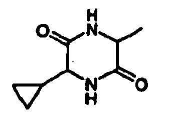

A solution of methyl (L-alanylamino)(cyclopropyl)acetate hydrochloride (230 mg, 0.97 mmol) in 7 N NH₃ in MeOH (6 mL) was heated at reflux for 5 hr. Cool to rt and concentrate. The resulting material was dried at rt/0.5 mm Hg to provide 0.21 g of 3-cyclopropyl-6-methylpiperazine-2,5-dione as a solid: ¹H NMR (CDCl₃) δ 0.26-0.51, 1.06-1.37, 3.16-3.25, 3.50, 3.80, 4.00, 8.13.

### Preparation 49

### 3-cyclopropyl-6-ethylpiperazine-2,5-dione

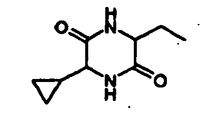

Following the procedure for the preparation of 3-cyclopropyl-6-methylpiperazine-2,5-dione but substituting methyl [(2-aminobutanoyl)amino](cyclopropyl)acetate hydrochloride and making non-critical variations provided the title compound as a solid: ¹H NMR (DMSO-d6) δ 0.29-0.48, 0.79-1.20,1.69, 3.20, 3.93, 8.05.

### Preparation 50

### 3-ethyl-6-methylpiperazine-2,5-dione

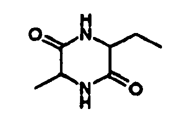

Following the procedure for the preparation of 3-cyclopropyl-6-methylpiperazine-2,5-dione but substituting methyl N-(2-aminobutanoyl)alaninate hydrochloride and making non-critical variations provided the title compound as a solid: ¹H NMR (DMSO-d6) δ 0.81-0.86, 1.25-1.27, 1.64-1.76, 3.73-3.91, 7.37, 8.07-8.15.

### Preparation 51

### 3-chloro-2,5-dicyclopropylpyrazine

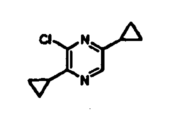

Following the procedure for the preparation of 3-chloro-2,5-diethylpyrazine but substituting 3,6-dicyclopropylpiperazine-2,5-dione and making non-critical variations provided the title compound as an oil: ¹H NMR (CDCl₃) δ 0.99-1.13, 1.94-2.03, 2.38-2.47; MS (ESI+) for C₁₀H₁₁N₂ *m*/*z* 195 (M+H)⁺.

### Preparation 52A and 52B

### 3-chloro-2-cyclopropyl-5-methylpyrazine (A) and 3-chloro-5-cyclopropyl-2-methylpyrazine (B)

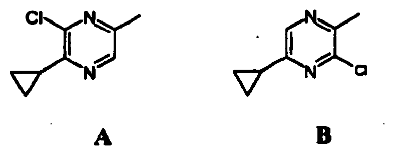

Following the procedure for the preparation of 3-chloro-2,5-diethylpyrazine but substituting 3-cyclopropyl-6-methylpiperazine-2,5-dione and making non-critical variations provided 3-chloro-2-cyclopropyl-5-methylpyrazine (A) and 3-chloro-5-cyclopropyl-2-methylpyrazine (B) as oils. Analytical data for 3-chloro-2-cyclopropyl-5-methylpyrazine (A): ¹H NMR (CDCl₃) δ 1.44, 2.35, 2.90, 3.07, 8.10; MS (ESI+) for C₈H₉N₂Cl *m*/*z* 169 (M+H)⁺; Analytical data for 3-chloro-5-cyclopropyl-2-methylpyrazine (B): ¹H NMR (CDCh) 81.04, 2.00, 2.57, 8.10; MS (ESI+) for C₈H₉N₂Cl *m*/*z* 169 (M+H)⁺.

### Preparation 53A and 53B

### 3₋chloro-2-cyclopropyl-5-ethylpyrazine (A) and 3-chloro-5-cyclopropyl-2-ethylpyrazine (B)

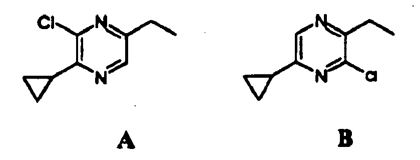

Following the procedure for the preparation of 3-chloro-2,5-diethylpyrazine but substituting 3-cyclopropyl-6-ethylpiperazine-2,5-dione and making non-critical variations provided 3-chloro-2-cyclopropyl-5-methylpyrazine (A) and 3-chloro-5-cyclopropyl-2-methylpyrazine (B) as oils. Analytical data for 3-chloro-2-cyclopropyl-5-ethylpyrazine (A): ¹H NMR (CDCl₃) δ 1.06-1.10, 1.28-1.33, 2.42-2.51, 2.73-2.81, 8.18; Analytical data for 3-chloro-5-cyclopropyl-2-ethylpyrazine (B): ¹H NMR (CDCl₃) δ 1.04-1.08, 1.24-1.39, 1.97-2.06, 2.8 8-2.95, 8.31.

### Preparation 54A and 54B

### 3-chloro-5-ethyl-2-methylpyrazine (A) and 3-chloro-2-ethyl-5-methylpyrazine (B)

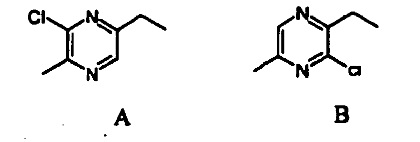

Following the procedure for the preparation of 3-chloro-2,5-diethylpyrazine but substituting 3-ethyl-6-methylpiperazine-2,5-dione and making non-critical variations provided a mixture of 3-chloro-5-ethyl-2-methylpyrazine (A) and 3-chloro-2-ethyl-5-methylpyrazine (B) as an oil: ¹H NMR (CDCl₃) δ 1.29-1.39, 2.53, 2.63, 2.77-2.99, 8.27, 8.30.

### Preparation 55

### (1R2S)-1-[(3,6-dicyclopropylpyrazin-2-yl)amino]-2,3-dihydro- 1H-inden-2-ol

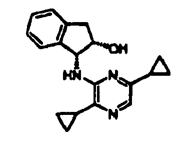

Following the procedure for the preparation of (1R,2S)-1-[(3,6-diethylpyrazin-2-yl)amino]-2,3-dihydro-1H-inden-2-ol but substituting 3-chloro-2,5-dicyclopropylpyrazine and making non-critical variations provided the title compound as a solid: ¹H NMR (CDCl₃) δ 0.86-0.89,2.00, 2.89, 3.11,4.54,5.17,5.42,6.22-6.24, 7.17-7.25, 7.64 ; MS (ESI+) for C₁₉H₂₁N₃O *m*/*z* 308 (M+H)⁺.

### Preparation 56

### (1R,2SS)-1-[(6-cyclopropyl-3-methylpyrazin-2-yl)amino]-2,3-dihydro- 1H-inden-2-ol

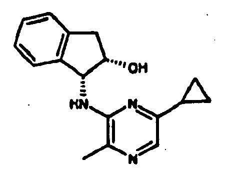

Following the procedure for the preparation of (1R,2S)-1-[(3,6-diethylpyrazin-2-yl)amino]-2,3-dihydro-1 H-inden-2-ol but substituting 3-chloro-5-cyclopropyl-2-methylpyrazine and making non-critical variations provided the title compound as a solid: ¹H NMR (CDCl₃) δ 0.95, 1.93, 2.38, 2.61, 3.09, 3.22, 4.75, 5.50, 7.30, 7.77; MS (ESI+) for C₁₇H₁₉N₃O *m*/*z* 282(M+H)⁺.

### Preparation 57

### (1R,2S)-1-[(3-cyclopropyl-6-methylpyrazin-2-yl)amino]-2,3-dihydro-1H-inden-2-ol

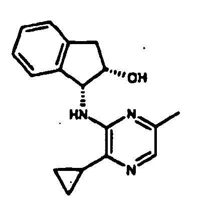

Following the procedure for the preparation of (1R,2S)-1-[(3,6-diethylpyrazin-2-yl)amino]-2,3-dihydro-1 H-inden-2-ol but substituting 3-chloro-2-cyclopropyl-5-methylpyrazine and making non-critical variations provided the title compound as a solid: ¹H NMR (CDCl₃) δ 0.92-1.01, 1.25, 1.76, 2.36, 3.08, 3.24, 4.80, 5.38, 5.59, 7.22-7.49,7.64; MS (ESI+) for C₁₇H₁₉N₃O *m*/*z* 282 (M+H)⁺.

### Preparation 58

### (1R,2S)-1-[(6-Cyclopropyl-3-ethylpyrazin-2-yl)amino]-2,3-dihydro-1H-inden-2-ol

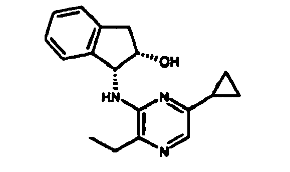

Following the procedure for the preparation of (1R2S)-1-[(3,6-diethylpyrazin-2-yl)amino]-2,3-dihydro-1H-inden-2-ol but substituting 3-chloro-5'-cyclopropyl-2-ethylpyrazine and making non-critical variations provided the title compound as a solid: ¹H NMR (CDCh) δ 0.92-0.95, 1.31-1.35, 1.93,2.59-2.70,3.03.3.10,3.22-3.30, 4.74, 4.91, 5.49-5.52, 7.28-7.32, 7.82; MS (ESI+) for C₁₈H₂₁N₃O *m*/*z* 296 (M+H)⁺.

### Preparation 59

### (1R,2S)-1-[(3-cyclopropyl-6-ethylpyrazin-2-yl)amino]-2,3-dihydro-1 H-inden-2-ol

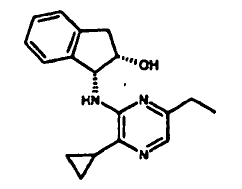

Following the procedure for the preparation of (1R,2S)-1-[(3,6-diethylpyrazin-2-yl)amino]-2,3-dihydro-1H-inden-2-ol but substituting 3-chloro-2-cyclopropyl-5-ethylpyrazine and making non-critical variations provided the title compound as a solid: ¹H NMR (CDCl₃) δ 0.93-1.02, 1.24-1.30, 1.72-1.81, 2.61-2.68, 3.06-3.30, 4.79-4.84,5.36,5.58,7.24-7.35,7.66; MS (ESI+) for C₁₈H₂₁N₃O *m*/*z* 296 (M+H)⁺.

### Preparation 60A and 60B

### (1R,2S)-1-[(6-ethyl-3-methylpyrazin-2-yl)amino]-2,3-dihydro-1H-inden-2-ol (A) and (1R,2S)-1-[(3-ethyl-6-methylpyrazin-2-yl)amino]-2,3-dihydro-1H-inden-2-ol (B)

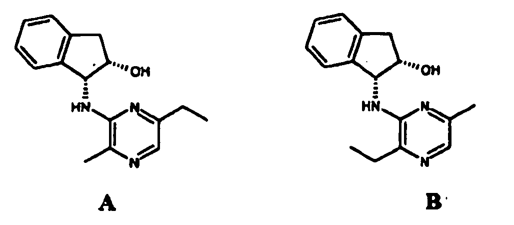

Following the procedure for the preparation of (1R,2S)-1-[(3,6-diethylpyrazin-2-yl)amino]-2,3-dihydro-1H-inden-2-ol but substituting a mixture of 3-chloro-5-ethyl-2-methylpyrazine (A) and 3-chloro-2-ethyl-5-methylpyrazine (B) and making non-critical variations provided a mixture of the title compounds as a solid: ¹H NMR (CDCl₃) δ 1.24-1.34, 2.38, 2.63-2.69, 3.04-3.10, 3.23-3.29, 4.78-4.92, 5.58-5.63, 7.24-7.32,7.68, 7.73.

### Preparation 61

### (1S,2S)-1-[(5-bromo-3,6-dicyclopropylpyrazin-2-yl)amino]-2,3-dihydro-1H-inden-2-ol

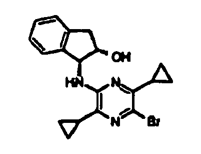

Following the procedure for the preparation of (1R,2S)-1-[(5-bromo-3,6-diethylpyrazin-2-yl)amino]-2,3-dihydro-1H-inden-2-ol but substituting (1R,2S)-1-[(3,6dicyclopropylpyrazin-2-yl)amino]-2,3-dihydro-1H-inden-2-ol and making non-critical variations provided the title compound as a solid: ¹H NMR (CDCl₃) δ 0.89-1.03, 1.27-1.30, 1.71-1.75, 2.36-2.39, 3.05-3.09, 3.25-3.30, 4.72-4.75, 5.45-5.46, 7.26-7.33; MS (ESI+) for C₁₉H₂₀BrN₃O *m*/*z* 386 (M+H)⁺.

### Preparation 62

### (1R,2S)-1-[(5-bromo-6-cyclopropyl-3-methylpyrazin-2-yl)amino]-2,3-dihydro-1H-inden-2-ol

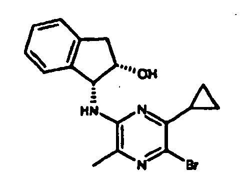

Following the procedure for the preparation of (1R,2S)-1-[(5-bromo-3,6-diethylpyrazin-2-yl)amino]-2,3-dihydro-1H-inden-2-ol but substituting (1R,2S)-1-[(6-cyclopropyl-3-methylpyrazin-2-yl)amino]-2,3-dihydro-1H-inden-2-ol and making non-critical variations provided the title compound as a solid: ¹H NMR (CDCl₃) δ 0.99, 2.08, 2.38, 3.04, 3.25, 4.72, 4.97, 5.45, 7.24-7.31; MS (ESI+) for C₁₇H₁₈BrN₃O m/z 362 (M+H)⁺.

### Preparation 63

### (1R,2S)-1-[(5-bromo-3-cyclopropyl-6-methylpyrazin-2-yl)amino]-2,3-dihydro-1H-inden-2-ol

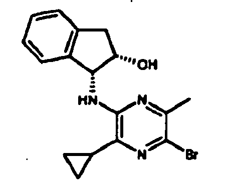

Following the procedure for the preparation of (1R,2S)-1-[(5-bromo-3,6-diethylpyrazin-2-yl)amino]-2,3-dihydro-1H-inden-2-ol but substituting (1R,2S)-1-[(3-cyclopropyl-6-methylpyrazin-2-yl)amino]-2,3-dihydro-1H-inden-2-ol and making non-critical variations provided the title compound as a solid: ¹H NMR (CDCl₃) δ 0.95-1.08, 1.69-1.78, 2.49, 3.04, 3.24, 4.78, 5.39, 5.58, 7.24-7.33; MS (ESI+) for C₁₇H₁₈BrN₃O *m*/*z* 360 (M+H)⁺.

### Preparation 64

### (1R,2S)-1-[(5-bromo-6-cyclopropyl-3-ethylpyrazin-2-yl)amino]-2,3-dihydro-1H-inden-2-ol

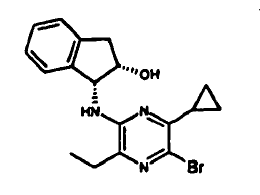

Following the procedure for the preparation of (1R,2S)-1-[(5-bromo-3,6-diethylpyrazin-2-yl)amino]-2,3-dihydro-1H-inden-2-o1 but substituting (1R,2S)-1-[(6-cyclopropyl-3-ethylpyrazin-2-yl)amino]-2,3-dihydro-1H-inden-2-ol and making non-critical variations provided the title compound as a solid: ¹H NMR (CDCl₃) δ 0.93-1.09, 1.28-1.39, 2.35-2.43, 2.60-2.67, 2.99-3.05, 3.22-3.29, 4.70, 5.04, 5.44-5.49, 7.23-7.32; MS (ESI+) for C₁₈H₂₀BrN₃O *m*/*z* 374 (M+H)⁺.

### Preparation 65

### (1R,2S)-1-[(5-bromo-3-cyclopropyl-6-ethylpyrazin-2-yl)amino]-2,3-dihydro-1H-inden-2-ol

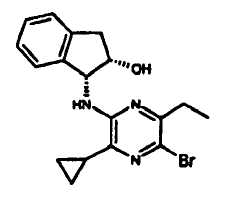

Following the procedure for the preparation of (1R,2S)-1-[(5-bromo-3,6-diethylpyrazin-2-yl)amino]-2,3-dihydro-1H-inden-2-ol but substituting (1R,2S)-1-[(3-cyclopropyl-6-ethylpyrazin-2-yl)amino]-2,3-dihydro-1H-inden-2-ol and making non-critical variations provided the title compound as a solid: ¹H NMR (CDCl₃) δ 0.86-1.08, 1.23-1.30, 1.70-1.79, 2.77-2.84, 3.03-3.09, 3.23-3.30, 4.77-4.81, 5.44, 5.56, 7.23-7.32; MS (EST+) for C₁₈H₂₀BrN₃O *m*/*z* 374 (M+H)⁺.

### Reparation 66

### (1R,2S)-1-[(5-bromo-6-ethyl-3-methylpyrazin-2-yl)amino]-2,3-dihydro-1H-inden-2-ol (A) and (1R2S)-1-[(5-bromo-3-ethyl-6-methylpyrazin-2-yl)amino]-2,3-dihydro-1H-inden-2-ol (B)

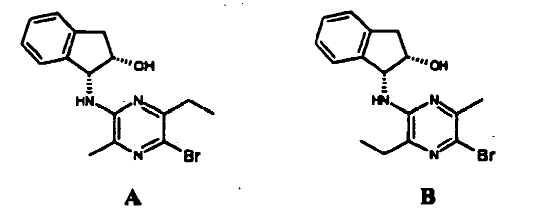

Following the procedure for the preparation of (1R,2S)-1-[(3,6-diethylpyrazin-2-yl)amino]-2,3-dihydro-1H-inden-2-ol but substituting a mixture of (1R,2S)-1-[(6-ethyl-3-methylpyrazin-2-yl)amino]-2,3-dihydro-1H-inden-2-ol (A) and (1R,2S)-1-[(3-ethyl-6-methylpyrazin-2-yl)amino]-2,3-dihydro-1H-inden-2-ol (B) and making non-critical variations provided a mixture of the title compound as a solid: ¹H NMR (CDCl₃) δ 1.11-1.24, 2.26-3.16, 4.63-4.67, 4.94-4.98, 5.20, 5.46-5.50, 7.12-7.30; MS (ESI+) for C₁₆H₁₈BrN₃O m/z 348 (M+H)⁺.

### Preparation 67

### (1R,2S)-1-{[3,6-dicyclopropyl-5-(2,4-dichlorophenyl)pyrazin-2-yl]amino}-2,3-dihydro-1H-inden-2-ol

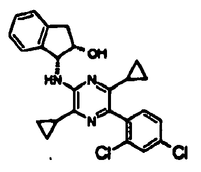

Following the procedure for the preparation of (1R,2S)-1-{[5-(2,4-dichlorophenyl)-3,6-diethylpyrazin-2-yl]amino}-2,3-dihydro-1H-inden-2-ol but substituting (1S,2S)-1-[(5-bromo-3,6-dicyclopropylpyrazin-2-yl)amino]-2,3-dihydro-1 H-inden-2-ol and making non-critical variations provided the title compound as a solid: ¹H NMR(CDCl₃) δ 0.85-0.99,1.67,1.82.2.48, 3.10-3.13,3.27-3.28,4.79, 5.40, 5.42, 7.30-7.51; MS (ESI+) for C₂₅H₂₃Cl₂N₃O *m*/*z* 452 (M+H)⁺.

### Preparation 68

### (1R,2S)-1-{[6-cyclopropyl-5-(2,4-dichlorophenyl)-3-methylpyrazin-2-yl]amino}-2,3-dihydro-1 H-inden-2-ol

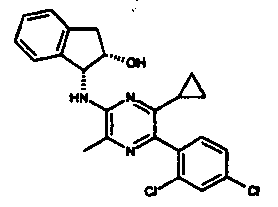

Following the procedure for the preparation of 5-(2,4-dichlorophenyl)-N-[(1R,2S)-2-ethoxy-2,3-dihydro-1H-inden-1-yl]-3,6-diethylpyrazin-2-amine but substituting (1R,25)-1-[(5-bromo-6-cyclopropyl-3-methylpyrazin-2-yl)amino]-2,3-dihydro-1H-inden-2-ol and making non-critical variations provided the title compound as a solid: ¹H NMR (CDCl₃) δ 0.87-1.03, 1.60-1.69, 2.42, 2.71, 3.03, 3.25, 4.73, 4.99, 5.53, 7.12-7.66; MS (ESI+) for C₂₃H₂₁Cl₂N₃O *m*/*z* 426 (M+H)⁺.

### Preparation 69

### (1R,2S)-1-{[3-cyclopropyl-5-(2,4-dichlorophenyl)-6-methylpyrazin-2-yl]amino}-2,3-dihydro-1H-inden-2-ol

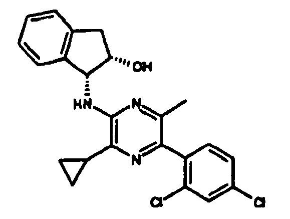

Following the procedure for the preparation of 5-(2,4-dichlorophenyl)-N-[(1R,2S)-2-ethoxy-2,3-dihydro-1H-inden-1-yl]-3,6-diethylpyrazin-2-amine but substituting (1R,2S)-1-[(5-bromo-3-cyclopropyl-6-methylpyrazin-2-yl)amino]-2,3-dihydro-1H-inden-2-ol and making non-critical variations provided the title compound as a solid: ¹H NMR (CDCl₃) δ 0.96-0.99, 1.58, 1.81, 2.26, 2.85, 3.14, 3.26,4.86,5.44,5.63,7.26-7.50; MS (ESI+) for C₂₃H₂₁Cl₂N₃O *m*/*z* 426 (M+H)⁺.

### Preparation 70

### (1R,2S)-1-{[6-cyclopropyl-5-(2,4-dichlorophenyl)-3-ethylpyrazin-2-yl]amino}-2,3-dihydro-1H-inden-2-ol

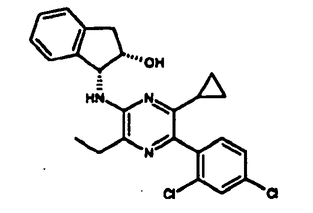

Following the procedure for the preparation of 5-(2,4-dichlorophenyl)-N-[(1R,2S)-2-ethoxy-2,3-dihydro-1H-inden-1-yl]-3,6-diethylpyrazin-2-amine but substituting (1R,2S)-1-[(5-bromo-6-cyclopropyl-3-ethylpyrazin-2-yl)amino]-2,3-dihydro-1H-inden-2-ol and making non-critical variations provided the title compound as a solid: ¹H NMR (CDCl₃) δ 0.96-1.00, 1.26-1.32, 2.04, 2.35-2.40, 2.61-2.66, 3.0-3.04, 3.22-3.27, 4.09-4.14, 4.68-4.70, 5.08, 5.45-5.48, 7.23-7.31; MS (ESI+) for C₂₄H₂₃Cl₂N₃O *m*/*z* 440 (M+H)⁺.

### Preparation 71

### (1R,2S)-1-{[5-(2-chloro-4-methoxyphenyl)-6-cyclopropyl-3-ethylpyrazin-2-yl]amino}-2,3-dihydro-1H-inden-2-ol

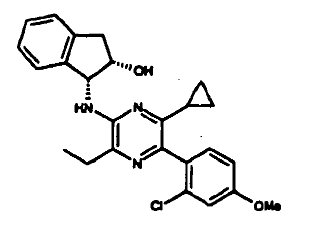

Following the procedure for the preparation of 5-(2,4-dichlorophenyl)-N-[(1 R,2S)-2-ethoxy-2,3-dihydro- 1H-inden-1-yl]-3,6-diethylpyrazin-2-amine but substituting (1R2S)-1-[(5-bromo-6-cyclopropyl-3-ethylpyrazin-2-yl)amino]-2,3-dihydro-1 H-inden-2-ol and making non-critical variations provided the title compound as a solid: ¹H NMR (CDCl₃) δ ; MS (ESI+) for C₂₃H₂₁Cl₂N₃O *m*/*z* (M+H)⁺.

### Preparation 72

### (1R,2S)-1-({6-cyclopropyl-5-[6-(dimethylamino)-4-methylpyridin-3-yl]-3-ethylpyrazin-2-yl}amino)-2,3-dihydro-1H-inden-2-ol

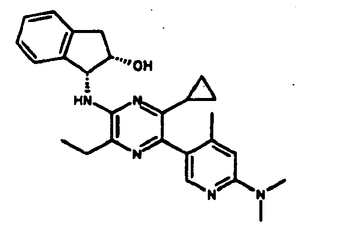

Following the procedure for the preparation of 5-(2,4-dichlorophenyl)-N-[(1 R,2S)-2-ethoxy-2,3-dihydro-1 H-inden-1-yl]-3,6-diethylpyrazin-2-amine but substituting (1R,2S)-1-[(5-bromo-6-cyclopropyl-3-ethylpyrazin-2-yl)amino]-2,3-dihydro-1H-inden-2-ol and making non-critical variations provided the title compound as a solid: ¹M NMR (CDCl₃) δ ; MS (ESI+) for C₂₃H₂₁Cl₂N₃O *m*/*z* (M+H)⁺.

### Preparation 73

### (1R2S)-1-{[3-cyclopropyl-5-(2,4-dichlorophenyl)-6-ethylpyrazin-2-yl]amino}-2,3-dihydro-1 H-inden-2-ol

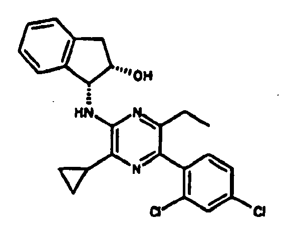

Following the procedure for the preparation of 5-(2,4-dichlorophenyl)-N-[(1R,2S)-2-ethoxy-2,3-dihydro-1H-inden-1-yl]-3,6-diethylpyrazin-2-amine but substituting (1R,2S)-1-[(5-bromo-3-cyclopropyl-6-ethylpyrazin-2-yl)amino]-2,3-dihydro-1H-inden-2-ol and making non-critical variations provided the title compound as a solid: ¹H NMR (CDCl₃) δ 0.94-1.02, 1.14-1.19, 1.26-1.30, 1.68-1.86, 2.06, 2.28-2.49, 3.08-3.32, 4.06-4.18, 4.83-4.87, 5.49, 5.62-5.66, 7.27-7.50; MS (ESI+) for C₂₄H₂₃Cl₂N₃O *m*/*z* 440(MH)⁺.

### Preparation 74

### (1R,2S)-1-({3-cyclopropyl-5-[6-(dimethylamino)-4-methylpyridin-3-yl]-6-ethylpyrazin-2-yl}amino)-2,3-dihydro-1H-inden-2-ol

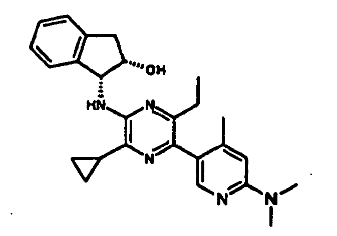

Following the procedure for the preparation of 5-(2,4-dichlorophenyl)-N-[(1R,2S)-2-ethoxy-2,3-dihydro-1H-inden-1-yl]-3,6-diethylpyrazin-2-amine but substituting (1R,2S)-1-[(5-bromo-3-cyclopropyl-6-ethylpyrazin-2-yl)amino]-2,3-dihydro-1H-inden-2-ol and making non-critical variations provided the title compound as a solid: ¹H NMR (CDCl₃) δ 0.88-1.00, 1.13-1.171.25-1.34, 1.77-1.82, 2.11, 2.52-2.58, 3.08-3.12, 3.23.-3.28, 4.82-4.85, 5.41, 5.60-5.62, 7.26-7.70, 7.97; MS (ESI+) for C₂₆H₃₁N₅O *m*/*z* 430(MH)⁺.

### Preparation 75

### (1R,2S)-1-{[5-(2-chloro-4-methoxyphenyl)-3-cyclopropyl-6-ethylpyrazin-2-yl]amino}-2,3-dihydro-1H-inden-2-ol

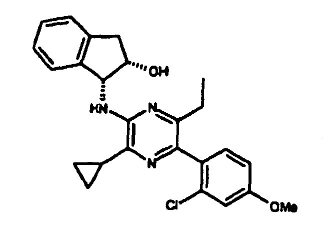

Following the procedure for the preparation of 5-(2,4-dichlorophenyl)-N-[(1R,2S)-2-ethoxy-2,3-dihydro-1H-inden-1-yl]-3,6-diethylpyrazin-2-amine but substituting (1R,2S)-1-[(5-bromo-3-cyclopropyl-6-ethylpyrazin-2-yl)amino]-2,3-dihydro-1H-inden-2-ol and making non-critical variations provided the title compound as a solid: ¹H NMR (CDCl₃) δ 0.95-1:01, 1.14-1.30, 1.80-1.84, 2.52, 2.76-2.84, 3.11-3.16, 3.26-3.31, 3.80-3.88, 4.12-4.17, 4.85-4.88. 5.36, 5.61-5.63, 6.88-7.03. 7.21-7.46; MS (ESI+) for C₂₅H₂₆ClN₃C₂ *m*/*z* 436(MH)⁺.

### Preparation 76

### (1R,25)-1-{[5-(2,4-dichlorophenyl)-6-ethyl-3-methylpyrazin-2-yl]amino}-2,3-dihydro-1H-inden-2-ol

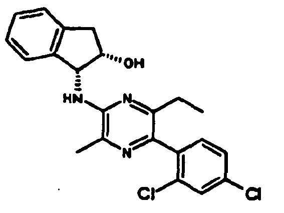

Following the procedure for the preparation of (1R,2S)-1-[(3,6-diethylpyrazin-2-yl)amino]-2,3-dihydro-1H-inden-2-ol but substituting (1R,2S)-1-[(5-bromo-6-ethyl-3-methylpyrazin-2-yl)amino]-2,3-dihydro-1H-inden-2-ol (A) and (1R,2S)-1-[(5-bromo-3-ethyl-6-methylpyrazin-2-yl)amino]-2,3-dihydro-1H-inden-2-ol (B) and making non-critical variations provided the title compound as a solid: ¹H NMR (CDCl₃) δ 1.06-1.09, 1.13-1.20, 2.36-2.38, 3.00-3.22, 4.76, 5.57, 7.19-7.42.

### Preparation 77

### (1R,2S)-1-{[5-(2,4-dichlorophenyl)-3-ethyl-6-methylpyrazin-2-yl]amino} -2,3-dihydro-1H-inden-2-ol

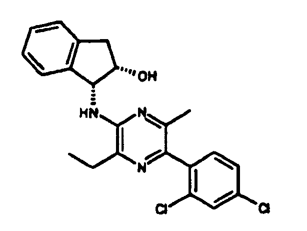

Following the procedure for the preparation of (1R,2S)-1-[(3,6-diethylpyrazin-2-yl)amino]-2,3-dihydro-1H-inden-2-ol but substituting (1R,2S)-1-[(5-bromo-6-ethyl-3-methylpyrazin-2-yl)amino]-2,3-dihydro-1H-inden-2-ol (A) and (1R,2S)-1-[(5-bromo-3-ethyl-6-methylpyrazin-2-yl)amino]-2,3-dihydro- 1H-inden-2-ol (B) and making non-critical variations provided the title compound as a solid: ¹H NMR (CDCl₃) δ 1.15-1.28, 2.36-2.52, 2.74; 3.09-3.30, 4.85, 5.68, 7.28-7.68.

### Example 38

### 3,6-dicyclopropyl-5-(2,4-dichlorophenyl)-N-[(1R,2S)-2-ethoxy 2,3-dihydro-1H-inden-1-yl]pyrazin-2-amine

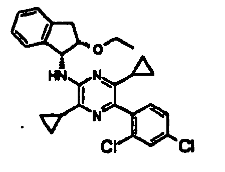

Following the procedure for the preparation of 5-(2,4-dichlorophenyl)-N-[(1R,2S)-2-ethoxy-2,3-dihydro-1H-inden-1-yl]-3,6-diethylpyrazin-2-amine but substituting (1 R,2S)-1-{[3,6-dicyclopropyl-5-(2,4-dichlorophenyl)pyrazin-2-yl]amino}-2,3-dihydro-1H-inden-2-ol and making non-critical variations provided the title compound as a solid: ¹H NMR (CDCl₃) δ 0.80-1.07, 1.17-1.20, 1.67, 1.79, 3.14-3.20, 3.46-3.50, 3.70-3.72, 4.35, 5.72, 5.97, 7.24-7.50; MS (ESI+) for C₂₇H₂₇Cl₂N₃O *m*/*z* 480 (M+H)⁺.

### Example 39

### 6-cyclopropyl-5-(2,4-dichlorophenyl)-N-[(1R,2S)-2-ethoxy-2,3-dihydro-1H-inden-1-yl]-3-methylpyrazin-2-amine

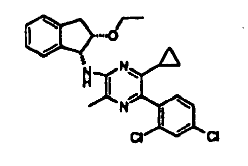

Following the procedure for the preparation of 5-(2,4-dichlorophenyl)-N-[(1R,2S)-2-ethoxy-2,3-dihydro-1H-inden-1-yl]-3,6-diethylpyrazin-2-amine but substituting (1R,2S)-1-{[6-cyclopropyl-5-(2,4-dichlorophenyl)-3-methylpyrazin-2-yl]amino}-2,3-dihydro-1H-inden-2-ol and making non-critical variations provided the title compound as a solid: ¹H NMR (CDCl₃) δ 0.75.-1.13, 1.53-1.59, 2.31, 2.98.3.09, 3.35-3.40, 3.57-3.65, 4.23-4.25, 5.37, 5.62, 7.12-7.42; MS (ESI+) for C₂₅H₂₅Cl₂N₃O *mlz* 454 (M+H)⁺.

### Example 40

### 3-cyclopropyl-5-(2,4-dichlorophenyl)-N-[(1R,2S)-2-ethoxy-2,3-dihydro-1H-inden-1-yl]-6-methylpyrazin-2-amine

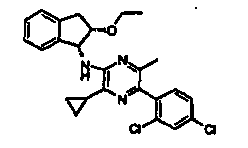

Following the procedure for the preparation of 5-(2,4-dichlorophenyl)-N-[(1R,2S)-2-ethoxy-2,3-dihydro-1H-inden-1-yl]-3,6-diethylpyrazin-2-amine but substituting(1R,2S)-1-{[3-cyclopropyl-5-(2,4-dichlorophenyl)-6-methylpyrazin-2-yl]amino}-2,3-dihydro-1H-inden-2-ol and making non-critical variations provided the title compound as a solid: ¹H NMR (CDCl₃) δ 0.87-1.07, 1.18-1.23, 1.82; 2.27, 3.10-3.16, 3.47-3.57, 3.66-3.7 6, 4.40, 5.83, 5.98, 7.25-7.50; MS (ESI+) for C₂₅H₂₅Cl₂N₃O *m*/*z* 454 (M+H)⁺.

### Example 41

### 6-cyclopropyl-5-(2,4-dichlorophenyl)-N-[(1R,2S)-2-ethoxy-2,3-dihydro-1H-inden-1-yl]-3-ethylpyrazin-2-amine

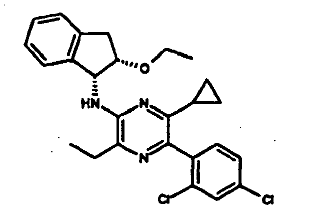

Following the procedure for the preparation of 5-(2,4-dichlorophenyl)-N-[(1R,2S)-2-ethoxy-2,3-dihydro-1H-inden-1-yl]-3,6-diethylpyrazin-2-amine but substituting (1R,2S)-1-{[6-cyclopropyl-5-(2,4-dichlorophenyl)-3-ethylpyrazin-2-yl]amino}-2,3-dihydro-1H-inden-2-ol and making non-critical variations provided the title compound as a solid: ¹H NMR (CDCl₃) δ 0.82-1.33, 1.58-1.71, 2.68-2.76, 3.06-3.19, 3.41-3.51, 3.65-3.75, 4.32-4.36, 5.52-5.54, 5.71-5.76, 7.23-7.52; MS (ESI+) for C₂₆H₂₇Cl₂N₃O *m*/*z* 469 (M+H)⁺.

### Example 42

### 5-(2-chloro-4-methoxyphenyl)-6-cyclopropyl-N-[(1R,2S)-2-ethoxy-2,3-dihydro-1H-inden-1 -yl]-3-ethylpyrazin-2-amine

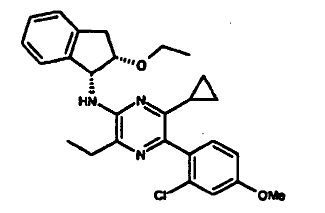

Following the procedure for the preparation of 5-(2,4-dichlorophenyl)-N-[(1R,2S)-2-ethoxy-2,3-dihydro-1H-inden-1-yl]-3,6-diethylpyrazin-2-amine but substituting (1 R,2S)-1- {[5-(2-chloro-4-methoxyphenyl)-6-cyclopropyl-3-ethylpyrazin-2-yl]amino}-2,3-dihydro-1H-inden-2-ol and making non-critical variations provided the title compound as a solid: MS (ESI+) for C₂₇H₃₀ClN₃O₂ *m*/*z* 465 (M+H)⁺.

### Example 43

### 6-cyclopropyl-5-[6-(dimethylamino)-4-methylpyridin-3-yl]-N-[(1R,2S)-2-ethoxy-2,3-dihydro-1H-inden-1-yl]-3-ethylpyrazin-2-amine

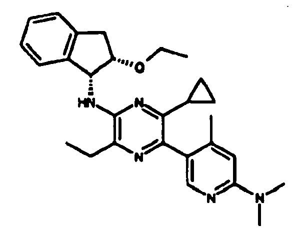

Following the procedure for the preparation of 5-(2,4-dichlorophenyl)-N-[(1R,2S)-2-ethoxy-2,3-dihydro-1H-inden-1-yl]-3,6-diethylpyrazin-2-amine but substituting (1R,2S)-1-({6-cyclopropyl-5-[6-(dimethylamino)-4-methylpyridin-3-yl]-3-ethylpyrazin-2-yl}amino)-2,3-dihydro-1H-inden-2-ol and making non-critical variations provided the title compound as a solid: ¹H NMR (CDCl₃) δ 0.81-1.19, 1.28-1.33, 2.67-2.73, 3.11-3.14, 3.42-3.51, 3.65-3.73, 4.32-4.35, 5.40-5.42, 5.72-5.75, 6.497.22-7.39, 8.19; MS (ESI+) for C₂₈H₃₅N₅O *m*/*z* 458 (M+H)⁺.

### Example 44

### 3-cyclopropyl-5-(2,4-dichlorophenyl)-N-[(1R2S)-2-ethoxy-2,3-dihydro-1H-inden-1-yl]-6-ethylpyrazin-2-amine

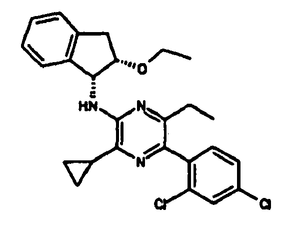

Following the procedure for the preparation of 5-(2,4-dichlorophenyl)-N-[(1R,2S)-2-ethoxy-2,3-dihydro-1H-inden-1-yl]-3,6-diethylpyrazin-2-amine but substituting (1R,2S)-1-{[3-cyclopropyl-5-(2,4-dichlorophenyl)-6-ethylpyrazin-2-yl]amino}-2,3-dihydro-1H-inden-2-ol and making non-critical variations provided the title compound as a solid: ¹H NMR (CDCl₃) δ 0.87-1.21, 1.58, 1.80-1.85, 2.51, 3.15-3.17, 3.46-3.56, 3.66-3.76, 4.40-4.45, 5.88, 6.04, 7.21-7.34, 7.47-7.50; MS (ESI+) for C₂₆H₂₇Cl₂N₃O *m*/*z* 469 (M+H)⁺.

### Example 45

### 3-cyclopropyl-5-[6-(dimethylamino)-4-methylpyridin-3-yl]-N-[(1R,2S)-2-ethoxy-2,3-dihydro-1H-inden-1-yl)-6-ethylpyrazin-2-amine

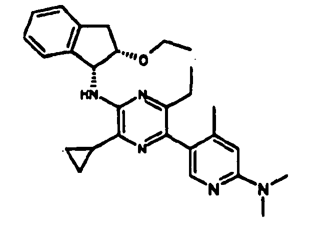

Following the procedure for the preparation of 5-(2,4-dichlorophenyl)-N-[(1R,2S)-2-ethoxy-2,3-dihydro-1H-inden-1-yl]-3,6-diethylpyrazin-2-amine but substituting (1R,2S)-1-{[3-cyclopropyl-5-(2,4-dichlorophenyl)-6-ethylpyrazin-2-yl]amino}-2,3-dihydro-1H-inden-2-ol and making non-critical variations provided the title compound as a solid: ¹H NMR (CDCl₃) δ 0.85.0.95, 1.16-1.34, 1.80-1.83, 2.53-2.58, 3.49-3.55, 3.68-3.72, 4.39-4.42, 5.32, 5.81-5.88, 6.46, 7.24-7.28, 7.51-7.53, 8.01; MS (ESI+) for C₂₈H₃₅N₅O *m*/*z* 458 (M+H)⁺.

### Example 46

### 5-(2-chloro-4-methoxyphenyl)-3-cyclopropyl-N-[(1R,2S)-2-ethoxy-2,3-dihydro-1H-inden-1-yl]-6-ethylpyrazin-2-amine

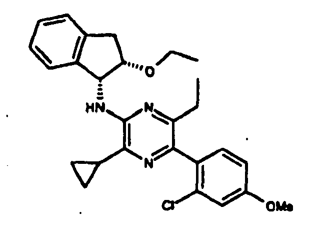

Following the procedure for the preparation of 5-(2,4-dichlorophenyl)-N-[(1R,2S)-2-ethoxy-2,3-dihydro-1H-inden-1-yl]-3,6-diethylpyrazin-2-amine but substituting (1R,2S)-1-{[3-cyclopropyl-5-(2,4-dichlorophenyl)-6-ethylpyrazin-2-yl]amino}-2,3-dihydro-1H-inden-2-ol and making non-critical variations provided the title compound as a solid: ¹H NMR (CDCl₃) δ 0.77-0.86, 0.97-1.23, 1.70-1.76, 2.42, 2.67-2.69, 3.00-3.11, 3.38-3.45, 3.57-3.64, 3.76-3.78,4.30-4.34,5.73-5.84,6.78-6.92, 7.13-7.19, 7.37-7.42; MS (ESI+) for C₂₇H₃₀ClN₃O₂ *m*/*z* 465(M+H)⁺.

### Example 47

### 5-(2-chloro-4-methoxyphenyl-3-cyclopropyl-N-[(1R,2S)-2-ethoxy-2,3-dihydro-1H-inden-1-yl]-6-ethylpyrazin-2-amine

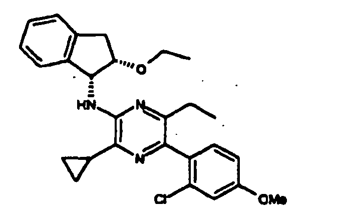

Following the procedure for the preparation of 5-(2,4-dichlorophenyl)-N-[(1R,2S)-2-ethoxy-2,3-dihydro-1H-inden-1-yl]-3,6-diethylpyrazin-2-amine but substituting (1 R,2S)-1- {[3-cyclopropyl-5-(2,4-dichlorophenyl)-6-ethylpyrazin-2-yl]amino}-2,3-dihydro-1H-inden-2-ol and making non-critical variations provided the title compound as a solid: ¹H NMR (CDCl₃) δ 0.77-0.86,0.97-1.23,1.70-1.78,2.42. 2.63-2.75, 3.00-3.11, 3.58-3.64, 3.76-3.78, 4.30-4.34, 5.73-5.84, 6.76-6.93, 7.13-7.19, 7.37-7.42; MS (ESI+) for C₂₇H₃₀ClN₃O₂ *m*/*z* 465 (M⁺H)⁺.

### Example 48

### 5-(2,4-dichlorophenyl)-N-[(1 R,2S)-2-ethoxy-2,3-dihydro-1H-inden-1-yl]-6-ethyl-3-methylpyrazin-2-amine

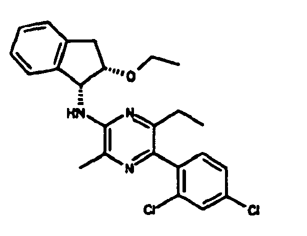

Following the procedure for the preparation of 5-(2,4-dichlorophenyl)-N-[(1R,2S)-2-ethoxy-2,3-dihydro-1H-inden-1-yl]-3,6-diethylpyrazin-2-amine but substituting (1R,2S)-1-{[5-(2,4-dichlorophenyl)-6-ethyl-3-methylpyrazin-2-yl]amino}-2,3-dihydro-1H-inden-2-ol and making non-critical variations provided the title compound as a solid: ¹H NMR (CDCl₃) δ 0.76-0.82, 1.02-1.34, 1.56-1.58, 2.24, 2.62, 3.38-3.55, 3.90-3.95, 5.22, 5.50-5.54, 6.14-6.16, 6.70-6.72, 7.04-7.62; MS (ESI+) for C₂₄H₂₅Cl₂N₃O *m*/*z* 443 (M+H)⁺.

### Example 49

### 5-(2,4-dichlorophenyl)-N-[(1R,2S)-2-ethoxy-2,3-dihydro-1H-inden-1-yl]-3-ethyl-6-methylpyrazin-2-amine

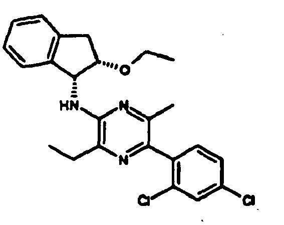

Following the procedure for the preparation of 5-(2,4-dichlorophenyl)-N-[(1R,25)-2-ethoxy-2,3-dihydro-1H-inden-1-yl]-3,6-diethylpyrazin-2-amine but substituting (1R2S)-1-{[5-(2,4-dichlorophenyl)-3-ethyl-6-methylpyrazin-2-yl]amino}-2,3-dihydro-1H-inden-2-ol and making non-critical variations provided the title compound as a solid: MS (ESI+) for C₂₄H₂₅Cl₂N₃O *m*/*z* 443 (M+H)⁺.

### Preparation 78

### N-[(1R,2R)-2-azido-2,3-dihydro-1H-inden-1-yl]-3,6-dicyclopropyl-5-(2,4-dichlorophenyl)pyrazin-2-amine

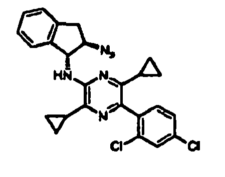

To a solution of (1R,2S)-1-{(3,6-dicyclopropyl-5-(2,4-dichlorophenyl)pyrazin-2-yl]amino}-2,3-dihydro-1H-inden-2-ol (0.76 g, 1.7 mmol) in toluene (10 mL) and THF (10 mL) was added PPh₃ (1.14 g, 4.57 mmol). The solution was cooled to 0 °C and HN3 (5.6 mL, 6.38 mmol) and a solution of diethylazodicarboxylate (0.69 mL, 4.37 mmol) in toluene (10 mL) were added. The reaction was stirred at rt overnight. The precipitates were removed by filtration and the filtrate was concentrated. Dissolve residue in 1 N HCl and EtOAc. Extract with EtOAc (3 x 30 mL). The aqueous phase was neutralized with sat. aq. NaHCO₃ to pH 7.5 followed by a small amount of 6 N NaOH to pH 9. The aqueous phase was extracted with CH₂Cl₂ (3 x 30 mL). The combined organic extracts were dried over MgSO₄, filtered and concentrated. Purify by biotage MPLC (90 g column, 29 - 39% ethyl acetate/hexanes) to afford 0.8 g (78%) of N-[(1R,2R)-2-azido-2,3-dihydro-1H-inden-1-yl]-3,6-dicyclopropyl-5-(2,4-dichlorophenyl)pyrazin-2-amine as a solid: ¹H NMR (CDCl₃) δ 0.74-1.10,1.63-1.71, 2.97-3.03, 3.36-3.42, 4.09-4.13, 4.26-4.29, 5.05, 5.57, 7.25-7.53; MS (ESI+) for C₂₅H₂₂Cl₂N₆ *m*/*z* 477 (M+H)⁺.

### Preparation 79

### (1R,2R)-N'-[3,6-dicyclopropyl-5-(2,4-dichlorophenyl)pyrazin-2-yl]-2,3-dihydro-1H-indene-1,2-diamine

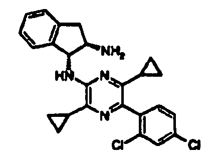

To a solution of N-[(1R,2R)-2-azido-2,3-dihydro-1H-inden-1-yl]-3,6-dicyclopropyl-5-(2,4-dichlorophenyl)pyrazin-2-amine (0.55 g, 1.15 mmol) in anhydrous THF (30 mL) was added PPh₃ (0.396 g, 1.51 mmol). The resulting mixture was stirred at rt for 3 hr. To the solution was added water (0.25 mL, 13.9 mmol). The reaction is then allowed to stir overnight at rt. The mixture is concentrated, and the resulting solid is dissolved in MeOH. Purify by passage over Biorad acidic resin by applying the MeOH solution onto the resin and rinsing the resin with MeOH. The resin was then rinsed with 5% triethylamine/MeOH solution to afford 0.52 g (75%) of (1R,2R)-N'-[3,6-dicyclopropyl-5-(2,4-dichlorophenyl)pyrazin-2-yl]-2,3-dihydro-1H-indene-1,2-diamine as a solid: ¹H NMR (CDCl₃) δ 0.85.1.12, 1.64-1.76, 1.89, 2.68-2.82, 3.31-3.39, 3.66-3.72, 5.16, 5.33, 7.28-7.53; MS (ESI+) for C₂₅H₂₄Cl₂N₄ *m*/*z* 451 (M+H)⁺.

### Example 50

### (1R,2R)-N¹-[3,6-dicyclopropyl-5-(2,4-dichlorophenyl)pyrazin-2-yl]-N²-ethyl-2,3-dihydro-1H-indene-1,2-diamine

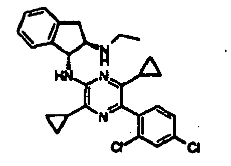

To a solution of (1R,2R)-N¹-[3,6-dicyclopropyl-5-(2,4-dichlorophenyl)pyrazin-2-yl]-2,3-dihydro-1H-indene-1,2-diamine (0.15 g, 0.33 mmol) in MeOH (3.3 mL) was sequentially added acetaldehyde (22 mg, 0.5 mmol) and AcOH (5 drops). This mixture was stirred for 30 min before addition of NaBH₃CN (1 M solution in THF, 0.6 mL, 0.6 mmol). Stir at rt overnight. Add additional acetic acid (2 drops), acetaldehyde (29 µL, 0.5 mmol) and NaBH₃CN (1 M solution in THF, 0.6 mL, 0.6 mmol). After 2 hr, add additional acetic acid (2 drops), acetaldehyde (29 µL, 0.5 mmol) and NaBH₃CN (1 M solution in THF, 0.6 mL, 0.6 mmol). After 3 hr, dilute mixture with EtOAc and wash with sat. aq. NaHCO₃ and extract with EtOAc (3 x 40 mL). The combined organic extracts were dried over MgSO₄, filtered and concentrated. Purify by biotage MPLC (90 g column, 100% ethyl acetate) to afford 0.16 g (44%) of (1R,2R)-N¹-[3,6-dicyclopropyl-5-(2,4-dichlorophenyl)pyrazin-2-yl]-N²-ethyl-2,3-dihydro-1H-indene-1,2-diamine as a solid: ¹H NMR (CDCl₃) δ 0.84-1.05, 1.62-1.75, 2.78-2.86, 3.31-3.37, 3.52, 5.22, 5.52, 7.28-7.51; MS (ESI+) for C₂₇H₂₈Cl₂N₄ *m*/*z* 479 (M+H)⁺.

### Example 51

### N-((1R,2R)-1-{[3,6-dicyclopropyl-5-(2,4-dichlorophenyl)pyrazin-2-yl]amino} -2,3-dihydro-1H-inden-2-yl)acetamide

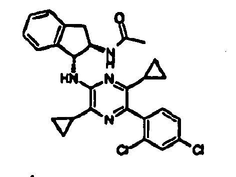

To a solution of (1R,2R)-N¹-[3,6-dicyclopropyl-5-(2,4-dichlorophenyl)pyrazin-2-yl]-2,3-dihydro-1H-indene-1,2-diamine (0.10 g, 0.22 mmol) in CHCl₃ (1 mL) at 0°C under N₂ was added trichloroacetone (30 µL, 0.26 mmol). The mixture was allowed to slowly warm to rt for 1.5 hr. The reaction was then heated at 60°C for 1.5 h. Cool to rt, add additional trichloroacetone (10 µL) and continue heating at 60°C for 2 hr. The reaction was concentrated and purified by biotage MPLC (90 g column, 80% ethyl acetate/hexanes) to afford 0.11 g (55%) ofN-((1R,2R)-1-{[3,6-dicyclopropyl-5-(2,4-dichlorophenyl)pyrazin-2-yl]amino}-2,3-dihydro-1H-inden-2-yl)acetamide as a solid: ¹H NMR (CDCl₃) δ 0.84-1.01, 1.61-1.81, 2.04, 2.80-2.86, 3.58-3.64, 4.07-4.13, 4.46, 5.56, 6.56, 7.28-7.51; MS (ESI+) for C₂₇H₂₆Cl₂N₄O *m*/*z* 493 (M+H)⁺.

### Example 52

### (1R,2R)-N'-[3,6-dicyclopropyl-5-(2,4-dichlorophenyl)pyrazin-2-yl]-N²-(2-methoxyethyl)-2,3-dihydro-1H-indene-1,2-diamine

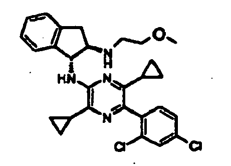

To a solution of (1R,2R)-N¹-[3,6-dicyclopropyl-5-(2,4-dichlorophenyl)pyrazin-2-yl]-2,3-dihydro-1H-indene-1,2-diamine (0.10 g, 0.22 mmol) in MeOH (2 mL) containing acetic acid (2 drops) and methoxyacetaldehye (49 mg, 0.66 mmol) was added NaBH₃CN (1 M solution in THF, 0.8 mL, 0.8 mmol). The mixture was stirred at rt overnight. Dilute with sat. aq. NaHCO₃ and extract ED with EtOAc (3 x 30 mL). The combined organic extracts were dried over MgSO₄, filtered and concentrated to afford an oil that was purified by biotage MPLC (90 g column, 10% ethyl acetate/hexanes) to afford 0.11 g (20%) of (1R,2R)-N¹-[3,6-dicyclopropyl-5-(2,4-dichlorophenyl)pyrazin-2-yl]-N²-(2-methoxyethyl)-2,3-dihydro-1H-indene-1,2-diamine as a solid: ¹H NMR(CDCl₃) δ 0.60-1.01,1.45.1.70,2.83-2.99, 3.22-3.33, 3.47-3.56, 5.19, 5.53, 7.11-7.42; MS (ESI+) for C₂₈H₃₀Cl₂N₄O *m*/*z* 509 (M+H)⁺.

### Example 53

### 5-(2,4-dichlorophenyl]-N-[(1S,2R)-2-ethoxy-2,3-dihydro-1H-inden-1-yl]-3,6-diethylpyrazin-2-amine

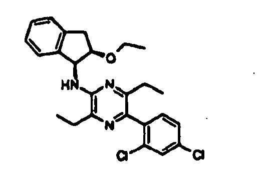

Following the procedure for the preparation of 5-(2,4-dichlorophenyl)-3,6-diethyl-N-[(1S,2R)-2-methoxy-2,3-dihydro-1H-inden-1-yl]pyrazin-2-amine but substituting (1S, 2R)-1- {[5-(2,4-dichlorophenyl)-3,6-diethylpyrazin-2-yl]amino} -2,3-dihydro-1H-inden-2-ol and iodoethane and making non-critical variations provided the title compound as a solid: MS (ESI+) for C₂₅H₂₇Cl₂N₃O *m*/*z* 457 (M+H)⁺.

### Example 54

### 5-(2,4-dichlorophenyl)-N-[(1S,2S)-2-ethoxy-2,3-dihydro-1H-inden-1-yl]-3,6-diethylpyrazin-2-amine

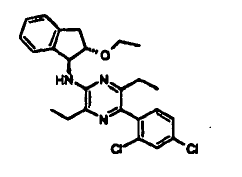

Following the procedure for the preparation of 5-(2,4-dichlorophenyl)-3,6-diethyl-N-[(1S,2S)-2-methoxy-2,3-dihydro-1H-inden-1-yl]pyrazin-2-amine but substituting (1S,2S)-1-{[5-(2,4-dichlorophenyl)-3,6-diethylpyrazin-2-yl]amino}-2,3-dihydro-1H-inden-2-ol and iodoethane and making non-critical variations provided the title compound as a solid: ¹H NMR (CDCl₃) δ 0.81, 1.17-1.21, 1.30-1.34, 1.51-1.59, 1.98, 3.90-3.96, 4.02-4.08, 6.71-6.73, 7.04-7.07, 7.19; MS (ESI+) for C₂₅H₂₇Cl₂N₃O *m*/*z* 457 (M+H)⁺.

### Example 55

### 5-(2,4-dichlorophenyl)-3,6-diethyl-N-[(1S,2S)-2-(2-fluoroethoxy)-2,3-dihydro-1H-inden-1-yl]pyrazin-2-amine

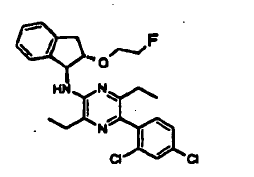

Following the procedure for the preparation of 5-(2,4-dichlorophenyl)-3,6-diethyl-N-[(1S,2S)-2-methoxy-2,3-dihydro-1H-inden-1-yl]pyrazin-2-amine but substituting (1S,2S)-1-{[5-(2,4-dichlorophenyl)-3,6-diethylpyrazin-2-yl]amino} -2,3-dihydro-1H-inden-2-ol and 1-bromo-2-fluoroethane and making non-critical variations provided the title compound as a solid: MS (ESI+) for C₂₅H₂₆Cl₂FN₃O *m*/*z* 475 (M+H)⁺.

### Preparation 80

### (1R,2S)-1-[(6-chloropyrazin-2-yl)amino]-2,3-dihydro-1H-inden-2-ol

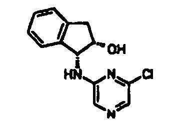

To a solution of 2,6-dichloropyrazine (5.0 g, 34 mmol) in n-BuOH (30 mL) was added triethylamine (6.8 g, 9.4 mL, 67 mmol) and (1R,2S)-(+)-cis-1-amino-2-indanol (5.0 g, 34 mmol). The mixture was heated at 115°C overnight. The solution was cooled to rt and concentrated. Dissolve material in EtOAc (200 mL) and remove insoluble material by vacuum filtration. Wash filtrate sequentially with 4 N NaOH and sat. aq. NaCl. Dry organic extract over MgSO₄, filter and concentrate. Purify by biotage MPLC (90 g column, 10% ethyl acetate/hexanes) to afford 4.85 g (57%) of (1R,2S)-1-[(6-chloropyrazin-2-yl)amino]-2,3-dihydro-1H-inden-2-ol as a solid: ¹H NMR (CDCl₃) δ 2.83-2.87, 3.08-3.13, 3.32, 4.54, 5.11, 5.28-5.31, 7.18-7.28, 7.73, 8.12; MS (ESI+) for C₁₃H₁₂CIN₃O *mlz* 262 (M+H)⁺.

### Preparation 81

### methyl 6-{[(1R,2S)-2-hydroxy-2,3-dihydro-1H-inden-1-yl]amino}pyrazine-2-carboxylate

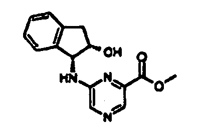

An autoclave was charged with (1R,2S)-1-[(6-chloropyrazin-2-yl)amino]-2,3-dihydro-1H-inden-2-ol (1.7 g, 6.5 mmol), MeOH (20 mL), triethylamine (1.1 mL, 7.8 mmol), bis(diphenylphosphino)ferrocene (21.6 mg, 39 µmol), PdCl₂(PhCN)₂ (12.5 mg, 32.5 µmol) and dried, powdered 4 A molecular sieves (2.8 g). Evacuate and charge (3x) with carbon monoxide (350 psi). Heat at 145°C for 18 hours. Cool to rt, release pressure and remove solids by filtration. The mixture was diluted with CH₂Cl₂ and washed with water. The organic extract was dried over MgSO₄, filtered and concentrated. Purify by biotage MPLC (90 g column, 50% ethyl acetate/hexanes) to afford 1.4 g (77%) of methyl 6-{[(1R,2S)-2-hydroxy-2,3-dihydro-1H-inden-1-yl]amino}pyrazine-2-carboxylate as a solid: ¹H NMR (CDCl₃) δ 2.84-2.88, 3.09-3.14, 3.85, 4.55-4.57, 5.41-5.44, 7.14-7.28, 7.61-7.63, 8.30, 8.39; MS (ESI+) for C₁₅H₁₅N₃O₃ m/z 286 (M+H)⁺.

### Preparation 82

### methyl 3-bromo-6-{[(1R,2S)-2-hydroxy-2,3-dihydro-1H-inden-1-yl]amino}pyrazine-2-carboxylate

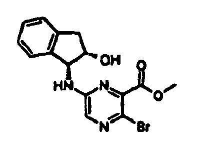

To a solution of methyl 6-{[(1R,2S)-2-hydroxy-2,3-dihydro-1H-inden-1-yl]amino}pyrazine-2-carboxylate (1.0 g, 3.5 mmol) in CH₂Cl₂ (35 mL) was added N-bromsuccinimide (0.69 g, 3.86 mmol). Stir at t for 2 hr. Wash the reaction with sat. aq. NaHCO₃. The organic extract was dried over MgSO₄, filtered and concentrated. Purify by biotage MPLC (90 g column, 20% ethyl acetate/hexanes) to afford 380 mg (30%) of methyl 3-bromo-6-{[(1R,2S)-2-hydroxy-2,3-dihydro-1H-inden-1-yl]amino}pyrazine-2-cartioxylate as a solid: ¹H NMR (CDCl₃) δ 3.01-3.06, 3.24-3.29, 4.00, 4.75-4.78, 5.45-5.46, 7.25-7.32, 7.94; MS (ESI+) for C₁₅H₁₄BrN₃O₃ *m*/*z* 386 (M+H)⁺.

### Preparation 83

### methyl 3-(2,4-dichlorophenyl)-6-{[(1R,2S)-2-hydroxy-2,3-dihydro-1H-inden-1-yl]amiino}pyrazine-2-carboxylate

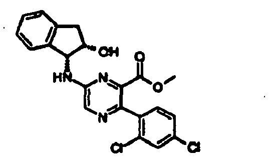

Charge a Kimble vial with methyl 3-bromo-6-{[(1R,2S)-2-hydroxy-2,3-dihydro-1H-inden-1-yl]amino}pyrazine-2-carboxylate (0.45 g, 1.14 mmol), Pd(PPh₃)₄ (198 mg, 0.17 mmol), and 2,4-dichlorophenylboronic acid (325 mg, 1.71 mmol). Flush with N₂ and add DMF (4 mL) and 2 M Na₂CO₃ (1.1 mL). The resulting mixture was heated at 85 °C overnight. The mixture was diluted with sat. aq. NaHCO₃. Extract with Et2O. The organic extract was dried over MgSO₄, filtered and concentrated Purify by biotage MPLC (90 g column, 30% ethyl acetate/hexanes) to afford 290 mg (59%) of methyl 3-(2,4-dichlorophenyl)-6-{[(1R,2S)-2-hydroxy-2,3-dihydro-1H-inden-1-yl]amino}pyrazine-2-carboxylate as a solid: ¹H NMR (CDCl₃) δ 3.06-3.09, 3.27-3.32, 3.80, 4.09-4.15, 4.84, 5.58, 7.28-7.49, 8.25; MS (ESI+) for C₂₁H₁₇Cl₂N₃O₃ *m*/*z* 430 (M+H)⁺.

### Preparation 84

### methyl 5-bromo-3-(2,4-dichlorophenyl)-6-{[(1R,2S)-2-hydroxy-2,3-dihydro-1H-inden-1-yl]amino}pyrazine-2-carboxylate

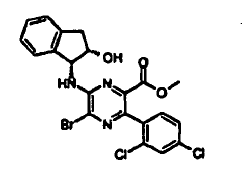

Following the procedure for the preparation of methyl 3-bromo-6-{[(1R,2S)2-hydroxy-2,3-dihydro-1H-inden-1-yl]amino}pyrazine-2-carboxylate but substituting methyl 3-(2,4-dichlorophenyl)-6-{[(1R,2S)-2-hydroxy-2,3-dihydro-1H-inden-1-yl]amino}pyrazine-2-carboxylate and making non-critical variations provided the title compound as a solid: ¹H NMR (CDCl₃) δ 2.99-3.04, 3.20-3.25, 3.70, 3.99-4.08, 4.77, 5.59-5.62, 6.11, 7.24-7.38; MS (ESI+) for C₂₁H₁₆BrCl₂N₃O₃ *m*/*z* 509 (M+H)⁺.

### Preparation 85

### ethyl 3-(2,4-dichlorophenyl)-6-{[(1R,2S)-2-hydroxy-2,3-dihydro-1H-inden-1-yl]amino} -5-methoxypyrazine-2-carboxylate

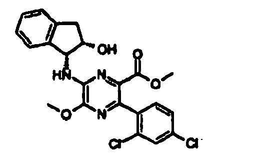

To a solution of methyl 5-bromo-3-(2,4-dichlorophenyl)-6-{[(1R,2S)-2-hydroxy-2,3-dihydro-1H-inden-1-yl]amino}pyrazine-2-carboxylate (0.19 g, 0.37 mmol) in MeOH (3.7 mL) was added NaOMe (60 mg, 1.1 mmol). The mixture was heated at reflux for 3 hr. Cool to rt, and dilute with EtOAc. Wash with sat. aq. NaHCO₃. Dry organic extract over MgSO₄, filter and concentrate. Purify by biotage MPLC (90 g column, 25% ethyl acetate/hexanes) to afford 0.15 g (73%) of ethyl 3-(2,4-dichlorophenyl)-6-{[(1R,2S)-2-hydroxy-2,3-dihydro-1H-inden-1-yl]amino}-5-methoxypyrazine-2-carboxylate as a solid: ¹H NMR (CDCl₃) δ 3.07-3.12, 3.25-3.31, 3.76, 4.04, 4.85-4.89, 5.68-5.76, 7.28-7.49; MS (ESI+) for C₂₂H₁₉Cl₂N₃O₄ *m*/*z* 460 (M+H)⁺.

### Example 56

### methyl 3-(2,4-dichlorophenyl)-6-{[(1R,2S)-2-ethoxy-2,3-dihydro-1H-inden-1-yl]amino}-5-methoxypyrazine-2-carboxylate

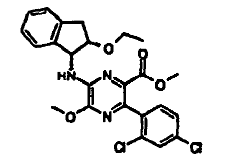

Following the procedure for the preparation of 5-(2,4-dichlorophenyl)-3,6-diethyl-N-[(1S,2S)-2-methoxy-2,3-dihydro-1H-inden-1-yl]pyrazin-2-amine but substituting methyl 3-(2,4-dichlorophenyl)-6-{[(1R,2S)-2-hydroxy-2,3-dihydro-1H-inden-1-yl]amino}-5-methoxypyrazine-2-carboxylate and making non-critical variations provided the title compound as a solid: ¹H NMR (CDCl₃) δ 1.13-.20, 3.15, 3.55-3.58, 3.66-3.70, 3.76, 4.02, 4.41, 5.75-5.79, 6.16, 7.25-7.34, 7.48, 7.59-7.61; MS (ESI+) for C₂₄H₂₃Cl₂N₃O₄ *m*/*z* 488 (M+H)⁺.

### Example 57

### methyl 6-{[(1R,2S)-2-(acetyloxy)-2,3-dihydro-1H-inden-1-yl]amino}-3-(2,4-dichlorophenyl)-5-methoxypyrazine-2-carboxylate

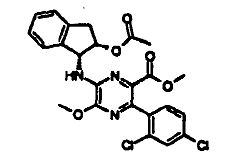

Following the procedure for the preparation of (1R,2S)-1-{[5-(2,4-dichlorophenyl)-3,6-diethylpyrazin-2-yl]amino}-2,3-dihydro-1H-inden-2-yl acetate but substituting methyl 3-(2,-dichlorophenyl)-6- {[(1R,2S)-2-ethoxy-2,3-dihydro-1H-inden-1-yl]amino}-5-methoxypyrazine-2-carboxylate and making non-critical variations provided the title compound as a solid: ¹H NMR (CDCl₃) δ 2.03, 3.10-3.143.31-3.36, 3.75, 4.05, 5.68-5.77, 6.07-6.11, 7.31-7.48; MS (ESI+) for C₂₄H₂₁Cl₂N₃O₅ *m*/*z* 502 (M+H)⁺.

### Preparation 86

### (1R,2S)-1-[(6-methoxypyrazin-2-yl)amino]-2,3-dihydro-1H-inden-2-ol

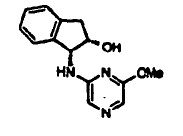

To a solution of (1R,2S)-1-[(6-chlompyrazin-2-yl)amino]-2,3-dihydro-1H-inden-2-ol (2 g, 7.6 mmol) in MeOH (26 mL) was added a solution of NaOMe (1.3 g, 23 mmol) in MeOH (40 mL). The mixture was heated at reflux for 24 hr. Additional NaOMe (1.3 g) was added. Continue heating at reflux for 24 hr, and then add additional NaOMe (1.3 g). Continue heating at reflux for 24 hr and cool to rt. Dilute with EtOAc and was with sat. aq. NaHCO₃. The solids were removed by filtration. Separate phases and wash organic extract with additional sat. aq. NaHCO₃. Dry organic extracts over MgSO₄, filter and concentrate. Purify by biotage MPLC (90 g column, 25% ethyl acetate/hexanes) to afford 1.4 g (71%) of (1R,2S)-1-[(6-methoxypyrazin-2-yl)amino]-2,3-dihydro-1H-inden-2-ol as an oil: ¹H NMR (CDCl₃) 8 3.04-3.08, 3.23-3.29, 3.91, 4.75-4.78, 5.18, 5.42-5.45, 7.25-7.34, 7.57-7.61; MS (ESI+) for C₁₄H₁₃N₃O₂ *m*/*z* 258 (M+H)⁺.

### Preparation 87

### (1R,2S)-1-[(5-bromo-6-methoxypyrazin-2yl)amino]-2,3-dihydro-1H-inden-2-ol

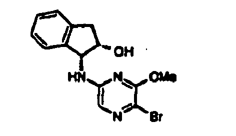

Following the procedure for the preparation of methyl 3-bromo-6-{[(1R,2S)-2-hydroxy-2,3-dihydro-1H-inden-1-yl]amino}pyrazine-2-carboxylate but substituting methyl (1R,2S)-1-[(6-methoxypyrazin-2-yl)amino]-2,3-dihydro-1H-inden-2-ol and making non-critical variations provided the title compound as a solid: ¹H NMR (CDCl₃) δ 2.07, 3.03-3.07, 3.25-3.30, 3.98, 4.76, 5.38, 7.26-7.32, 7.45; MS (ESI+) for C₁₄H₁₄BrN₃O₂ *m*/*z* 336 (M+H)⁺.

### Preparation 88

### (1R,2S)-1-{[5-2,4-dichlorophenyl)-6-methoxypyrazin-2-yl]amino}-2,3-dihydro-1H-inden-2-ol

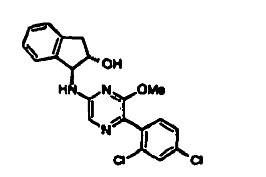

A Kimble vial was charged with (1R,2S)-1-[(5-bromo-6-methoxypyrazin-2-yl)amino]-2,3-dihydro-1H-inden-2-ol (0.55 g, 1.63 mmol), Pd₂(dba)₃ (33 mg, 35 µmol), dicyclohexyl[2-(9-phenanthryl)phenyl]phosphine (64 mg), 2,4-dichlomphenylboronic acid (567 mg, 2.67 mmol), K₃PO₄ (1.13 g, 5.34 mmol) and toluene (15 mL). The vial was flushed with N2, sealed and heated at 110 °C for 6 hr. Cool to rt, add additional Pd₂(dba)₃ (33 mg) and 2,4-dichlorophenylboronic acid (0.5 g, 2.7 mmol). Continue heating overnight. Add additional Pd₂(dba)₃ (33 mg) and 2,4-dichlorophenylboronic acid (0.5 g, 2.7 mmol) and continue heating for 5 hr. Add additional Pd₂(dba)₃ (33 mg) and 2,4-dichlorophenylboronic acid (0.5 g, 2.7 mmol) and continue heating overnight. The reaction was cooled to rt, diluted with CH₂Cl₂, washed with sat. aq NaHCO₃ and sat. aq. NaCl. Dry organic extract over MgSO₄, filter and concentrate. Purify by biotage MPLC (90 g column, 30% ethyl acetate/hexanes) to afford 0.2 g (31%) of (1R,2S)-1-{[5-(2,4-dichrorophenyl)-6-methoxypyrazin-2-yl]amino}-2,3-dihydro-1H-inden-2-ol as an oil: ¹H NMR (CDCl₃) δ3.06-3.10, 3.26-3.32, 3.92, 4.79, 5.33, 5.49-5.50, 7.28-7.41, 7.50, 7.74 ; MS (ESI+) for C₂₀H₁₇Cl₂N₃O₂ *m*/*z* 402 (M+H)⁺.

### Preparation 89

### (1R,2S)-1-{[3-bromo-5-(2,4-dichlorophenyl)-6-methoxypyrazin-2-yl]amino}-2,3-dihydro-1H-inden-2-ol

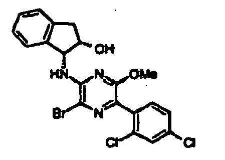

Following the procedure for the preparation of methyl 3-bromo-6-{[(1R,2S)-2-hydroxy-2,3-dihydro-1H-inden-1-yl]amino}pyrazine-2-carboxylate but substituting (1R,2S)-1-{[5-(2,4-dichlorophenyl)-6-methoxypyrazin-2-yl]amino}-2,3-dihydro-1H-inden-2-ol and making non-critical variations provided the title compound as a solid: ¹H NMR (CDCl₃) δ 3.09-3.13, 3.29-3.34, 3.92, 4.81, 5.60-5.63, 5.99, 7.33-7.42, 7.49; MS (ESI+) for C₂₀H₁₆BrCl₂N₃O₂ *m*/*z* 479 (M+H)⁺.

### Preparation 90

### (1R,2S)-1-{[5-(2,4-dichlorophenyl)-6-methoxy-3-vinylpyrazin-2-yl]amino}-2,3-dihydro-1H-inden-2-ol

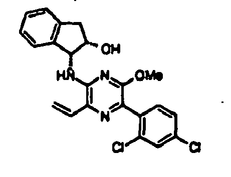

To a solution of (1R,2S)-1-{[3-bromo-5-(2,4-dichlorophenyl)-6-methoxypyrazin-2-yl]amino}-2,3-dihydro-1H-inden-2-ol (150 mg, 0.31 mmol) in DMF (1.6 mL) is added Pd(PPh₃)₄ (7 mg, 6 µmol), and tributyl vinyl tin (114 mg, 0.36 mmol). The reaction is heated at 120 °C overnight. Sat. aq. KF (15 mL) is added and the mixture is stirred at rt for 1 hr. Extract with CH₂Cl₂ (5 x 30 mL). The combined organic extracts were dried over MgSO₄, filtered and concentrated. Purify by biotage MPLC (90 g column, 15% ethyl acetate/hexanes) to afford 132 mg (38%) of (1R,2S)-1-{ [5-(2,4-dichlorophenyl)-6-methoxy-3-vinylpyrazin-2-yl]amino}-2,3-dihydro-1H-inden-2-ol as a solid: ¹H NMR (CDCl₃) δ 3.08-3.12, 3.28-3.34, 3.93, 4.82, 5.46-5.49, 5.54-5.67, 6.09-6.13, 6.77-6.84, 7.28-7.50; MS (ESI+) for C₂₀H₁₇Cl₂N₃O₂ *m*/*z* 402 (M+H)⁺.

### Example 58

### 5-(2,4-dichlorophenyl)-N-[(1R,2S)-2-ethoxy-2,3-dihydro-1H-inden-1-yl]-6-methoxy-3-vinylpyrazin-2-amine

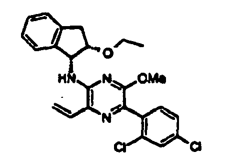

Following the procedure for the preparation of 5-(2,4-dichlorophenyl)-3,6-diethyl-N-[(1S,2S)-2-methoxy-2,3-dihydro-1H-inden-1-yl]pyrazin-2-amine but substituting (1R,2S)-1-{[5-(2,4-dichlorophenyl)-6-methoxy-3-vinylpyrazin-2-yl]amino}-2,3-dihydro-1H-inden-2-ol and making non-critical variations provided the title compound as a solid: ¹H NMR (CDCl₃) δ 1.11-1.14, 3.04-3.08, 3.37-3.44, 3.60-3.68, 3.84, 4.28-4.30, 5.34-5.37, 5.68-5.71, 5.90-5.99, 6.66-6.73, 7.14-7.26, 7.32-7.40; MS (ESI+) for C₂₄H₂₃Cl₂N₃O₂ *m*/*z* 456 (M+H)⁺.

## Claims

1. A compound of Formula IV 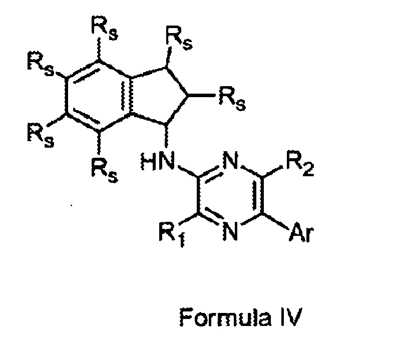 or a stereoisomer thereof, or a pharmaceutically acceptable salt thereof, wherein in Formula IV,
Ar is selected from aryl, substituted aryl, heteroaryl, or substituted heteroaryl;
m is 0,1 or 2;
R₁ and R₂ are independently selected from halogen, -NO₂, -CN, -Rₐ, -ORₐ, -S(O)ₘRₐ, -NRₐRₐ, -C(O)NRₐRₐ, -C(S)NRₐRₐ -S(O)ₘNRₐRₐ, -NRₐS(O)ₘRₐ, -NRₐC(O)ORₐ, -OC(O)NRₐRₐ, -NRₐC(O)NRₐRₐ, -NRₐC(S)NRₐRₐ, -C(O)ORₐ, -C(S)ORₐ, or -OC(O)ORₐ;
Rₛ each is independently selected from halogen, -NO₂, -CN, -Rₐ, -ORₐ, -S(O)ₘRₐ, -NRₐRₐ, -C(O)NRₐR_{a,} -C(S)NRₐRₐ, -S(O)ₘNRₐRₐ, -NRₐS(O)ₘRₐ, -NRₐC(O)ORₐ, -OC(O)NRₐRₐ, -NRₐC(O)NRₐRₐ, -NRₐC(S)NRₐRₐ, -C(O)ORₐ, -C(S)ORₐ, or -OC(O)ORₐ;
Rₐ each is independently selected from H, alkyl, cycloalkyl, haloalkyl, aryl, heteroaryl, or heterocycloalkyl optionally substituted with 1 to 5 of Rₜ, -ORₜ, -S(O)ₘRₜ, NRₜRₜ, oxo (=O), thione (=S), phenyl, heteroaryl, or heterocycloalkyl where phenyl, heteroaryl, and heterocycloalkyl are optionally substituted with 1 to 5 independently taken from Rₜ; and
Rₜ each is independently selected from H, halogen, -NO₂, -NH₂, -OH, -SH, -CN, -C(O)NH₂, -C(O)-NHalkyl, -C(O)Nalkylalkyl, -Oalkyl, NHalkyl, Nalkylalkyl, -S(O)ₘalkyl, SO₂NH₂, SO₂NHalkyl and SO₂Nalkylalkyl, alkyl, cycloalkyl, haloalkyl, phenyl, benzyl, heteroaryl, or heterocycloalkyl where phenyl, benzyl heteroaryl and heterocycloalkyl may be optionally substituted with alkyl or halogen, wherein "substituted aryl" means an aryl group optionally substituted with 1-5 substituents independently selected from halogen, -NO₂, -CN, -Rₐ, -ORₐ, -S(O)ₘRₐ, -NRₐRₐ, -C(O)NRₐRₐ, -C(S)NRₐRₐ, -S(O)ₘNRₐRₐ, -NRaS(O)ₘRₐ, -NRₐC(O)ORₐ, -OC(O)NRₐRₐ, -NRₐC(O)NRₐRₐ, -NRₐC(S)NRₐRₐ, -C(O)ORₐ, -C(S)ORₐ, and -OC(O)ORₐ;
"heteroaryl" means a radical attached via a ring carbon or nitrogen atom of a monocyclic aromatic ring containing five or six ring atoms consisting of carbon and 1, 2, 3, or 4 heteroatoms each selected from the group consisting of non-peroxide O, S, N, with appropriate bonding to satisfy valence requirements as well as a radical (attachment at either carbon or nitrogen) of a fused bicyclic heteroaromatic of about eight to ten ring atoms,
"substituted heteroaryl" means a heteroaryl group having 1-5 substituents independently selected from halogen, -NO₂, -CN, -Rₐ, -ORₐ, -S(O)ₘRₐ, -NRₐRₐ, -C(O)NRₐRₐ, -C(S)NRₐRₐ, -S(O)ₘNRₐRₐ, -NRₐS(O)ₘRₐ, -NRₐC(O)ORₐ, -OC(O)NRₐRₐ, -NRₐC(O)NRₐRₐ, -NRₐC(S)NRₐRₐ, -C(O)ORₐ, -C(S)ORₐ and -OC(O)ORₐ;
"alkyl" means both straight and branched chain moieties having from 1-10 carbon atoms optionally containing one or more double or triple bonds;
"cycloalkyl" means a monocyclic or bicyclic alkyl moiety having from 3-10 carbon atoms optionally containing 1 to 2 double bonds provided that the moiety is not aromatic;
"haloalkyl" means an alkyl moiety having from 1-10 carbon atoms and having 1 to (2v+1) independently selected halogen substituent(s) where v is the number of carbon atoms in the moiety;
"heterocycloalkyl", unless otherwise specified, means a 4 to 8 membered monocyclic ring or bicyclic ring, wherein at least one carbon atom is replaced with a heteromember selected from oxygen, nitrogen. -NH-, or -S(O)ₘ- wherein m is zero, 1, or 2, optionally containing from one to three double bonds, provided that the molecule is not aromatic; and provided that ring attachment can occur at either a carbon or nitrogen atom;
"substituted heterocycloalkyl" is a heterocycloalkyl group having 1-5 substituents independently selected from halogen, -NO₂, -CN, -Rₐ, -ORₐ, -S(O)ₘRₐ, -NRₐRₐ, -C(O)NRₐRₐ, -C(S)NRₐRₐ, -S(O)ₘNRₐRₐ, -NRₐS(O)ₘRₐ, -NRₐC(O)ORₐ, -OC(O)NRₐRₐ, -NRₐC(O)NRₐRₐ, -NRₐC(S)NRₐRₐ, -C(O)ORₐ, -C(S)ORₐ, and -OC(O)ORₐ;
and
halogen is a group selected from -F, -Cl, -Br, and -I.

2. A compound of claim 1 which is (+/-)-5-(2,4-dichlorophenyl)-3,6-diethyl-N-[cis-2-ethyl-2,3-dihydro-1H-inden-1-yl]pyrazin-2-amine, or (+/-)-5-(2,4-dichlorophenyl)-3,6-diethyl-N-[trans-2-ethyl-2,3-dihydro-1H-inden-lyl]pyrazin-2-amine, or a pharmaceutically acceptable salt of either said compound.

3. A compound of Formula V 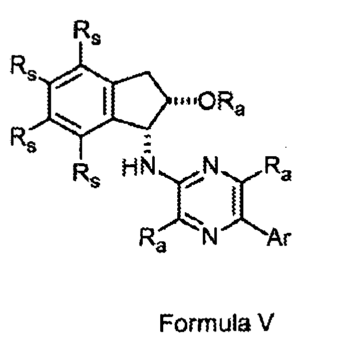 or a stereoisomer thereof, or a pharmaceutically acceptable salt thereof, wherein in Formula V,
Ar is selected from aryl, substituted aryl, heteroaryl, or substituted heteroaryl;
m is 0, 1 or 2;
Rₛ each is independently selected from halogen, -NO₂, -CN, -Rₐ, -ORₐ, -S(O)ₘRₐ, -NRₐRₐ, -C(O)NRₐRₐ, -C(S)NRₐRₐ, -S(O)ₘNRₐRₐ, -NRₐS(O)ₘRₐ, -NRₐC(O)ORₐ, -OC(O)NRₐRₐ, -NRₐC(O)NRₐRₐ, -NRₐC(S)NRₐRₐ, -C(O)ORₐ, -C(S)ORₐ, or -OC(O)ORₐ;
Rₐ each is independently selected from H, alkyl, cycloalkyl, haloalkyl, aryl, heteroaryl, or heterocycloalkyl optionally substituted with 1 to 5 of Rₜ, -ORₜ, -S(O)ₘRₜ, NRₜRₜ, oxo (=O), thione (=S), phenyl, heteroaryl, or heterocycloalkyl where phenyl, heteroaryl, and heterocycloalkyl are optionally substituted with 1 to 5 independently taken from Rₜ; and
Rₜ each is independently selected from H, halogen, -NO₂, -NH₂, -OH, -SH, -CN, -C(O)NH₂, -C(O)-NHalkyl, -C(O)Nalkylalkyl, -Oalkyl, NHalkyl, Nalkylalkyl, -S(O)ₘalkyl, SO₂NH₂, SO₂NHalkyl and SO₂Nalkylalkyl, alkyl, cycloalkyl, haloalkyl, phenyl, benzyl, heteroaryl, or heterocycloalkyl where phenyl, benzyl heteroaryl and heterocycloalkyl may be optionally substituted with alkyl or halogen, wherein "substituted aryl", "heteroaryl", "substituted heteroaryl", "alkyl", "cycloalkyl", "haloalkyl" and "heterocycloalkyl" are as defined in claim 1.

4. A compound of claim 3 which is 5-(2,4-dichlorophenyl)-N-[(1R,2S)-2-ethoxy-2,3-dihydro-1H-inden-1-yl]-3,6-diethylpyrazin-2-amine,
5-(2,4-dichlorophenyl)-3,6-diethyl-N-[(1R,2S)-2-methoxy-2,3-dihydro-1H-inden-1-yl]pyrazin-2-amine,
5-(2,4-dichlorophenyl)-3,6-diethyl-N-[(1R,2S)-2-isopropoxy-2,3-dihydro-1H-inden-1-yl]pyrazin-2-amine,
N-[(1R,2S)-2-(cyclopropylmethoxy)-2,3-dihydro-1H-inden-1-yl]-5-(2,4-dichlorophenyl)-3,6-diethylpyrazin-2-amine,
5-(2-chloro-4-methoxyphenyl)-N-[(1R,2S)-2-ethoxy-2,3-dihydro-1H-inden-1-yl]-3,6-diethylpyrazin-2-amine, or
5-[2-chloro-4-(dimethylamino)phenyl]-N-[(1R,2S)-2-ethoxy-2,3-dihydro-1H-inden-1-yl]-3,6-diethylpyrazin-2-amine, or a pharmaceutically acceptable salt of any said compound.
5-(2,4-dichlorophenyl)-3,6-diethyl-N-[(1R,2S)-2-(prop-2-ynyloxy)-2,3-dihydro-1H-inden-1-yl]pyrazin-2-amine.
N-[(1R,2S)-2-(allyloxy)-2,3-dihydro-1H-inden-1-yl]-5-(2,4-dichlorophenyl)-3,6-diethylpyrazin-2-amine.
N-[(1R,2S)-2-ethoxy-2,3-dihydro-1H-inden-1-yl]-3,6-diethyl-5-(4-methoxy-2-methylphenyl)pyrazin-2-amine, or a pharmaceutically acceptable salt of any said compound.

5. A compound of Formula VI 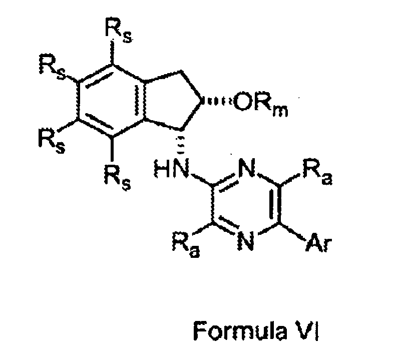 or a stereoisomer thereof, or a pharmaceutically acceptable salt thereof, wherein in Formula VI,
Ar is selected from aryl, substituted aryl, heteroaryl, or substituted heteroaryl;
m is 0,1 or 2;
Rₛ each is independently selected from halogen, -NO₂, -CN, -Rₐ, -ORₐ, -S(O)ₘRₐ, -NRₐRₐ, -C(O)NRₐRₐ, -C(S)NRₐRₐ, -S(O)ₘNRₐRₐ, -NRₐS(O)ₘRₐ, -NRₐC(O)ORₐ, -OC(O)NRₐRₐ, -NRₐC(O)NRₐRₐ, -NRₐC(S)NRₐRₐ, -C(O)ORₐ, -C(S)ORₐ, or -OC(O)ORₐ;
Rₐ each is independently selected from H, alkyl, cycloalkyl, haloalkyl, aryl, heteroaryl, or heterocycloalkyl optionally substituted with 1 to 5 of Rₜ, -ORₜ, -S(O)ₘRₜ, NRₜRₜ, oxo (=O), thione (=S), phenyl, heteroaryl, or heterocycloalkyl where phenyl, heteroaryl, and heterocycloalkyl are optionally substituted with 1 to 5 independently taken from Rₜ;
Rₘ is C₁-C₆ alkyl substituted with from 1-2 of halogen, -NO₂, -NH₂, -OH, -SH, -CN, -C(O)NH₂, -C(O)-NHalkyl, -C(O)Nalkylalkyl, -Oalkyl, NHalkyl, Nalkylalkyl, -S(O)ₘalkyl, SO₂NH₂, SO₂NHalkyl and SO₂Nalkylalkyl, oxo (=O), thione (=S), heterocycloalkyl, or substituted heterocycloalkyl; and
Rₜ each is independently selected from H, halogen, -NO₂, -NH₂, -OH, -SH, -CN, -C(O)NH₂, -C(O)-NHalkyl, -C(O)Nalkylalkyl, -Oalkyl, NHalkyl, Nalkylalkyl, -S(O)ₘalkyl, SO₂NH₂, SO₂NHalkyl and SO₂Nalkylalkyl, alkyl, cycloalkyl, haloalkyl, phenyl, benzyl, heteroaryl, or heterocycloalkyl where phenyl, benzyl heteroaryl and heterocycloalkyl may be optionally substituted with alkyl or halogen, wherein "substituted aryl", "heteroaryl", "substituted heteroaryl", "alkyl", "cycloalkyl", "haloalkyl", "heterocycloalkyl" and "substituted heterocycloalkyl", are as defined in claim 1.

6. A compound of claim 5 which is 5-(2,4-dichlorophenyl)-3,6-diethyl-N-[(1R,2S)-2-(2-fluoroethoxy)-2,3-dihydro-1H-inden-1-yl]pyrazin-2-amine,
2-[((1R,2S)-1-{[5-(2,4-dichlorophenyl)-3,6-diethylpyrazin-2-yl]amino}-2,3-dihydro-1H-inden-2-yl)oxy]ethanol,
(1R,2S)-1-{[5-(2,4-dichlorophenyl)-3,6-diethylpyrazin-2-yl]amino}-2,3-dihydro-1H-inden-2-yl acetate,
5-(2-chloro-4-methoxyphenyl)-3,6-diethyl-N-[(1R,2S)-2-(2-fluoroethoxy)-2,3-dihydro-1H-inden-1-yl]pyrazin-2-amine,
(1R,2S)-1-{[5-(2-chloro-4-methoxyphenyl)-3,6-diethylpyrazin-2-yl]amino}-2,3-dihydro-1H-inden-2-yl acetate,
5-[2-chloro-4-(dimethylamino)phenyl]-3,6-diethyl-N-[(1R,2S)-2-(2-fluoroethoxy)-2,3-dihydro-1H-inden-1-yl]pyrazin-2-amine,
(1R,2S)-1-{[5-(2,4-dichlorophenyl)-3,6-diethylpyrazin-2-yl]amino}-2,3-dihydro-1H-inden-2-yl dimethylcarbamate.
5-(2,4-dimethoxyphenyl)-3,6-diethyl-N-[(1R,2S)-2-(2-fluoroethoxy)-2,3-dihydro-1H-inden-1-yl]pyrazin-2-amine, or a pharmaceutically acceptable salt of any said compound.

7. A pharmaceutical composition comprising a compound as claimed in any one of claims 1 to 6 and a pharmaceutically acceptable carrier or excipient.

8. A method of inhibiting the binding of CRF to the CRF₁ receptor *in vitro,* the method comprising contacting, in the presence of CRF, a solution comprising a compound as claimed in any one of claims 1 to 6 with cells expressing the CRF₁ receptor, wherein the compound is present in the solution at a concentration sufficient to reduce levels of CRF binding to the cells *in vitro.*

9. An article of manufacture comprising: a) a packaging material; b) a compound as claimed in any of claims 1 to 6 and c) a label or package insert contained within said packaging material indicating that said compound is effective for treating affective disorder, anxiety or depression.

10. A compound as claimed in any one of claims 1 to 6 for use as an active pharmaceutical substance.

11. Use of a compound as claimed in any one of claims 1 to 6 for the preparation of a pharmaceutical composition.

12. Use of a compound as claimed in any one of claims 1 to 6 for the preparation of a medicament for treating a disorder manifesting hypersecretion of CRF in a mammal.

13. Use of a compound as claimed in any one of claims 1 to 6 for the preparation of a medicament for treating a disorder the treatment of which can be effected or facilitated by antagonizing CRF in a mammal.

14. Use of a compound as claimed in any one of claims 1 to 6 for the preparation of a medicament for treating a disorder in a mammal, wherein the disorder is selected from social anxiety disorder; panic disorder; obsessive-compulsive disorder; anxiety with co-morbid depressive illness; affective disorder; anxiety; depression; irritable bowel syndrome; post-traumatic stress disorder; supranuclear palsy; immune suppression; anorexia nervosa or other feeding disorder; drug or alcohol withdrawal symptoms; substance abuse disorder; fertility problems; inflammatory disorders; pain; asthma; psoriasis; allergies; generalized anxiety disorder; panic, phobias; sleep disorders induced by stress; fibromyalgia; mood disorders; dysthemia; bipolar disorders; cyclothymia; fatigue syndrome; stress-induced headache; cancer; human immunodeficiency virus infections; neurodegenerative diseases; gastrointestinal diseases; eating disorders; hemorrhagic stress; stress-induced psychotic episodes; euthyroid sick syndrome; syndrome of inappropriate antidiarrhetic hormone; obesity; infertility; head traumas; spinal cord trauma; ischemic neuronal damage; excitotoxic neuronal damage; epilepsy; cardiovascular and heart related disorders; stroke; immune dysfunctions; muscular spasms; urinary incontinence; senile dementia of the Alzheimer's type; multiinfarct dementia; amyotrophic lateral sclerosis; chemical dependencies and addictions; osteoporosis; psychosocial dwarfism; and hypoglycemia.

15. A compound as claimed in any one of claims 1 to 6 for use in the treatment of social anxiety disorder; panic disorder; obsessive-compulsive disorder; anxiety with co-morbid depressive illness; affective disorder; anxiety; depression; irritable bowel syndrome; post-traumatic stress disorder; supranuclear palsy; immune suppression; anorexia nervosa or other feeding disorder; drug or alcohol withdrawal symptoms; substance abuse disorder; inflammatory disorders; fertility problems; pain; asthma; psoriasis; allergies; generalized anxiety disorder; panic, phobias; sleep disorders induced by stress; fibromyalgia; mood disorders; dysthemia; bipolar disorders; cyclothymia; fatigue syndrome; stress-induced headache; cancer; human immunodeficiency virus infections; neurodegenerative diseases; gastrointestinal diseases; eating disorders; hemorrhagic stress; stress-induced psychotic episodes; euthyroid sick syndrome; syndrome of inappropriate antidiarrhetic hormone; obesity; infertility; head traumas; spinal cord trauma; ischemic neuronal damage; excitotoxic neuronal damage; epilepsy; cardiovascular and heart related disorders; stroke; immune dysfunctions; muscular spasms; urinary incontinence; senile dementia of the Alzheimer's type; multiinfarct dementia; amyotrophic lateral sclerosis; chemical dependencies and addictions; osteoporosis; psychosocial dwarfism; and hypoglycemia.

## Patentansprüche

1. Verbindung der Formel IV 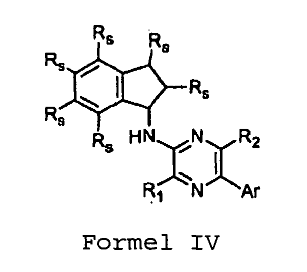 oder ein Stereoisomer davon oder ein pharmazeutisch verträgliches Salz davon,
wobei in Formel IV
Ar ausgewählt ist aus Aryl, substituiertem Aryl, Heteroaryl und substituiertem Heteroaryl;
m 0, 1 oder 2 ist;
R₁ und R₂ unabhängig ausgewählt sind aus Halogen, -NO₂, -CN, -Rₐ, -ORₐ, S(O)ₘRₐ, -NRₐRₐ, C(O)NRₐRₐ,-C(S)NRₐRₐ, -S(O)ₘNRₐRₐ, -NRₐS(O)ₘRₐ, -NRₐC(O)ORₐ, -OC(O)NRₐRₐ, -NRₐC(O) NRₐRₐ, -NRₐC(S)NRₐRₐ, -C(O)ORₐ, C(S)ORₐ und -OC(O)OR_{a;}
Rₛ jeweils unabhängig ausgewählt ist aus Halogen, -NO₂, -CN, -Rₐ, -ORₐ, S(O)ₘRₐ, -NRₐRₐ, -C(O)NRₐRₐ, -C(S)NRₐRₐ, S(O)ₘNRₐRₐ, -NRₐS (O) ₘRₐ, -NRₐC (O) ORₐ, -OC (O) NRₐRₐ, -NRₐC (O) NRₐRₐ, -NRₐC(S)NRₐRₐ, -C(O)ORₐ, C(S)ORₐ und -OC(O)ORₐ;
Rₐ jeweils unabhängig ausgewählt ist aus H, Alkyl, Cycloalkyl, Halogenalkyl, Aryl, Heteroaryl und Heterocycloalkyl, gegebenenfalls substituiert mit 1 bis 5 von Rₜ, -ORₜ, -S(O)ₘRₜ, NRₜRₜ, Oxo (=O), Thion (=S), Phenyl, Heteroaryl und Heterocycloalkyl, wobei Phenyl, Heteroaryl und Heterocycloalkyl gegebenenfalls substituiert sind mit 1 bis 5, unabhängig genommen aus Rₜ; und
Rₜ jeweils unabhängig ausgewählt ist aus H, Halogen, -NO₂, -NH₂, -OH, -SH, -CN, -C(O)NH₂, -C(O)-NHAlkyl, -C(O)NAlkylalkyl, -OAlkyl , NHAlkyl, NAlkylalkyl, -S(O)ₘAlkyl, SO₂NH₂, SO₂NHAlkyl und SO₂NAlkylalkyl, Alkyl, Cycloalkyl, Halogenalkyl, Phenyl, Benzyl, Heteroaryl und Heterocycloalkyl, wobei Phenyl, Benzyl, Heteroaryl und Heterocycloalkyl gegebenenfalls substituiert sein können mit Alkyl oder Halogen, wobei
"substituiertes Aryl" eine Arylgruppe bedeutet, gegebenenfalls substituiert mit 1-5 Substituenten, unabhängig ausgewählt aus Halogen, -NO₂, -CN, -Rₐ, -ORₐ, S(O)ₘRₐ, -NRₐRₐ, -C(O)NRₐR_{a,} -C(S)NRₐRₐ, -S(O)ₘNRₐRₐ, -NRₐS(O)ₘRₐ, -NRₐC(O)ORₐ, -OC(O)NRₐRₐ, -NRₐC(O)NRₐRₐ, -NRₐC(S)NRₐRₐ, -C(O)ORₐ, -C(S)ORₐ und -OC(O)ORₐ;
"Heteroaryl" einen Rest, gebunden über ein Ringkohlenstoff- oder -stickstoffatom eines monocyclischen aromatischen Rings, enthaltend fünf oder sechs Ringatome, bestehend aus Kohlenstoff und 1, 2, 3 oder 4 Heteroatomen, jeweils ausgewählt aus der Gruppe, bestehend aus Nicht-Peroxid-O, S, N, mit geeigneter Bindung, um die Valenzanforderungen zu erfüllen, so wie einen Rest (Bindung entweder an Kohlenstoff oder Stickstoff) eines kondensierten bicyclischen Heteroaromaten von etwa acht bis zehn Ringatomen bedeutet;
"substituiertes Heteroaryl" eine Heteroarylgruppe bedeutet mit 1-5 Substituenten, unabhängig ausgewählt aus Halogen, -NO₂, -CN, -Rₐ, -ORₐ , -S(O)ₘRₐ, -NRₐRₐ, -C(O)NRₐRₐ, C(S)NRₐRₐ, -S(O)ₘNRₐRₐ, -NRₐS(O)ₘRₐ, -NRₐC(O)ORₐ, -OC(O)NRₐR_{a,} -NRₐC(O)NRₐRₐ, -NRₐC(S)NRₐRₐ, -C(O)ORₐ, -C(S)ORₐ und -OC(O)ORₐ; "Alkyl" sowohl gerad- als auch verzweigtkettige Gruppierungen mit 1-10 Kohlenstoffatomen, gegebenenfalls eine oder mehrere Doppel- oder Dreifachbindung(en) enthaltend, bedeutet; "Cycloalkyl" eine monocyclische oder bicyclische Alkylgruppierung mit 3-10 Kohlenstoffatomen, gegebenenfalls 1 bis 2 Doppelbindungen enthaltend, bedeutet, mit der Maßgabe, dass die Gruppierung nicht aromatisch ist;
"Halogenalkyl" eine Alkylgruppierung mit 1-10 Kohlenstoffatomen und mit 1 bis (2v+1) unabhängig ausgewähltem/n Halogensubstituent(en) bedeutet, wobei v die Anzahl an Kohlenstoffatomen in der Gruppierung ist; "Heterocycloalkyl", soweit nichts anderes angegeben ist, einen 4- bis 8-gliedrigen monocyclischen Ring oder bicyclischen Ring bedeutet, wobei mindestens ein Kohlenstoffatom durch ein,Heteroglied ersetzt ist, ausgewählt aus Sauerstoff, Stickstoff, -NH- und -S(O)ₘ-, wobei m Null, 1 oder 2 ist, gegebenenfalls eine bis drei Doppelbindung(en) enthaltend, mit der Maßgabe, dass das Molekül nicht aromatisch ist, und mit der Maßgabe, dass die Ringbindung entweder an einem Kohlenstoff- oder einem Stickstoffatom erfolgen kann;
"substituiertes Heterocycloalkyl" eine Hetercycloalkylgruppe ist mit 1-5 Substituenten, unabhängig ausgewählt aus Halogen, -NO₂, -CN, -Rₐ, -ORₐ, -S(O)ₘRₐ, -NRₐRₐ, -C(O)NRₐRₐ, C(S)NRₐRₐ, -S(O)ₘNRₐRₐ, -NRₐS (O)ₘRₐ, -NRₐC(O) ORₐ, -OC(O) NRₐRₐ, -NRₐC(O)NRₐRₐ, -NRₐC(S)NRₐRₐ, -C(O)ORₐ, -C(S)ORₐ und -OC(O)ORₐ; und
Halogen eine Gruppe ist, ausgewählt aus -F, -Cl, -Br und -I.

2. Verbindung nach Anspruch 1, welche (+/-)-5-(2,4-Dichlorphenyl)-3,6-diethyl-N-[cis-2-ethyl-2,3-dihydro-1H-inden-1-yl]pyrazin-2-amin oder (+/-)-5-(2,4-Dichlorphenyl)-3,6-diethyl-N-[trans-2-ethyl-2,3-dihydro-1H-inden-1-yl]pyrazin-2-amin oder ein pharmazeutisch verträgliches Salz einer dieser Verbindungen ist.

3. Verbindung der Formel V 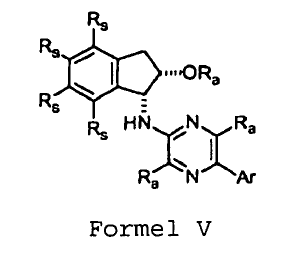 oder ein Stereoisomer davon oder ein pharmazeutisch verträgliches Salz davon,
wobei in Formel IV
Ar ausgewählt ist aus Aryl, substituiertem Aryl, Heteroaryl und substituiertem Heteroaryl;
m 0, 1 oder 2 ist;
Rₛ jeweils unabhängig ausgewählt ist aus Halogen, -NO₂, -CN, -Rₐ, -ORₐ, S(O)ₘRₐ, -NRₐRₐ, -C(O)NRₐRₐ, -C(S)NRₐRₐ, -S(O)ₘNRₐRₐ, -NRₐS(O)ₘRₐ, -NRₐC(O)ORₐ, -OC(O)NRₐRₐ, -NRₐC(O) NRₐRₐ, -NRₐC(S)NRₐRₐ, -C(O)ORₐ, -C(S)ORₐ und -OC(O)ORₐ;
Rₐ jeweils unabhängig ausgewählt ist aus H, Alkyl, Cycloalkyl, Halogenalkyl, Aryl, Heteroaryl und Heterocycloalkyl, gegebenenfalls substituiert mit 1 bis 5 von Rₜ, -ORₜ, -S(O)ₘRₜ, NRₜRₜ, Oxo (=O) Thion (=S), Phenyl, Heteroaryl und Heterocycloalkyl, wobei Phenyl, Heteroaryl und Heterocycloalkyl gegebenenfalls substituiert sind mit 1 bis 5, unabhängig genommen aus Rₜ; und
Rₜ jeweils unabhängig ausgewählt ist aus H, Halogen, -NO₂, -NH₂, -OH, -SH, -CN, -C(O)NH₂, -C(O)-NHAlkyl, -C(O)NAlkylalkyl, -OAlkyl, NHAlkyl, NAlkylalkyl, -S(O)ₘAlkyl, SO₂NH₂, SO₂NHAlkyl und SO₂NAlkylalkyl, Alkyl, Cycloalkyl, Halogenalkyl Phenyl, Benzyl, Heteroaryl und Heterocycloalkyl, wobei Phenyl, Benzyl, Heteroaryl und Heterocycloalkyl gegebenenfalls substituiert sein können mit Alkyl oder Halogen, wobei
"substituiertes Aryl", "Heteroaryl", "substituiertes Heteroaryl", "Alkyl", "Cycloalkyl", "Halogenalkyl" und "Heterocycloalkyl" wie in Anspruch 1 definiert sind.

4. Verbindung nach Anspruch 3, welche
5-(2,4-Dichlorphenyl)-N-[(1R;2S)-2-ethoxy-2,3-dihydro-1H-inden-1-yl]-3,6-diethylpyrazin-2-amin,
-5-(2,4-Dichlorphenyl)-3,6-diethyl-N-[(1R*,*2S)-2-methoxy-2,3-dihydro-1H-inden-1-yl]pyrazin-2-amin,
5-(2,4-Dichlorphenyl)-3,6-diethyl-N-[(1R,2S)-2-isopropoxy-2,3-dihydro-1H-inden-1-yl]pyrazin-2-amin,
N-[(1R,2S)-2-(Cyclopropylmethoxy)-2,3-dihydro-1H-inden-1-yl]-5-(2,4-dichlorphenyl)-3,6-diethylpyrazin-2-amin,
5-(2-Chlor-4-methoxyphenyl)-N-[(1R,2S)-2-ethoxy-2,3-dihydro-1H-inden-1-yl]-3,6-diethylpyrazin-2-amin oder
5-[2-Chlor-4-(dimethylamino)phenyl]-N-[(1R,2S)-2-ethoxy-2,3-dihydro-1H-inden-1-yl]-3,6-diethylpyrazin-2-amin
oder ein pharmazeutisch verträgliches Salz einer dieser Verbindungen,
5-(2,4-Dichlorphenyl)-3,6-diethyl-N-[(1R,2S)-2-(prop-2-inyloxy)-2,3-dihydro-1H-inden-1-yl]pyrazin-2-amin,
N-[(1R,2S)-2-(Allyloxy)-2,3-dihydro-1H-inden-1-yl]-5-(2,4-dichlorphenyl)-3,6-diethylpyrazin-2-amin,
N-[(1R,2S)-2-Ethoxy-2,3-dihydro-1H-inden-1-yl]-3,6-diethyl-5-(4-methoxy-2-methylphenyl)pyrazin-2-amin
oder ein pharmazeutisch verträgliches Salz einer dieser Verbindungen ist.

5. Verbindung der Formel VI 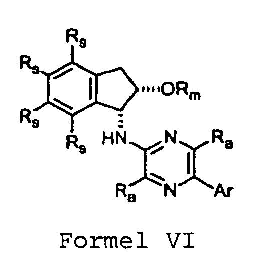 oder ein Stereoisomer davon oder ein pharmazeutisch verträgliches Salz davon,
wobei in Formel VI
Ar ausgewählt ist aus Aryl, substituiertem Aryl, Heteroaryl und substituiertem Heteroaryl;
m 0, 1 oder 2 ist;
Rₛ jeweils unabhängig ausgewählt ist aus Halogen, -NO₂, -CN, -Rₐ, -ORₐ, -S(O)ₘR_{a,} -NRₐRₐ , - C(O)NRₐRₐ, -C(S)NRₐRₐ, -S(O)ₘNRₐRₐ, -NRₐS(O)ₘRₐ, -NRₐC(O)ORₐ, -OC(O)NRₐRₐ, -NRₐC(O)NRₐRₐ, -NRₐC(S)NRₐRₐ, -C(O)ORₐ, -C(S)ORₐ und -OC(O)ORₐ;
Rₐ jeweils unabhängig ausgewählt ist aus H, Alkyl, Cycloalkyl, Halogenalkyl, Aryl, Heteroaryl und Heterocycloalkyl, gegebenenfalls substituiert mit 1 bis 5 von Rt, -ORₜ, -S(O)ₘRₜ, NRₜRₜ, Oxo (=O), Thion (=S), Phenyl, Heteroaryl und Heterocycloalkyl, wobei Phenyl, Heteroaryl und Heterocycloalkyl gegebenenfalls substituiert sind mit 1 bis 5, unabhängig genommen aus Rₜ;
Rₘ C₁-C₆-Alkyl ist, substituiert mit 1-2 Halogen, -NO₂, -NH₂, -OH, -SH, -CN, -C(O)NH₂, -C(O)-NHAlkyl, -C(O)NAlkylalkyl, -OAlkyl , NHAlkyl, NAlkylalkyl , - -S(O)ₘAlkyl, SO₂NH₂, SO₂NHAlkyl und SO₂NAlkylalkyl, Oxo (=O), Thion (=S), Heterocycloalkyl oder substituierten Heterocycloalkyl; und
Rₜ jeweils unabhängig ausgewählt ist aus H, Halogen, -NO₂, -NH₂, -OH, -SH, -CN, -C (O) NH₂, -C(O)-NHAlkyl, -C (O) NAlkylalkyl, -OAlkyl, NHAlkyl, NAlkylalkyl, -S(O)ₘAlkyl, SO₂NH₂, SO₂NHAlkyl und SO₂NAlkylalkyl, Alkyl, Cycloalkyl, Halogenalkyl, Phenyl, Benzyl, Heteroaryl und Heterocycloalkyl, wobei Phenyl, Benzyl, Heteroaryl und Heterocycloalkyl gegebenenfalls substituiert sein können mit Alkyl oder Halogen, wobei
"substituiertes Aryl", "Heteroaryl", "substituiertes Heteroaryl", "Alkyl", "Cycloalkyl", "Halogenalkyl", "Heterocycloalkyl" und "substituiertes Heterocycloalkyl" wie in Anspruch 1 definiert sind.

6. Verbindung nach Anspruch 5, welche
5-(2,4-Dichlorphenyl)-3,6-diethyl-N-[(1R,2S)-2-(2-fluorethoxy)-2,3-dihydro-1H-inden-1-yl]pyrazin-2-amin,
2-[((1R,2S)-1-{[5-(2,4-Dichlorphenyl)-3,6-diethylpyrazin-2-yl]amino}-2,3-dihydro-1H-inden-2-yl)oxy]ethanol,
(1R,2S)-1-{[5-(2,4-Dichlorphenyl)-3,6-diethylpyrazin-2-yl]amino}-2,3-dihydro-1H-inden-2-ylacetat,
5-(2-Chlor-4-methoxyphenyl)-3,6-diethyl-N-[(1R,2S)-2-(2-fluorethoxy)-2,3-dihydro-1H-inden-1-yl]pyrazin-2-amin,
(1R,2S)-1-{[5-(2-Chlor-4-methoxyphenyl)-3,6-diethylpyrazin-2-yl]amino}-2,3-dihydro-1H-inden-2-ylacetat,
5-[2-Chlor-4-(dimethylamino)phenyl]-3,6-diethyl-N-[(1R,2S)-2-(2-fluorethoxy)-2,3-dihydro-1H-inden-1-yl]pyrazin-2-amin,
(1R,2S)-1-{[5-(2,4-Dichlorphenyl)-3,6-diethylpyrazin-2-yl]amino}-2,3-dihydro-1H-inden-2-yldimethylcarbamat,
5-(2,4-Dimethoxyphenyl)-3,6-diethyl-N-[(1R,2S)-2-(2-fluorethoxy)-2,3-dihydro-1H-inden-1-yl]pyrazin-2-amin,
oder ein pharmazeutisch verträgliches Salz einer dieser Verbindungen ist.

7. Pharmazeutische Zusammensetzung, umfassend eine Verbindung, wie in einem der Ansprüche 1 bis 6 beansprucht, und ein/en pharmazeutisch verträglichen/s Träger oder Exzipiens.

8. Verfahren zum Inhibieren des Bindens von CRF an den CRF₁-Rezeptor in vitro, wobei das Verfahren das Inkontaktbringen einer Lösung, umfassend eine Verbindung, wie in einem der Ansprüche 1 bis 6 beansprucht, in Gegenwart von CRF mit Zellen, die den CRF₁-Rezeptor exprimieren, umfasst, wobei die Verbindung in der Lösung in einer Konzentration vorhanden ist, die ausreichend ist, um die Level der CRF-Bindung an die Zellen in vitro zu verringern.

9. Gegenstand, umfassend: a) ein Verpackungsmaterial; b) eine Verbindung, wie in einem der Ansprüche 1 bis 6 beansprucht; und c) eine Etikettierung oder Packungsbeilage, enthalten innerhalb des Verpackungsmaterials, die angibt, dass die Verbindung beim Behandeln einer Affektstörung, von Angst oder Depression wirksam ist.

10. Verbindung, wie in einem der Ansprüche 1 bis 6 beansprucht, zur Verwendung als eine aktive pharmazeutische Substanz.

11. Verwendung einer Verbindung, wie in Anspruch 6 beansprucht, zur Herstellung einer pharmazeutischen Zusammensetzung.

12. Verwendung einer Verbindung, wie in einem der Ansprüche 1 bis 6 beansprucht, zur Herstellung eines Medikaments zum Behandeln einer Störung, die sich in einer Hypersekretion von CRF bei einem Säuger manifestiert.

13. Verwendung einer Verbindung, wie in einem der Ansprüche 1 bis 6 beansprucht, zur Herstellung eines Medikaments zum Behandeln einer Störung, wobei deren Behandlung durch Antagonisieren von CRF bei einem Säuger bewirkt oder erleichtert werden kann.

14. Verwendung einer Verbindung, wie in einem der Ansprüche 1 bis 6 beansprucht, zur Herstellung eines Medikaments zum Behandeln einer Störung bei einem Säuger, wobei die Störung ausgewählt ist aus sozialer Angststörung, Panikstörung, obsessiv-kompulsiver Störung, Angst mit komorbider depressiver Erkrankung, Affektstörung, Angst, Depression, Reizdarmsyndrom, posttraumatischer Stressstörung, supranukleärer Lähmung, Immunsuppression, Anorexia nervosa oder anderer Ernährungsstörung, Drogen- oder Alkoholentzugssymptomen, Substanzmissbrauchsstörung, Entzündungsstörung, Fertilitätsproblemen, Schmerz, Asthma, Psoriasis, Allergien, generalisierter Angststörung, Panik, Phobien, Schlafstörungen, die durch Stress induziert sind, Fibromyalgie, Gemütsstörungen, Dysthemie, bipolaren Störungen, Zyklothymie, Erschöpfungssyndrom, Stressinduziertem Kopfschmerz, Krebs, humanes Immundefizienz-VirusInfektionen, neurodegenerativen Erkrankungen, gastrointestinalen Erkrankungen, Essstörungen, hämorrhagischem Stress, Stress-induzierten psychotischen Episoden, Niedrig-T₃(T₄)-Syndrom ("euthyroid sick syndrome"), Syndrom der inadäquaten ADH-Sekretion ("syndrome of inappropriate antidiaarhetic hormone"), Adipositas, Unfruchtbarkeit, Schädeltraumata, Rückenmarkstrauma, ischämischer neuronaler Schädigung, exzitotoxischer neuronaler Schädigung, Epilepsie, kardiovaskulären Störungen und Störungen mit Bezug zum Herzen, Schlaganfall, Immundysfunktionen, Muskelspasmen, Harninkontinenz, seniler Demenz vom Alzheimer-Typ, Multiinfarktdemenz, amyotropher Lateralsklerose, Abhängigkeiten von und Suchten nach Chemikalien, Osteoporose, psychosozialem Kleinwuchs und Hypoglykämie.

15. Verbindung, wie in einem der Ansprüche 1 bis 6 beansprucht, zur Verwendung bei der Behandlung von sozialer Angststörung, Panikstörung, obsessiv-kompulsiver Störung, Angst mit komorbider depressiver Erkrankung, Affektstörung, Angst, Depression, Reizdarmsyndrom, posttraumatischer Stressstörung, supranukleärer Lähmung, Immunsuppression, Anorexia nervosa oder anderer Ernährungsstörung, Drogen- oder Alkoholentzugssymptomen, Substanzmissbrauchsstörung, Entzündungsstörungen, Fertilitätsproblemen, Schmerz, Asthma, Psoriasis, Allergien, generalisierter Angststörung, Panik, Phobien, Schlafstörungen, die durch Stress induziert sind, Fibromyalgie, Gemütsstörungen, Dysthemie, bipolaren Störungen, Zyklothymie, Erschöpfungssyndrom, Stressinduziertem Kopfschmerz, Krebs, humanes Immundefizienz-VirusInfektionen, neurodegenerativen Erkrankungen, gastrointestinalen Erkrankungen, Essstörungen, hämorrhagischem Stress, Stress-induzierten psychotischen Episoden, Niedrig-T₃(T₄)-Syndrom, Syndrom der inadäquaten ADH-Sekretion, Adipositas, Unfruchtbarkeit, Schädeltraumata, Rückenmarkstrauma, ischämischer neuronaler Schädigung, exzitotoxischer neuronaler Schädigung, Epilepsie, kardiovaskulären Störungen und Störungen mit Bezug zum Herzen, Schlaganfall, Immundysfunktionen, Muskelspasmen, Harninkontinenz, seniler Demenz vom Alzheimer-Typ, Multiinfarktdemenz, amyotropher Lateralsklerose, Abhängigkeiten von und Suchten nach Chemikalien, Osteoporose, psychosozialem Kleinwuchs und Hypoglykämie.

## Revendications

1. Composé de Formule IV 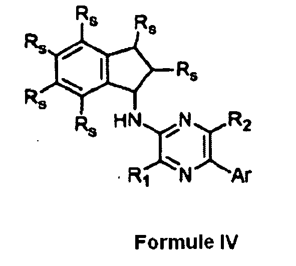 ou stéréoisomère de celui-ci, ou sel pharmaceutiquement acceptable correspondant, dans lequel, dans la Formule IV :
Ar est sélectionné parmi aryle, aryle substitué, hétéroaryle et hétéroaryle substitué ;
m représente 0, 1 ou 2 ;
R₁ et R₂ sont indépendamment sélectionnés parmi halogéno, -NO₂, -CN, -Rₐ, -ORₐ, -S(O)ₘRₐ, -NRₐRₐ, -C(O)NRₐRₐ, -C(S)NRₐRₐ, -S(O)ₘNRₐRₐ, -NRₐS(O)ₘRₐ, -NRₐC(O)ORₐ, -OC(O)NRₐRₐ, -NRₐC(O)NRₐRₐ, -NRₐC(S)NRₐRₐ, -C(O)ORₐ, -C(S)ORₐ et -OC(O)ORₐ ;
chaque Rₛ est indépendamment sélectionné parmi halogéno, -NO₂, -CN, -Rₐ, -ORₐ, -S(O)ₘRₐ, -NRₐRₐ, -C(O)NRₐRₐ, -C(S)NRₐRₐ, -S(O)ₘNRₐRₐ, -NRₐS(O)ₘRₐ, - NRₐC(O)ORₐ , - OC(O)NRₐRₐ, -NRₐC(O)NRₐRₐ, -NRₐC(S)NRₐRₐ, -C(O)ORₐ, -C(S)ORₐ et -OC(O)ORₐ ;
chaque Rₐ étant indépendamment sélectionné parmi H, alkyle, cycloalkyle, halogénoalkyle, aryle, hétéroaryle et hétérocycloalkyle facultativement substitués par de 1 à 5 groupe (s) choisis parmi Rₜ, -ORₜ, -S (O)ₘRₜ, NRₜRₜ, oxo (=O), thione (=S), phényle, hétéroaryle et hétérocycloalkyle, les groupes phényle, hétéroaryle et hétérocycloalkyle étant facultativement substitués par de 1 à 5 groupe(s) indépendamment choisi(s) parmi Rₜ ;
chaque Rₜ étant indépendamment sélectionné parmi H, halogéno, -NO₂, -NH₂, -OH, -SH, -CN, -C(O)NH₂, -C(O)-NH-alkyle, -C(O)N(alkyl)alkyle, -O-alkyle, NH-alkyle, N (alkyl) alkyle, -S(O)ₘalkyle, SO₂NH₂, SO₂NH-alkyle, SO₂N(alkyl)alkyle, alkyle, cycloalkyle, halogénoalkyle, phényle, benzyle, hétéroaryle et hétérocycloalkyle, les groupes phényle, benzyle, hétéroaryle et hétérocycloalkyle pouvant être facultativement substitués par alkyle ou halogéno,
"aryle substitué" désignant un groupe aryle facultativement substitué par de 1 à 5 substituants indépendamment sélectionnés parmi halogéno, -NO₂, -CN, -Rₐ, -ORₐ, -S(O)ₘRₐ, - NRₐRₐ, -C(O)NRₐR_{a,} -C(S)NRₐRₐ, -S(O)ₘNRₐRₐ, -NRₐS(O)ₘRₐ, -NRₐC(O)ORₐ, -OC(O)NRₐRₐ, -NRₐC(O)NRₐRₐ, -NRₐC(S)NRₐRₐ, -C(O)ORₐ, -C(S)ORₐ et -OC(O)ORₐ;
"hétéroaryle" désignant un radical fixé via un atome de carbone ou d'azote de noyau d'un noyau aromatique monocyclique contenant cinq ou six atomes de noyau consistant en du carbone et 1, 2, 3 ou 4 hétéroatomes, dont chacun est sélectionné dans le groupe consistant en O, S, N non peroxydique, avec une liaison appropriée pour satisfaire aux exigences de valence, ainsi qu'un radical (fixation sur un carbone ou un azote) d'un composé hétéroaromatique bicyclique condensé ayant d'environ huit à dix atomes de noyau,
"hétéroaryle substitué" désignant un groupe hétéroaryle ayant de 1 à 5 substituants indépendamment sélectionnés parmi halogéno, -NO₂, -CN, -Rₐ, -ORₐ, -S (O) ₘRₐ, -NRₐRₐ, -C(O)NRₐRₐ, - C(S)NRₐRₐ , -S(O)ₘNRₐRₐ, -NRₐS(O)ₘRₐ, -NRₐC(O)ORₐ, -OC(O)NRₐRₐ, -NRₐC(O)NRₐRₐ, -NRₐC(S)NRₐRₐ, -C(O)ORₐ, -C(S)ORₐ et -OC(O)ORₐ ;
"alkyle" désignant à la fois des groupes fonctionnels à chaîne droite ou ramifiée ayant de 1 à 10 atomes de carbone contenant facultativement une ou plusieurs double ou triple-liaisons ;
"cycloalkyle" désignant un groupe fonctionnel alkyle monocyclique ou bicyclique, ayant de 3 à 10 atomes de carbone contenant facultativement 1 ou 2 double-liaisons, étant entendu que le groupe fonctionnel n'est pas aromatique ;
"halogénoalkyle" désignant un groupe fonctionnel alkyle ayant de 1 à 10 atomes de carbone et ayant de 1 à (2v+1) substituant(s) halogéno indépendamment sélectionnés, v étant le nombre d'atomes de carbone dans le groupe fonctionnel ;
"hétérocycloalkyle", sauf indication contraire, désignant un noyau monocyclique ou un noyau bicyclique ayant de 4 à 8 éléments, dans lequel au moins un atome de carbone est remplacé par un hétéroélément sélectionné parmi l'oxygène, l'azote, -NH- et -*S*(O)ₘ-, m représentant zéro, 1 ou 2, le noyau contenant facultativement de une à trois double-liaisons, étant entendu que la molécule n'est pas aromatique ; et étant entendu que la fixation du noyau peut se faire sur un atome de carbone ou d'azote ;
"hétérocycloalkyle substitué" désignant un groupe hétérocycloalkyle ayant de 1 à 5 substituants indépendamment sélectionnés parmi halogéno, -NO₂, -CN, -Rₐ, - ORₐ, - S(O)ₘRₐ, - NRₐRₐ , -C(O)NRₐRₐ, -C(S)NRₐRₐ, -S(O)ₘNRₐRₐ, -NRₐS(O)ₘRₐ, -NRₐC(O)ORₐ, -OC(O)NRₐRₐ, -NRₐC(O)NRₐRₐ, -NRₐC(S)NRₐRₐ, -C(O)ORₐ, -C(S)ORₐ et -OC(O)ORₐ ; et
halogéno étant un groupe sélectionné parmi -F, -Cl, -Br et -I.

2. Composé selon la revendication 1, qui est la (+/-)-5-(2,4-dichlorophényl)-3,6-diéthyl*-N*-[*cis*-2-éthyl-2,3-dihydro-1*H*-indén-1-yl]pyrazin-2-amine ou la (+/-)-5-(2,4-dichlorophényl)-3,6-diéthyl-*N*-[*trans*-2-éthyl-2,3-dihydro-1*H-*indén-1-yl]pyrazin-2-amine, ou sel pharmaceutiquement acceptable de l'un ou l'autre desdits composés.

3. Composé de Formule V 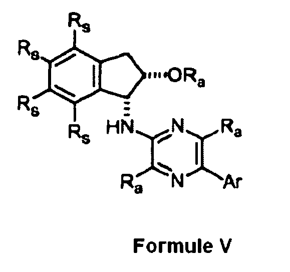 ou stéréoisomère de celui-ci, ou sel pharmaceutiquement acceptable correspondant, dans lequel, dans la Formule V,
Ar est sélectionné parmi aryle, aryle substitué, hétéroaryle et hétéroaryle substitué ;
m représente 0, 1 ou 2 ;
chaque Rₛ est indépendamment sélectionné parmi halogéno, -NO₂, -CN, -Rₐ, -ORₐ, -S(O)ₘRₐ, -NRₐRₐ, -C(O)NRₐRₐ, -C(S)NRₐRₐ, -S(O)ₘNRₐRₐ, -NRₐS (O) ₘRₐ, -NRₐC(O)ORₐ, -OC(O)NRₐRₐ, -NRₐC(O)NRₐRₐ, -NRₐC(S)NRₐRₐ, -C(O)ORₐ, -C(S)ORₐ et -OC(O)ORₐ ;
chaque Rₐ étant indépendamment sélectionné parmi H, alkyle, cycloalkyle, halogénoalkyle, aryle, hétéroaryle et hétérocycloalkyle facultativement substitués par de 1 à 5 groupe(s) choisis parmi Rₜ, -ORₜ, -S(O)ₘRₜ, NRₜRₜ, oxo (=O), thione (=S), phényle, hétéroaryle et hétérocycloalkyle, les groupes phényle, hétéroaryle et hétérocycloalkyle étant facultativement substitués par de 1 à 5 groupe(s) indépendamment choisi(s) parmi Rₜ ;
chaque Rₜ étant indépendamment sélectionné parmi H, halogéno, -NO₂, -NH₂, -OH, -SH, -CN, -C(O)NH₂, -C(O)-NH-alkyle, -C(O)N(alkyl)alkyle, -O-alkyle, NH-alkyle, N (alkyl) alkyle, -S(O)ₘalkyle, SO₂NH₂, SO₂NH-alkyle, SO₂N(alkyl)alkyle, alkyle, cycloalkyle, halogénoalkyle, phényle, benzyle, hétéroaryle et hétérocycloalkyle, les groupes phényle, benzyle, hétéroaryle et hétérocycloalkyle pouvant être facultativement substitués par alkyle ou halogéno,
"aryle substitué", "hétéroaryle", "hétéroaryle substitué", "alkyle", "cycloalkyle", "halogénoalkyle" et "hétérocycloalkyle" étant tels que définis dans la revendication 1.

4. Composé selon la revendication 3, qui est :
• la 5-(2,4-dichlorophényl)*-N*-[(1*R*,2*S*)-2-éthoxy-2,3-dihydro-1*H-*indén-1-yl]-3,6-diéthylpyrazin-2-amine,
• la 5-(2,4-dichlorophényl)-3,6-diéthyl*-N*-[(1*R*,2*S*)-2-méthoxy-2,3-dihydro-1*H-*indén-1-yl]pyrazin-2-amine,
• la 5-(2,4-dichlorophényl)-3,6-diéthyl*-N*-[(1*R*,2*S*)-2-isopropoxy-2,3-dihydro-1*H-*indén-1-yl]pyrazin-2-amine,
• la *N*-[(1*R*,2*S*)-2-(cyclopropylméthoxy)-2,3-dihydro-1*H-*indén-1-yl]-5-(2,4-dichlorophényl)-3,6-diéthylpyrazin-2-amine,
• la 5-(2-chloro-4-méthoxyphényl)-*N*-[(1*R*,2*S*)-2-éthoxy-2,3-dihydro-1*H-*indén-1-yl]-3,6-diéthylpyrazin-2-amine,
• la 5-[2-chloro-4-(diméthylamino)phényl]-*N*-[(1*R*,2*S*)-2-éthoxy-2,3-dihydro-1*H-*indén-1-yl]-3,6-diéthylpyrazin-2-amine,
• la 5-(2,4-dichlorophényl)-3,6-diéthyl-*N*-[(1*R*,2*S*)-2-(prop-2-ynyloxy)-2,3-dihydro-1*H*-indén-1-yl]pyrazin-2-amine,
• la *N*-[(1*R*,2*S*)-2-(allyloxy)-2,3-dihydro-1*H*-indén-1-yl]-5-(2,4-dichlorophényl)-3,6-diéthylpyrazin-2-amine,
• la *N*-[(1*R*,2*S*)-2-éthoxy-2,3-dihydro-1*H*-indén-1-yl]-3,6-diéthyl-5-(4-méthoxy-2-méthylphényl)pyrazin-2-amine,
ou un sel pharmaceutiquement acceptable de l'un quelconque de ces composés.

5. Composé de Formule VI 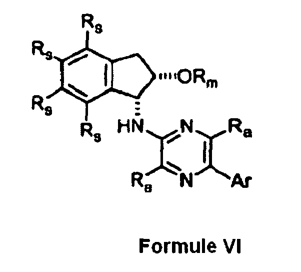 ou stéréoisomère de celui-ci, ou sel pharmaceutiquement acceptable correspondant, dans lequel, dans la Formule VI,
Ar est sélectionné parmi aryle, aryle substitué, hétéroaryle et hétéroaryle substitué ;
m représente 0, 1 ou 2 ;
chaque Rₛ est indépendamment sélectionné parmi halogéno, -NO₂, -CN, -Rₐ, -ORₐ, -S(O)ₘRₐ, -NRₐRₐ, -C(O)NRₐRₐ, -C(S)NRₐRₐ, -S(O)ₘNRₐRₐ, -NRₐS(O)ₘR_{a,} -NRₐC(O)ORₐ, -OC(O)NRₐRₐ, -NRₐC(O)NRₐRₐ, -NRₐC(S)NRₐRₐ, -C(O)ORₐ, -C(S)ORₐ et -OC(O)ORₐ ;
chaque Rₐ étant indépendamment sélectionné parmi H, alkyle, cycloalkyle, halogénoalkyle, aryle, hétéroaryle et hétérocycloalkyle facultativement substitués par de 1 à 5 groupe (s) choisis parmi Rₜ, -ORₜ, -S (O)ₘRₜ, NRₜRₜ, oxo (=O), thione (=S), phényle, hétéroaryle et hétérocycloalkyle, les groupes phényle, hétéroaryle et hétérocycloalkyle étant facultativement substitués par de 1 à 5 groupe(s) indépendamment choisi(s) parmi Rₜ ;
Rₘ représentant alkyle en C₁-C₆ substitué par 1-2 groupe(s) parmi halogéno, -NO₂, -NH₂, -OH, -SH, -CN, -C(O)NH₂, -C(O)-NH-alkyle, -C(O)N(alkyl)alkyle, -O-alkyle, NH-alkyle, N(alkyl)alkyle, -S(O)ₘalkyle, SO₂NH₂, SO₂NH-alkyle, SO₂N(alkyl)alkyle, oxo (=O), thione (=S), hétérocycloalkyle et hétérocycloalkyle substitué ;
chaque Rₜ étant indépendamment sélectionné parmi H, halogéno, -NO₂, -NH₂, -OH, -SH, -CN, -C(O)NH₂, -C(O)-NH-alkyle, -C(O)N(alkyl)alkyle, -O-alkyle, NH-alkyle, N(alkyl)alkyle, -S(O)ₘalkyle, SO₂NH₂, SO₂NH-alkyle, SO₂N(alkyl)alkyle, alkyle, cycloalkyle, halogénoalkyle, phényle, benzyle, hétéroaryle et hétérocycloalkyle, les groupes phényle, benzyle, hétéroaryle et hétérocycloalkyle pouvant être facultativement substitués par alkyle ou halogéno,
"aryle substitué", "hétéroaryle", "hétéroaryle substitué", "alkyle", "cycloalkyle", "halogénoalkyle", "hétérocycloalkyle" et "hétérocycloalkyle substitué" étant tels que définis dans la revendication 1.

6. Composé selon la revendication 5, qui est :
• la 5-(2,4-dichlorophényl)-3,6-diéthyl*-N*-[(1*R*,2*S*)-2-(2-fluoroéthoxy)-2,3-dihydro-1*H*-indén-1-yl]pyrazin-2-amine,
• le 2-[((1*R*,2*S*)-1-{[5-(2,4-dichlorophényl)-3,6-diéthylpyrazin-2-yl]amino}-2,3-dihydro-1*H*-indén-2-yl)oxy]éthanol,
• l'acétate de (1*R,*2*S*)-1-{[5-(2,4-dichlorophényl)-3,6-diéthylpyrazin-2-yl]amino}-2,3-dihydro-1*H*-indén-2-yle,
• la 5-(2-chloro-4-méthoxyphényl)-3,6-diéthyl*-N*-[(1*R*,2*S*)-2-(2-fluoroéthoxy)-2,3-dihydro-1*H*-indén-1-yl]pyrazin-2-amine,
• l'acétate de (1*R*,2*S*)-1-{[5-(2-chloro-4-méthoxyphényl)-3,6-diéthylpyrazin-2-yl]amino}-2,3-dihydro-1*H*-indén-2-yle,
• la 5-[2-chloro-4-(diméthylamino)phényl]-3,6-diéthyl*-N-*[(1*R*,2*S*)-2-(2-fluoroéthoxy)-2,3-dihydro-1*H*-indén-1-yl]pyrazin-2-amine,
• le diméthylcarbamate de (1*R*,2*S*)-1-{[5-(2,4-dichlorophényl)-3,6-diéthylpyrazin-2-yl]amino}-2,3-dihydro-1*H*-indén-2-yle,
• la 5-(2,4-diméthoxyphényl)-3,6-diéthyl*-N*-[(1*R*,2*S*)-2-(2-fluoroéthoxy)-2,3-dihydro-1*H*-indén-1-yl]pyrazin-2-amine,
ou un sel pharmaceutiquement acceptable de l'un quelconque de ces composés.

7. Composition pharmaceutique comprenant un composé tel que revendiqué dans l'une quelconque des revendications 1 à 6 et un support ou excipient pharmaceutiquement acceptable.

8. Procédé d'inhibition, in vitro, de la liaison du CRF au récepteur CRF₁, ce procédé comprenant la mise en contact, en présence de CRF, d'une solution comprenant un composé tel que revendiqué dans l'une quelconque des revendications 1 à 6 avec des cellules exprimant le récepteur CRF₁, le composé étant présent dans la solution à une concentration suffisante pour réduire, *in vitro,* le taux de liaison du CRF aux cellules.

9. Article manufacturé comprenant : a) un matériau de conditionnement ; b) un composé tel que revendiqué dans l'une quelconque des revendications 1 à 6 et c) une étiquette ou une notice d'accompagnement contenue au sein dudit matériau de conditionnement et indiquant que ledit composé est efficace pour traiter un trouble affectif, l'anxiété ou la dépression.

10. Composé tel que revendiqué dans l'une quelconque des revendications 1 à 6, pour une utilisation comme substance pharmaceutique active.

11. Utilisation d'un composé tel que revendiqué dans l'une quelconque des revendications 1 à 6, pour la préparation d'une composition pharmaceutique.

12. Utilisation d'un composé tel que revendiqué dans l'une quelconque des revendications 1 à 6, pour la préparation d'un médicament pour traiter un trouble manifestant une hypersécrétion de CRF chez un mammifère.

13. Utilisation d'un composé tel que revendiqué dans l'une quelconque des revendications 1 à 6, pour la préparation d'un médicament pour traiter un trouble dont le traitement peut être assuré ou facilité par l'antagonisation du CRF chez un mammifère.

14. Utilisation d'un composé tel que revendiqué dans l'une quelconque des revendications 1 à 6, pour la préparation d'un médicament pour traiter un trouble chez un mammifère, le trouble étant sélectionné parmi un trouble d'anxiété sociale ; un trouble panique ; un trouble obsessionnel compulsif ; l'anxiété associée à une maladie dépressive comorbide ; un trouble affectif ; l'anxiété ; la dépression ; le syndrome du côlon irritable ; un trouble de stress post-traumatique ; la paralysie supranucléaire ; une suppression immunitaire ; l'anorexie nerveuse ou un autre trouble alimentaire ; des symptômes de sevrage toxicomaniaque ou alcoolique ; un trouble de l'abus de certaines substances ; des problèmes de fertilité ; des troubles inflammatoires ; la douleur ; l'asthme ; le psoriasis ; les allergies ; le trouble d'anxiété généralisée ; la panique ; les phobies ; les troubles du sommeil induits par le stress ; la fibromyalgie ; les troubles de l'humeur ; la dysthymie ; les troubles bipolaires ; la cyclothymie ; le syndrome de fatigue ; les céphalées induites par le stress ; le cancer ; les infections par le virus de l'immunodéficience humaine ; les maladies neurodégénératives ; les maladies gastro-intestinales ; les troubles alimentaires ; le stress hémorragique ; les épisodes psychotiques induits par le stress ; le syndrome euthyroïdien ; le syndrome de l'hormone antidiarrhéique inappropriée ; l'obésité ; l'infertilité ; les traumatismes crâniens ; un traumatisme de la moelle épinière ; une lésion neuronale ischémique ; une lésion neuronale excitotoxique ; l'épilepsie ; les troubles cardio-vasculaires et relatifs au coeur ; l'accident vasculaire cérébral ; les dysfonctions immunitaires ; les spasmes musculaires ; l'incontinence urinaire ; la démence sénile de type Alzheimer ; la démence multi-infarctus ; la sclérose latérale amyotrophique ; les dépendances et addictions chimiques ; l'ostéoporose ; le nanisme psychosocial ; et l'hypoglycémie.

15. Composé tel que revendiqué dans l'une quelconque des revendications 1 à 6, pour une utilisation dans le traitement d'un trouble d'anxiété sociale ; d'un trouble panique ; d'un trouble obsessionnel compulsif ; de l'anxiété associée à une maladie dépressive comorbide ; d'un trouble affectif ; de l'anxiété ; de la dépression ; du syndrome du côlon irritable ; d'un trouble de stress post-traumatique ; de la paralysie supranucléaire ; d'une suppression immunitaire ; de l'anorexie nerveuse ou d'un autre trouble alimentaire ; des symptômes de sevrage toxicomaniaque ou alcoolique ; d'un trouble de l'abus de certaines substance ; des troubles inflammatoires ; des problèmes de fertilité ; de la douleur ; de l'asthme ; du psoriasis ; des allergies ; du trouble d'anxiété généralisée ; de la panique ; des phobies ; des troubles du sommeil induits par le stress ; de la fibromyalgie ; des troubles de l'humeur ; de la dysthymie ; des troubles bipolaires ; de la cyclothymie ; du syndrome de fatigue ; des céphalées induites par le stress ; du cancer ; des infections du virus de l'immunodéficience humaine ; des maladies neurodégénératives ; des maladies gastro-intestinales ; des troubles alimentaires ; du stress hémorragique ; des épisodes psychotiques induits par le stress ; du syndrome euthyroïdien ; du syndrome de l'hormone antidiarrhéique inappropriée ; de l'obésité ; de l'infertilité ; des traumatismes crâniens ; d'un traumatisme de la moelle épinière ; d'une lésion neuronale ischémique ; d'une lésion neuronale excitotoxique ; de l'épilepsie ; des troubles cardio-vasculaires et relatifs au coeur ; de l'accident vasculaire cérébral ; des dysfonctions immunitaires ; des spasmes musculaires ; de l'incontinence urinaire ; de la démence sénile de type Alzheimer ; de la démence multi-infarctus ; de la sclérose latérale amyotrophique ; des dépendances et addictions chimiques ; de l'ostéoporose ; du nanisme psychosocial ; et de l'hypoglycémie.
